(19) Europäisches Patentamt
European Patent Office
Office européen des brevets

(11) Veröffentlichungsnummer: **0 187 122**
**A2**

# (12) EUROPÄISCHE PATENTANMELDUNG

(21) Anmeldenummer: **85810612.3**

(22) Anmeldetag: **23.12.85**

(51) Int. Cl.[4]: **C 07 D 401/06**, C 07 D 403/06, A 61 K 31/40

(30) Priorität: **03.01.85 CH 13/85**
**15.07.85 CH 3053/85**

(43) Veröffentlichungstag der Anmeldung: **09.07.86 Patentblatt 86/28**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Fröstl, Wolfgang, Dr., St. Johanns-Vorstadt 6/2, CH-4056 Basel (CH)**
Erfinder: **Storni, Angelo, Dr., Im Feuerbusch 3, CH-4310 Rheinfelden (CH)**
Erfinder: **Schmitz, Beat, Dr., Maiengasse 27, CH-4123 Allschwil (CH)**

## (54) 1,3,4-Trisubstituierte Azacycloalkane bzw. Azacycloalkene.

(57) 1,3,4-Trisubstituierte Azacycloalkane bzw. Azacycloalkene der Formel

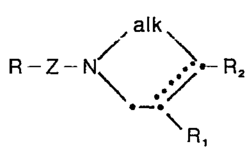

(I),

worin R einen gegebenenfalls substituierten 3-Indolylrest bedeutet, alk Niederalkylen darstellt, welches den Rest R durch 1 bis 3 Kohlenstoffatome von Stickstoffatom trennt, $R_1$ gegebenenfalls verestertes oder amidiertes Carboxy bedeutet und $R_2$ für eine gegebenenfalls veretherte oder acylierte Hydroxygruppe oder für eine gegebenenfalls acylierte Aminogruppe steht, und ihre Tautomeren und/oder Salze haben nootrope Wirkung und können als Nootropika Verwendung finden. Sie werden beispielsweise hergestellt, indem man Verbindungen der Formeln

$R-Z-X_3$ (V) und

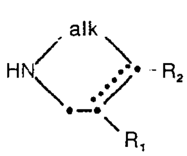

(VI),

worin $X_3$ eine nukleofuge Abgangsgruppe bedeutet, oder deren Salze miteinander umsetzt.

EP 0 187 122 A2

CIBA-GEIGY AG 4-15426/+

Basel (Schweiz)

# 1,3,4-Trisubstituierte Azacycloalkane bzw. Azacycloalkene

Die Erfindung betrifft neue 1,3,4-trisubstituierte Azacycloalkane und Azacycloalkene der Formel

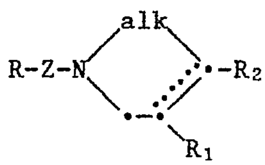

(I),

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, alk Methylen, Ethylen oder 1,3-Propylen darstellt, $R_1$ gegebenenfalls verestertes oder amidiertes Carboxy bedeutet und $R_2$ für eine gegebenenfalls veretherte oder acylierte Hydroxygruppe oder für eine gegebenenfalls acylierte Aminogruppe steht, und ihre Tautomeren und/oder Salze, insbesondere pharmazeutisch verwendbaren Salze, Verfahren zu ihrer Herstellung, diese enthaltende pharmazeutischen Präparate und ihre Verwendung

Die Punktreihe in Formel I bedeutet dabei, dass zwischen den durch $R_1$ und $R_2$ substituierten Kohlenstoffatomen (C-Atomen) eine Einfach- oder Doppelbindung vorliegen kann.

Als Substituenten des 3-Indolylrestes kommen beispielsweise aliphatische Kohlenwasserstoffreste, wie Niederalkyl oder Niederalkenyl, in 4- bis und mit 7-Stellung ebenso gegebenenfalls veresterte oder veretherte Hydroxygruppen, wie Hydroxy, Niederalkanoyloxy, Halogen, Niederalkoxy, Niederalkenyloxy oder an benachbarte Kohlenstoffatome (C-Atome) gebundenes Niederalkyl(id)endioxy, ferner Trifluormethyl,

in Betracht. Dabei können bis und mit 4, insbesondere 1, 2 oder 3 der genannten Substituenten vorhanden sein, beispielsweise 1 bis und mit 3, insbesondere 1 oder 2 Substituenten in 4- bis 7-Stellung und/oder jeweils 1 Substituent in 1- und/oder 2-Stellung.

Verethertes Hydroxy $R_2$ ist beispielsweise Niederalkoxy oder gegebenenfalls substituiertes Phenylniederalkoxy.

Acyl in acyliertem Hydroxy bzw. Amino $R_2$ ist beispielsweise von einer organischen Carbonsäure oder einem Halbester der Kohlensäure abgeleitetes Acyl.

Von organischen Carbonsäuren abgeleitetes Acyl ist beispielsweise der Rest einer aliphatischen oder monocyclisch- aromatischen Carbonsäure, wie Niederalkanoyl oder gegebenenfalls substituiertes Benzoyl, ferner Pyridoyl.

In von Halbestern der Kohlensäure abgeleitetem Acyl ist die zweite Hydroxygruppe beispielsweise mit einem aliphatischen oder aryl-, wie phenylaliphatischen Alkohol verestert. Als von Halbestern der Kohlensäure abgeleitete Acylgruppen seien beispielsweise Niederalkoxycarbonyl und gegebenenfalls substituiertes Phenylniederalkoxycarbonyl genannt.

Verestertes Carboxy bedeutet beispielsweise aliphatisch, cycloaliphatisch oder aryl-, wie phenylaliphatisch verestertes Carboxy, z.B. Niederalkoxycarbonyl, gegebenenfalls substituiertes Phenylniederalkoxycarbonyl, 3- bis und mit 8-gliedriges, wie 5- oder 6-gliedriges Cycloalkoxycarbonyl, d.h. Cyclopentyloxy- oder Cyclohexyloxycarbonyl, ferner Cyclopropyloxy-, Cyclobutyloxy- oder Cycloheptyloxycarbonyl.

Amidiertes Carboxy bedeutet beispielsweise unsubstituiertes, aliphatisch N-substituiertes oder N-acyliertes Carbamyl, wie Carbamyl, N-Mono- oder N,N-Diniederalkylcarbamyl, N,N-Niederalkylen- bzw. N,N-(Aza-, Oxa- oder Thia)niederalkylencarbamyl oder N-Acylcarbamyl, worin Acyl beispielweise eine der für Acyl in acyliertem Hydroxy bzw. Amino $R_2$ angegebene Bedeutung hat. Derartige N-Acylcarbamylgruppen sind beispielsweise: N-Niederalkanoylcarbamyl, N-Niederalkoxycarbonylcarbamyl, Ureidocarbonyl, N'-Mono- oder N',N'-Diniederalkylureidocarbonyl oder N',N'-Niederalkylen- bzw. N',N'-(Aza-, Oxa- oder Thia)niederalkylenureidocarbonyl.

Vor und nachstehend können Aryl-, wie Phenylgruppen als Bestandteil von Substituenten des Restes $R_1$ und/oder $R_2$ sowie von Ausgangsstoffen für die Herstellung von Verbindungen I auch substituiert sein, z.B. durch Niederalkyl, Niederalkoxy, Halogen, Nitro und/oder Trifluormethyl.

Ebenso sind, wenn nicht anders angegeben, unter "niederen" Resten und organischen Verbindungen solche Reste bzw. Verbindungen zu verstehen, die bis und mit 7, insbesondere bis und mit 4, C-Atome aufweisen.

Niederalkylen, welches den Rest R von dem in Formel I eingezeichneten Stickstoffatom durch 1 bis 3, vorzugsweise 2, C-Atome trennt, ist beispielsweise Methylen, 1,1-Ethylen (Ethyliden), 1,3-Propylen, 2,4-Butylen oder vorzugsweise Ethylen, 1,2-Propylen oder 1,2-Butylen, kann aber auch 2,3-Butylen sein.

Niederalkyl ist beispielsweise Methyl, Ethyl, Propyl, Isopropyl, Butyl oder Isobutyl, ferner Sekundärbutyl oder Tertiärbutyl.

Niederalkenyl ist beispielsweise Allyl oder Methallyl.

Halogen ist beispielsweise Halogen der Atomnummer bis und mit 35, wie Fluor, Chlor oder Brom.

Niederalkoxy ist beispielsweise Methoxy, Ethoxy, Propyloxy oder Butyloxy. Niederalkenyloxy ist beipsielsweise Allyloxy.

Niederalkyl(id)endioxy ist beispielsweise Methylendioxy, Ethylidendioxy, Ethylendioxy, Isopropylidendioxy oder 1,3-Propylendioxy.

Niederalkanoyl ist beispielsweise Formyl, Acetyl, Propionyl, Butyryl, Isobutyryl, Valeroyl oder Pivaloyl.

Niederalkoxycarbonyl ist beispielsweise Methoxy-, Ethoxy-, Propyloxy-, Isopropyloxy-, Butyloxy-, Isobutyloxy-, Sekundärbutyloxy- bzw. Tertiärbutyloxycarbonyl oder eine Pentyloxy-, Hexyloxy- oder Heptyloxycarbonylgruppe.

Cycloalkoxycarbonyl hat beispielsweise 3 bis 8, insbesondere 3 bis 6, Ringglieder und bedeutet z.B. Cyclopropyloxycarbonyl, Cyclobutyloxycarbonyl, Cyclopentyloxycarbonyl oder Cyclohexyloxycarbonyl.

Mono- oder Diniederalkylcarbamyl ist beispielsweise Methyl-, Dimethyl-, Ethyl-, Diethyl-, Propyl-, Isopropyl-, Butyl- oder Isobutylcarbamyl.

Niederalkylencarbamyl bzw. Aza-, Oxa- oder Thianiederalkylencarbamyl ist beispielsweise Pyrrolidinocarbonyl oder Piperidinocarbonyl, ferner Piperazino- oder N'-Niederalkyl-, wie N'-Methylpiperazinocarbonyl, Morpholinocarbonyl oder Thiomorpholinocarbonyl.

N'-Mono- oder N',N'-Diniederalkylureidocarbonyl ist beispielsweise N'-Methyl-, N'-Ethyl-, N'-Propyl-, N'-Butylureidocarbonyl bzw. N',N'-Dimethyl- oder N',N'-Diethylureidocarbonyl.

N',N'-Niederalkylen- bzw. N',N'-(Aza-, Oxa- oder Thia)niederalkylenureidocarbonyl ist beispielsweise N-Piperidinocarbonylcarbamyl, N-Pyrrolidinocarbonylaminocarbamyl, N- Morpholino-, N-Thiomorpholino oder N-Piperazino- bzw. N'-(Niederalkyl-, wie N'-Methyl)-piperazinocarbonylcarbamyl.

Phenylniederalkyl ist beispielsweise Benzyl, 1- oder 2-Phenethyl oder 3-Phenylpropyl. Ebenso ist Phenylniederalkoxy, auch als Bestandteil von Phenylniederalkoxycarbonyl z.B. Benzyloxy, 1- oder 2-Phenethoxy oder 3-Phenylpropyloxy.

Tautomere von Verbindungen der Formel I sind beispielsweise Keto- bzw. Ketiminderivate von Verbindungen der Formel I, worin der azacycloaliphatische Ring eine Doppelbindung aufweist und $R_2$ für Hydroxy oder gegebenenfalls acyliertes Amino steht, d.h. Verbindungen der Formel

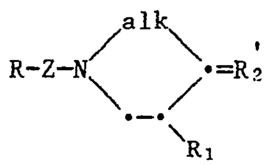

(I')

worin $R_2'$ für Oxo oder gegebenenfalls acyliertes Imino steht.

Salze von Verbindungen der Formel I sind beispielsweise deren pharmazeutisch verwendbare Säureadditionssalze, ebenso innere Salze von Verbindungen der Formel I, in denen $R_1$ Carboxy bedeutet und deren pharmazeutisch verwendbare Salze mit Basen.

Pharmazeutisch verwendbare Säureadditionssalze sind beispielsweise Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten organischen Carbon- oder Sulfonsäuren, wie gegebenenfalls hydroxylierten aliphatischen Mono- oder Dicarbonsäuren, z.B. Acetate, Oxalate, Succinate, Fumarate, Maleinate, Maleate, Ascorbinate oder Citrate, der aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Pharmazeutisch verwendbare Salze mit Basen sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylamin, z.B. N,N'-Dibenzylethylendiamin, Dibenzylamin oder N-Benzyl-β-phenylethylamin. Verbindungen der Formel I mit einer sauren und mit einer basischen Gruppe können auch in Form von inneren Salzen, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre pharmazeutisch verwendbaren Salze besitzen wertvolle pharmakologische, insbesondere nootrope Eigenschaften. So vermindern sie an der Maus in Dosen ab etwa 0,1 mg/kg i.p. sowie p.o. die amnesiogene Wirkung eines Elektroschocks in mindestens gleichem Ausmass wie nach Verabfolgung einer nootrop wirksamen Dosis von Piracetam (100 mg/kg i.p.). Zum Nachweis der nootropen Wirkung kann beispielsweise der Two-Compartment-Test herangezogen werden.

Als Literatur über pharmakologische Modelle dieser Art seien z.B. genannt: S.J. Sara und D. Lefevre, Psychopharmacologia 25, 32-40 (1972), Hypoxia-induced amnesia in one-trial learning and pharmacological protection by piracetam. Boggan, W.O., und Schlesinger, K., in Behavioral Biology 12, 127-134 (1974).

Im weiteren zeigen die Verbindungen der Formel I eine starke gedächtnisverbessernde Wirkung im Step-down Passive Avoidance Test nach Mondadori und Waser, Psychopharmacology 63, 297-300 (1979). Die Substanzen sind wirksam bei intraperitonealer Gabe 30 Minuten vor dem Lernversuch (wirksame Dosen 0,1, 1, 10 mg/kg). Eine deutliche

Wirkung war auch feststellbar bei peroraler Applikation 60 Minuten vor dem Lernversuch (wirksame Dosen 0,1, 1, 10 mg/kg) sowie bei intraperitonealer Applikation unmittelbar nach dem Lernversuch (wirksame Dosen 0,1, 1, 10 mg/kg).

Die Verbindungen der Formel I und ihre pharmazeutisch verwendbaren Salze können dementsprechend als Nootropika, beispielsweise zur Behandlung zerebraler Leistungsinsuffizienz, insbesondere von Gedächtnisstörungen unterschiedlicher Genese, wie senile Demenz oder Demenz vom Alzheimer-Typ, ferner von Folgezuständen von Hirntraumata und Apoplexien, Verwendung finden.

Die Erfindung betrifft beispielsweise Verbindungen der Formel I, worin R unsubstituiertes oder in 5-Stellung durch Methoxy substituiertes 3-Indolyl bedeutet, Z und alk Ethylen darstellen, $R_1$ Methoxycarbonyl bedeutet und $R_2$ Hydroxy oder Amino bedeutet oder, sofern der azacyclische Ring keine Doppelbindung aufweist, Niederalkanoyloxy mit bis und mit 4 C-Atomen, z.B. Acetoxy, darstellt, und ihre Tautomeren und/oder Salze.

Die Erfindung betrifft in erster Linie Verbindungen der Formel I, worin R unsubstituiertes oder in mindestens einer der Stellungen 1, 2 und 4 bis 7 substituiertes 3-Indolyl bedeutet, wobei als Substituent in 1-Stellung Niederalkyl oder Niederalkenyl, in 2-Stellung Niederalkyl und als Substituent(en) in 4- bis 7-Stellung Niederalkyl, Niederalkenyl, Niederalkoxy, Niederalkenyloxy, Hydroxy, Halogen, Trifluormethyl und/oder an benachbarte C-Atome gebundenes Niederalkyl(id)endioxy in Betracht kommen, Z Niederalkylen darstellt, welches den Rest R vom Stickstoffatom durch 1 bis 3, vorzugsweise 2 C-Atome trennt, alk Methylen, 1,3-Propylen oder vor allem Ethylen bedeutet, $R_1$ für Carboxy, Niederalkoxycarbonyl, gegebenenfalls durch Niederalkyl, Niederalkoxy, Halogen, Nitro und/oder Trifluormethyl substituiertes Phenylniederalkoxycarbonyl, 3- bis 8-gliedriges Cycloalkoxycarbonyl, Carbamyl, Mono- oder Diniederalkylcarbamyl, Niederalkylen- bzw. Aza-, Oxa- oder Thianiederalkylencarbamyl, N-Niederalkanoylcarbamyl, N-Niederalkoxycarbonylcarbamyl, Ureidocarbonyl, N'-Mono- oder N',N'-Diniederalkylureidocarbonyl, N',N'-Niederalkylen- bzw. N',N'-(Aza-, Oxa- oder Thia)niederalkylenureidocarbonyl steht und $R_2$ für Hydroxy, Niederalkoxy, gegebenenfalls durch Niederalkyl, Niederalkoxy, Halogen, Trifluormethyl und/oder Nitro substituiertes Phenylniederalkoxy, Amino, Niederalkanoylamino, Niederalkoxycarbonylamino oder gegebenenfalls durch Niederalkyl, Niederalkoxy, Halogen, Trifluormethyl und/oder Nitro substituiertes Phenylniederalkoxycarbonylamino steht, wobei Niederalkylen alk bis und mit 4, die übrigen niederen Reste insgesamt bis und mit 7 und in jeder niederen Teilstruktur bis und mit 4 C-Atome aufweisen, und ihre Tautomeren und/oder Salze, insbesondere pharmazeutisch verwendbaren Säureadditionssalze.

Die Erfindung betrifft vor allem Verbindungen der Formel I, worin R unsubstituiertes oder in einer oder zwei der Stellungen 4 bis 7 durch Niederalkyl mit bis und mit 4 C-Atomen, wie Methyl, Niederalkoxy mit bis und mit 4 C-Atomen, wie Methoxy, Halogen der Atomnummer bis und mit 35, wie Chlor, und/oder Trifluormethyl substituiertes 3-Indolyl bedeutet, Z Niederalkylen mit bis und mit 4 C-Atomen darstellt, welches den Rest R vom Stickstoffatom durch 1 bis 3, vorzugsweise 2, C-Atome trennt, wie Methylen, 1,3-Propylen oder vorzugsweise Ethylen, bedeutet, alk Methylen, 1,3-Propylen oder vor allem Ethylen bedeutet, $R_1$ für Carboxy, Niederalkoxycarbonyl mit bis und mit 4 C-Atomen im Alkoxyteil, wie Methoxy- oder Ethoxycarbonyl, 3- bis 8-, vorzugsweise 5- oder 6-gliedriges Cycloalkoxycarbonyl, wie Cyclopentyloxy- oder Cyclohexyloxycarbonyl, Carbamyl oder N-Mono-oder N,N-Diniederalkylcarbamyl mit bis und mit 4 C-Atomen in jedem Alkylteil, wie Methyl-, Ethyl- oder Dimethylcarbamyl, bedeutet und $R_2$ Hydroxy, Niederalkoxy mit bis und mit 4 C-Atomen, wie Methoxy, Phenylniederalkoxy mit 7 bis und mit 10 C-Atomen, wie Benzyloxy, Niederalkanoyloxy mit bis und mit 4 C-Atomen, wie Acetoxy, Amino oder Niederalkanoylamino mit bis und mit 4 C-Atomen, wie Acetylamino, bedeutet, und ihrer Tautomeren und/oder Salze, insbesondere pharmazeutisch verwendbaren Säureadditionssalze.

Die Erfindung betrifft insbesondere Verbindungen der Formel I, worin R unsubstituiertes oder in einer oder zwei der Stellungen 1 und 4 bis 7 substituiertes 3-Indolyl bedeutet, wobei als Substituent in 1-Stellung Niederalkyl, wie Methyl, und als Substituent(en) in 4-bis 7-Stellung Niederalkoxy mit bis und mit 4 C-Atomen, wie Methoxy, Halogen der Atomnummer bis und mit 35, wie Chlor, und/oder Niederalkyl mit bis und mit 4 C-Atomen, wie Methyl, in Betracht kommen, alk Ethylen darstellt, Z 1,1-, 1,3- oder vorzugsweise 1,2-Niederalkylen mit bis und mit 4 C-Atomen, wie Ethylen, ferner 1,3-Propylen oder Methylen, darstellt, $R_1$ Niederalkoxycarbonyl mit bis und mit 4 C-Atomen im Niederalkoxyteil, wie Methoxycarbonyl oder Ethoxycarbonyl, darstellt und $R_2$ Hydroxy oder Amino bedeutet, und ihre Tautomeren und/oder Salze, insbesondere pharmazeutisch verwendbare Säureadditionssalze.

Die Erfindung betrifft vorzugsweise Verbindungen der Formel I, worin R unsubstituiertes oder in einer oder zwei der Stellungen 4 bis 7 durch Niederalkyl mit bis und mit 4 C-Atomen, wie Methyl, und/oder Halogen der Atomnummer bis und mit 35, wie Chlor, substituiertes 3-Indolyl bedeutet, Z geradkettiges 1,1-, 1,3- oder vorzugsweise 1,2-Niederalkylen mit bis und mit 4 C-Atomen, wie Methylen, 1,3-Propylen oder vorzugsweise Ethylen, bedeutet, alk Methylen oder vor allem Ethylen bedeutet, $R_1$ Niederalkoxycarbonyl mit bis und mit 4 C-Atomen im Alkoxyteil, wie Methoxy- oder Ethoxycarbonyl, darstellt und $R_2$ Hydroxy bedeutet, und ihre Tautomeren und/oder Salze, insbesondere pharmazeutisch verwendbaren Säureadditionssalze.

Die Erfindung betrifft in allererster Linie Verbindungen der Formel I, worin R unsubstituiertes oder in 4- und 7-Stellung durch Niederalkyl mit bis und mit 4 C-Atomen, wie Methyl, substituiertes 3-Indolyl bedeutet, Z und alk Ethylen darstellen, $R_1$ Niederalkoxycarbonyl mit bis und mit 4 C-Atomen im Niederalkoxyteil, wie Methoxycarbonyl oder Ethoxycarbonyl, bedeutet und $R_2$ für Hydroxy steht, und ihre Tautomeren und/oder Salze, insbesondere pharmazeutisch verwendbaren Säureadditionssalze.

Die Erfindung betrifft namentlich die in den Beispielen genannten Verbindungen der Formel I und ihre pharmazeutisch verwendbaren Salze, insbesondere Säureadditionssalze, wie ihre Hydrochloride, Hydrobromide, Fumarate, Oxalate oder Cyclamate.

Die Erfindung betrifft jeweils vorzugsweise diejenigen Verbindungen der Formel I, in denen der azacycloaliphatische Ring gesättigt ist und $R_1$ und $R_2$ zueinander cis-ständig sind.

Die Erfindung betrifft ferner ein auf an sich bekannten Synthesemethoden beruhendes Verfahren zur Herstellung von Verbindungen der Formel I unter Einschluss ihrer Tautomeren und/oder Salze.

Das erfindungsgemässe Verfahren ist dadurch gekennzeichnet, dass man

a) zur Herstellung von Verbindungen der Formel I, worin alk Ethylen ist, in einer Verbindung der Formel

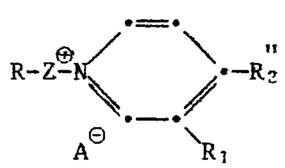

(II),

worin $A^{\ominus}$ ein Säureanion und $R_2''$ veretheres, acyliertes oder geschütztes Hydroxy oder acyliertes oder geschütztes Amino bedeutet, die überschüssigen Doppelbindungen zu Einfachbindungen reduziert und gegebenenfalls vorhandene Schutzgruppen abspaltet, oder

b) eine Verbindung der Formel

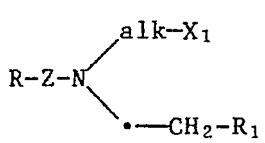

(III),

worin $X_1$ eine Gruppe der Formeln $-CH{=}R_2'$, $-C(X_2){=}R_2'$ oder Cyano darstellt, wobei $X_2$ einen abspaltbaren Rest bedeutet, oder ein Salz davon cyclisiert, oder

c) eine Verbindung der Formel

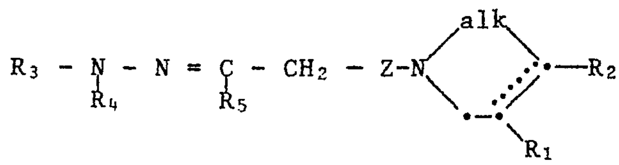 (IV),

worin $R_3$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel I ringschliesst, oder

d) Verbindungen der Formeln

$R - Z - X_3$ (V) und 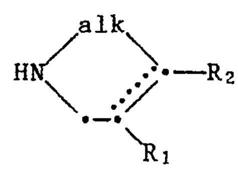 (VI),

worin $X_3$ eine nukleofuge Abgangsgruppe bedeutet, oder deren Tautomere und/oder Salze miteinander umsetzt, oder

e) zur Herstellung von Verbindungen der Formel I, worin alk Ethylen ist, eine Verbindung der Formel

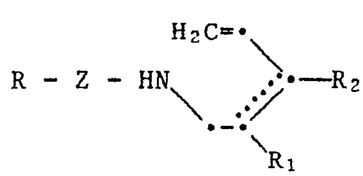 (VII)

oder ein Tautomeres und/oder Salz davon cyclisiert, oder

f) in einer Verbindung der Formel

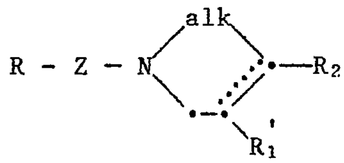 (VIII),

worin $R_1'$ einen in gegebenenfalls verestertes oder amidiertes Carboxy überführbaren Rest bedeutet, oder in einem Tautomeren und/oder Salz davon, $R_1'$ in gegebenenfalls verestertes oder amidiertes Carboxy überführt, oder

g) zur Herstellung von Verbindungen der Formel I, worin der azacycloaliphatische Rest eine Doppelbindung aufweist, eine Verbindung der Formel

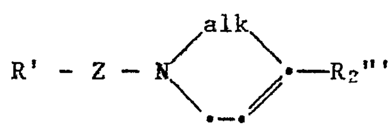 (IX),

worin R' einen in 1-Stellung substituierten oder intermediär geschützten 3-Indolylrest R und $R_2'''$ eine Gruppe $R_2$ oder intermediär geschütztes Hydroxy bedeutet, oder das entsprechende Keto- bzw. Iminotautomere davon mit einer Verbindung der Formel $R_1$-$X_6$ (VIa), worin $X_6$ einen abspaltbaren Rest bedeutet, kondensiert und gegebenenfalls die Schutzgruppe(n) abspaltet, oder

h) in einer Verbindung der Formel

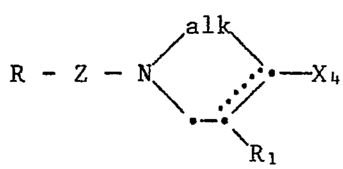 (X),

worin $X_4$ einen in eine Gruppe $R_2$ überführbaren Rest bedeutet, $X_4$ in eine Gruppe $R_2$ überführt, oder

i) zur Herstellung von Verbindungen der Formel I, worin der azacycloaliphatische Ring einfach ungesättigt ist, und $R_2$ Hydroxy bedeutet, bzw. der tautomeren Oxoverbindung eine Verbindung der Formel

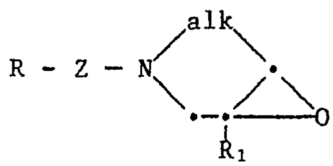 (XI),

umlagert, oder

j) zur Herstellung von Verbindungen der Formel I, worin der azacycloaliphatische Ring gesättigt ist, Verbindungen der Formeln

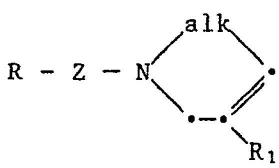 (XII) und $R_2 - H$ (XIII)

miteinander umsetzt, oder

k) zur Herstellung von Verbindungen der Formel I, worin Z den Rest R vom Stickstoffatom durch 1 C-Atom trennt, Verbindungen der Formeln

R–H (L) und 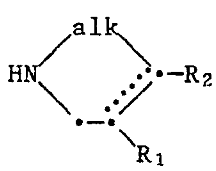 (VI)

mit einem Oxoniederalkan kondensiert und jeweils gewünschtenfalls die verfahrensgemäss erhältliche Verbindung in eine andere Verbindung der Formel I überführt, ein verfahrensgemäss erhältliches Isomerengemisch in die Komponenten auftrennt und das Isomere der Formel I isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere

isoliert und/oder eine verfahrensgemäss erhältliche freie Verbindung in ein Salz oder ein verfahrensgemäss erhältliches Salz in die freie Verbindung oder in ein anderes Salz umwandelt.

Verfahrensvariante a)

Das Anion $A^{\ominus}$ ist beispielsweise das Anion einer starken Protonensäure, die alkoholisches Hydroxy in reaktionsfähiges verestertes Hydroxy zu überführen vermag, z.B. ein Halogenidion, wie Chlorid, Bromid oder Iodid, oder ein Sulfonation, insbesondere das Anion einer aliphatischen oder aromatischen Sulfonsäure, z.B. das Methansulfonat-, Ethansulfonat- oder Methosulfato-ion bzw. das Benzolsulfonat-, p-Toluolsulfonat- oder p-Brombenzolsulfonation. $R''_2$ ist insbesondere verethertes Hydroxy $R_2$ oder geschütztes Hydroxy.
Die Reduktion der überschüssigen Doppelbindungen erfolgt in üblicher Weise, beispielsweise durch Einwirkung eines Dileichtmetallhydrides, wie eines Alkalimetall-triniederalkoxyaluminiumhydrides, z.B. von Lithium-tritertiärbutyloxyaluminiumhydrid, oder eines gegebenenfalls substituierten Alkalimetallborhydrides, wie von Lithiumborhydrid, Kaliumborhydrid, Natriumborhydrid oder Natriumcyanborhydrid. Die Reaktion wird vorzugsweise in einem dafür üblichen Lösungsmittel durchgeführt. Ueblich sind beispielsweise für die Umsetzung mit Lithium-tritertiärbutyloxyaluminiumhydrid etherartige Lösungsmittel, wie Diethylether, Dioxan oder Tetrahydrofuran, und für die Umsetzung mit gegebenenfalls substituierten Alkalimetallborhydriden insbesondere Niederalkanole, wie Methanol oder Ethanol, ferner Wasser.

Zwischenprodukte II können beispielsweise durch Umsetzung von Verbindungen der Formeln

R - Z - A (XIV) und 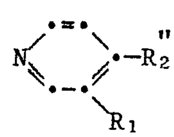 (XIVa),

worin A dem Anion $A^{\ominus}$ entsprechendes reaktionsfähiges verestertes Hydroxy bedeutet, erhalten werden.

Verfahrensvariante b)

In Zwischenprodukten III bedeutet ein abspaltbarer Rest $X_2$ gegebenenfalls verethertes oder verestertes Hydroxy, wie Hydroxy, aliphatisch, araliphatisch oder aromatisch verethertes Hydroxy, z.B. Niederalkoxy, Phenylniederalkoxy oder Phenoxy, mit einer Carbonsäure verestertes Hydroxy, z.B. Niederalkanoyloxy, z.B. Acetoxy, oder Halogen, z.B. Chlor oder Brom, oder eine gegebenenfalls substituierte Amino- oder Ammoniogruppe, wie Amino, gegebenenfalls substituiertes Anilino, Mono- oder Diniederalkylamino, z.B. Mono- oder Dimethylamino, oder Triniederalkylammonio, z.B. Trimethylammonio.

Die intramolekulare Kondensation erfolgt in üblicher Weise, beispielsweise in Gegenwart eines basischen Kondensationsmittels, wie eines Alkoholates, Amides oder Hydrides, eines Alkalimetalles oder Erdalkalimetalles, z.B eines Alkalimetall- oder Erdalkalimetallalkoholates, z.B. von Natriummethanolat, Magnesiummethanolat, Natriumethanolat oder Kaliumtertiärbutanolat, eines Alkalimetallamides, z.B. von Natriumamid, Lithiumdiisopropylamid, Natrium-N-methylanilid oder Natriumdiphenylamid, oder eines Alkalimetall- oder Erdalkalimetallhydrides, z.B. von Natriumhydrid oder Calciumhydrid, erforderlichenfalls unter Kühlen oder Erwärmen, z.B. bei etwa -10°C bis +120°C, und/oder unter Inertgas, wie Stickstoff.

In einer bevorzugten Ausführungsform der Verfahrensvariante b) kann man beispielsweise eine Verbindung der Formel

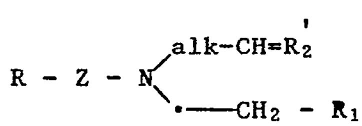 (XV),

worin $R_2'$ Oxo oder Imino bedeutet, der Behandlung mit einer der genannten Basen, insbesondere mit einem Alkalimetallniederalkanolat, z.B. mit Natriummethanolat oder Natriumethanolat unterwerfen. Dabei cyclisiert die Verbindung XV zu einer Verbindung der Formel I, worin

der azacycloaliphatische Ring keine Doppelbindung aufweist und $R_2$ Hydroxy oder Amino bedeutet. Ausgangsstoffe XV werden z.B. erhalten, indem man einen reaktionsfähigen Indol-3-yl-alkanolester der Formel

$$R - Z - X_3 \quad (V),$$

worin $X_3$ reaktionsfähiges verestertes Hydroxy ist, mit einer ß-Aminosäureverbindung der Formel $H_2N-CH_2-CH_2-R_1$ (XVa) kondensiert und das erhaltene Zwischenprodukt der Formel

$$R - Z - \underset{R_6}{\underset{|}{N}} - CH_2CH_2 - R_1 \quad (XVI),$$

worin $R_6$ Wasserstoff ist, mit Acrolein oder einem gegebenenfalls funktionell abgewandelten Aldehyd der Formel $Y_1$-alk-CH=$R_2$' (XVII; $Y_1$ = reaktionsfähiges verestertes Hydroxy; $R_2$' = ggf. acetalisiertes Oxo oder Imino) umsetzt.

In einer anderen bevorzugten Ausführungsform der Verfahrensvariante b) cyclisiert man eine durch Umsetzung einer Verbindung der Formel R-Z-$NH_2$ (XVIII) mit der annähernd doppelt-molaren Menge einer Verbindung der Formel $Y_1-CH_2CH_2-R_1''$ (XIXa; $Y_1$ = reaktionsfähiges verestertes Hydroxy; $R_1''$ = verestertes Carboxy) bzw. $CH_2=CH-R_1''$ (XIXb; $R_1''$ = verestertes Carboxy) erhältliche Verbindung der Formel

$$R - alk - N\begin{cases} -CH_2-R_1'' \\ -CH_2-R_1'' \end{cases} \quad (XX),$$

z.B. in Gegenwart einer Metallbase, wie eines Alkalimetallalkoholats, z.B. von Natriummethanolat, zu einer Verbindung der Formel I', worin $R_2$' Oxo ist, bzw. zu dem entspechenden Tautomeren der Formel I. Verestertes Carboxy $R_1''$ ist z.B. Methoxycarbonyl.

Beispielsweise kann man Verbindungen der Formel I, worin R unsubstituiertes oder in 5-Stellung durch Methoxy substituiertes 3-Indolyl bedeutet, Z und alk Ethylen darstellen, $R_1$ Methoxycarbonyl

bedeutet und $R_2$ Hydroxy oder Amino bedeutet oder, sofern der azacyclische Ring keine Doppelbindung aufweist, Niederalkanoyloxy mit bis und mit 4 C-Atomen, z.B. Acetoxy, darstellt, und ihrer Tautomeren und/oder Salze erhalten, indem man eine Verbindung der Formel

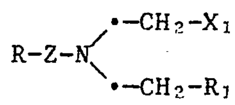

(III')

worin R gegebenenfalls in 5-Stellung durch Methoxy substituiertes 3-Indolyl, Z Ethylen, $R_1$ Methoxycarbonyl und $X_1$ Methoxycarbonyl, Cyano oder eine Gruppe $-CH=R_2'$ bedeutet, in der $R_2'$ Oxo oder Imino ist, cyclisiert und gewünschtenfalls in einer verfahrensgemäss erhältlichen Verbindung, worin $R_2$ Hydroxy oder Amino bedeutet und der azacycloaliphatische Ring eine Doppelbindung aufweist, bzw. in dessen Keto- oder Iminotautomeren die Doppelbindung zur Einfachbindung bzw. die Oxo- oder Iminogruppe zur Hydroxy- oder Aminogruppe reduziert, in einer verfahrensgemäss erhältlichen Verbindung, worin $R_2$ Hydroxy ist und der azacycloaliphatische Ring keine Doppelbindung aufweist, die Hydroxygruppe zu Niederalkanoyloxy verestert und gewünschtenfalls eine verfahrensgemäss erhältliche freie Verbindung in ein Salz oder ein verfahrensgemäss erhältliches Salz in die freie Verbindung oder in ein anderes Salz umwandelt. So erhält man beispielsweise 4-Hydroxy-1-[2-(1H-indol-3-yl)ethyl]-1,2,5,6-pyridin-3-carbonsäuremethylester bzw. 1-[2-(1H-Indol-3-yl)ethyl]-piperidin-4-on-3-carbonsäuremethylester oder ein Salz davon und Isomerengemische von cis-und trans-4-Hydroxy-1-[2-(1H-indol-3-yl)ethyl]-piperidin-3-carbonsäuremethylester sowie cis- und trans-4-Acetoxy-1-[2-(1H-indol-3-yl)ethyl]-piperidin-3-carbonsäuremethylester und jeweils 5-Methoxysubstitutionsprodukte derselben und ihre Salze.

Verfahrensvariante c)

Die Umlagerung, bei der im Sinne der Fischer'schen Indolsynthese Ammoniak abgespalten wird, kann in üblicher Weise erfolgen, beispielsweise durch Säurebehandlung, d.h. Einwirkung einer geeigneten Protonen- oder Lewissäure. Als Protonensäuren kommen beispielsweise Mineralsäuren, wie Schwefelsäure, Phosphorsäure, ebenso Chlorwasserstoff in einem Niederalkanol, wie ethanolische Salzsäure, und organische Sulfonsäuren, wie Niederalkan-, z.B. Methansulfonsäure, oder gegebenenfalls substituierte Benzolsulfonsäuren, wie Benzol- oder p-Toluolsulfonsäure, aber auch organische Carbonsäuren, wie Niederalkansäuren, z.B. Essigsäure, in Betracht. Als Lewissäuren sind beispielsweise koordinativ ungesättigte Schwermetallverbindungen, wie koordinativ ungesättigte Halogenide des Bors, Aluminium, Antimons, Zinks, Kupfers, Nickels, Eisens, Chroms oder Zinns, z.B. Zinkchlorid oder Kupfer-I-chlorid bzw. -bromid, geeignet. Die Umsetzung wird vorteilhaft unter Erwärmen, z.B. im Temperatureréich von etwa 60 bis 170°C, erforderlichenfalls unter Inertgas, durchgeführt.

Zwischenprodukte IV werden vorzugsweise in situ hergestellt, indem man ein gegebenenfalls entsprechend substituiertes Phenylhydrazin der Formel $R_3$-NH-$NH_2$ (XXI) in üblicher Weise mit einer Verbindung der Formel

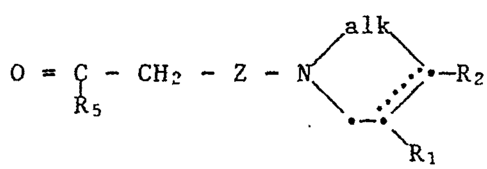

(XXII)

kondensiert und gewünschtenfalls das erhaltene Hydrazon am sekundären N-Atom durch einen von Wasserstoff verschiedenen Rest $R_4$ substituiert.

Verfahrensvariante d)

Nukleofuge Abgangsgruppen $X_3$ sind beispielsweise freie oder reaktionsfähige veresterte Hydroxygruppen, wie Hydroxy, Halogen, z.B. Chlor, Brom oder Iod, oder von Sulfonsäuren, wie Niederalkan- oder gegebenenfalls substituierten Benzolsulfonsäuren, oder Halogensulfonsäuren abgeleitete Sulfonyloxygruppen, z.B. Methansulfonyloxy, Benzolsulfonyloxy, p-Toluolsulfonyloxy, p-Brombenzolsulfonyloxy oder Fluorsulfonyloxy.

Die Umsetzung von Verbindungen V und VI erfolgt in üblicher Weise, beispielsweise in Gegenwart eines basischen Kondensationsmittels, wie einer tertiären organischen Stickstoffbase, z.B. von Pyridin oder eines Triniederalkylamins, wie von Triethylamin. Man kann aber auch Alkalimetallhydroxide oder -carbonate, z.B. Natriumhydroxid, verwenden, erforderlichenfalls in Gegenwart von Phasenübergangsvermittlern (phase transfer catalyst), z.B. von Benzyl-trimethyl-ammoniumhydroxid.

Ausgangsstoffe V können z.B. hergestellt werden, indem man ein entsprechendes Indol mit Formaldehyd und einer Halogenwasserstoffsäure, z.B. Salzsäure, umsetzt oder mit einem reaktionsfähigen Diester eines entsprechenden Niederalkandiols kondensiert bzw. mit einem Epoxyniederalkan umsetzt und das erhaltene R-Niederalkanol erforderlichenfalls, beispielsweise mittels Thionylchlorid oder Phosphortribromid, halogeniert bzw. mit einem Sulfonsäurehalogenides, z.B. p-Toluolsulfonylchlorid, umsetzt. Zur Herstellung von Verbindungen V, worin alk Ethylen ist, kann man die Indolvorstufe mittels Oxalyldichlorid in das entsprechende 3-Chloroxalylindol überführen, dieses, z.B. mit Ethanol, zum entsprechenden 3-Ethoxyoxalylindol alkoholisieren, den erhaltenen Oxalsäureester reduzieren, z.B. mittels Lithiumaluminiumhydrid in Diethylether, und das erhaltene 2-(3-Indolyl)-niederalkanol erforderlichenfalls anschliessend reaktionsfähig verestern, z.B. durch Umsetzung mit einem Halogenierungs- oder Sulfonylierungsmittel, z.B. mit Thionylchlorid, Phosphortribromid oder einem Sulfonsäurechlorid, wie p-Toluolsulfonylchlorid.

Ausgangsstoffe VI werden z.B. erhalten, indem man eine Verbindung der Formel

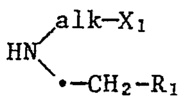

(XXIII),

worin $X_1$ eine Gruppe der Formeln $-CH=R_2'$, $-C(X_2)=R_2'$ oder Cyano darstellt, wobei $X_2$ einen abspaltbaren Rest bedeutet, oder ein Salz davon, durch Behandeln mit einem Alkalimetallniederalkanolat intramolekular cyclisiert. Man kann aber auch eine 5-Amino-3-$R_2$'-2-methylen-pentan-1-carbonsäure oder eine 2-Aminomethyl-3-$R_2$'-pent-4-en-1-carbonsäure bzw. einen Ester oder ein Amid davon intramolekular cylisieren.

Verfahrensvariante e)

Die Cyclisierung von Verbindungen VII erfolgt spontan oder durch Basenbehandlung, wobei als Basen beispielsweise Alkalimetallalkanolate, -amide oder -hydride, z.B. Natriummethanolat, Kaliumtertiärbutanolat, Natriumamid, Lithium-N,N-diisopropylamid oder Natriumhydrid, verwendet werden können. Als Lösungsmittel ist beispielsweise Dimethylformamid geeignet.

Die Zwischenprodukte VII werden vorteilhaft in situ hergestellt und ohne Isolierung erfindungsgemäss weiterumgesetzt, indem man eine entsprechende 2-Halogenmethyl-3-$R_2$'-pent-4-en-carbonsäure oder einen Ester oder ein Amid davon, in Gegenwart einer Base, wie einer der genannten, z.B. von Natriumhydrid, N-Ethyl-N,N-diisopropylamin, Natriumhydroxid oder Kaliumcarbonat, mit einer Verbindung der Formel $R-Z-NH_2$ (XVIII) umsetzt, z.B in Dimethylformamid, N-Methylpyrrolidon oder Dimethylsulfoxid.

Die dafür notwendigen Ausgangsstoffe werden z.B. erhalten, indem man eine Verbindung der Formel $R-Z-X_3$ (V; $X_3$ = Brom) mit Ammoniak umsetzt bzw. einen Acryloylessigsäureester mit einem Orthoameisensäuretriester, z.B. Trimethoxymethan, ketalisiert, das erhaltene

Ketal unter sauren Bedingungen erhitzt und den gebildeten Enolether an der 2-Methylgruppe halogeniert, z.B. mittels N-Brom-succinimid, bromiert. Man kann aber auch einen anderen 2-Methyl-3-$R_2$-penta-1,4-diencarbonsäureester bzw. 2-Methyl-3-$R_2'$-pent-4-en-carbonsäureter mit N-Bromsuccinimid bromieren.

In gemäss der Verfahrensvariante f) in gegebenenfalls verestertes oder amidiertes Carboxy überführbare Reste $R_1'$ sind beispielsweise solvolytisch in diese überführbare Reste, wie von veresterten oder amidierten Carboxy $R_1$ verschiedenes funktionell abgewandeltes Carboxy, beispielsweise anhydridisiertes Carboxy, Trihalogenmethyl, verethertes Hydroxydihalogenmethyl, z.B. Niederalkoxydichlormethyl, verethertes Trihydroxymethyl, z.B. Triniederalkoxymethyl, verethertes oder verestertes C-Hydroxyiminomethyl, z.B. C-Niederalkoxyiminomethyl, oder 2-Oxazinylgruppen, wie 4,4- oder 5,5-Dimethyloxazinyl-(2) oder 4,6,6-Trimethyldihydro-oxazinyl-(2), C-Halogeniminomethyl oder Cyano.

Die Solvolyse erfolgt in üblicher Weise, beispielsweise durch Hydrolyse, Alkoholyse, d.h. Umsetzung mit einem Alkohol oder Ammonolyse bzw. Aminolyse, d.h. Umsetzung mit Ammoniak oder einem mindestens ein Wasserstoffatom aufweisenden Amin.

Durch Hydrolyse kann man beispielsweise eines der genannten funktionellen Carboxyderivate in die Carbonsäuren der Formel I ($R_1$ = Carboxy), ferner Ortho-Anhydridester, Orthoester und Iminoether in Ester der Formel I ($R_1$ = verestertes Carboxy) und Iminoester, Amidine bzw. Nitrile in Amide ($R_1$ = amidiertes Carboxy) überführen.

Die Hydrolyse erfolgt in üblicher Weise durch Behandlung mit Wasser, erforderlichenfalls in Gegenwart eines Hydrolysemittels, in einem Lösungs- oder Verdünnungsmittel, unter Kühlen oder Erwärmen, z.B. im Temperaturbereich von etwa 0 bis 100°C, und/oder unter Inertgas, wie Stickstoff, Hydrolysemittel sind beispielsweise saure oder basische Hydrolysemittel. Saure Hydrolysemittel sind beispielsweise Protonensäuren, wie Mineralsäuren, z.B. Schwefelsäure, wie Halogen-, z.B.

Chlor-, Brom- oder Jodwasserstoffsäure, oder Phosphorsäure, Sulfonsäuren, wie p-Toluolsulfonsäure, oder Carbonsäuren, z.B. Niederalkansäuren, z.B. Ameisen- oder Essigsäure. Basische Hydrolysemittel sind beispielsweise Hydroxide oder Carbonate von Erdalkalimetallen, z.B. Natrium- oder Kaliumhydroxid, Natrium- oder Kaliumcarbonat oder Calciumhydroxid, oder Stickstoffbasen, wie Ammoniak oder organische Amine, wie Triniederalkylamine, Pyridin oder Benzyl-triäthylammoniumhydroxid. Lösungs- oder Verdünnungsmittel sind beispielsweise mit Wasser mischbare organische Lösungsmittel, wie Alkohole, z.B. Niederalkanole, Ketone, z.B. Diniederalkylketone, N,N-Diniederalkylniederalkansäureamide bzw. N-Niederalkylniederalkansäurelactame, z.B. Dimethylformamid oder N-Methylpyrrolidon, oder Diniederalkylsulfoxide, z.B. Dimethylsulfoxid.

Durch Alkoholyse kann man beispielsweise Säureanhydride, wie gemischte Anhydride mit Carbonsäuren oder anorganischen Säuren in Ester der Formel I ($R_1$ = verestertes Carboxy) überführen.

Die Alkoholyse erfolgt in üblicher Weise, erforderlichenfalls in Gegenwart eines Kondensationsmittels, in einem Lösungsmittel, unter Kühlen oder Erwärmen, z.B. im Temperaturbereich von etwa 0° bis 150°C, in einem geschlossenen Gefäss und/oder unter Inertgas, wie Stickstoff. Kondensationsmittel sind beispielsweise saure Kondensationsmittel, wie Mineralsäuren, z.B. Schwefelsäure oder Halogen-, wie Chlor-, Brom- oder Jodwasserstoffsäure, oder vor allem basische Kondensationsmittel, wie Alkalimetallalkoholate entsprechender Alkohole, wie Alkalimetallniederalkanolate, z.B. Natriummethanolat oder Natriumäthanolat, Alkalimetallhydroxide oder -carbonate, z.B. Natrium- oder Kaliumhydroxid oder Kaliumcarbonat, oder tertiäre Stickstoffbasen, wie heteroaromatische Stickstoffbasen, z.B. Pyridin, oder Triniederalkylamine, z.B. Triäthylamin. Als Lösungsmittel kommen neben einem Ueberschuss des betreffenden Alkohols bzw. der verwendeten tertiären Stickstoffbase beispielsweise Aether, wie Diniederalkyl- oder Niederalkylenäther, z.B. Diäthyläther, Tetrahydrofuran oder Dioxan, Kohlenwasserstoffe, wie aromatische oder araliphatische Kohlenwasserstoffe, z.B. Benzol, Toluol oder Xylole,

N,N-Diniederalkylniederalkansäureamide bzw. N-Niederalkylniederalkansäurelactame, z.B. Dimethylformamid oder N-Methylpyrrolidon, oder Diniederalkylsulfoxide, z.B. Dimethylsulfoxid, in Betracht.

Durch Ammono- bzw. Aminolyse kann man beispielsweise Säureanhydride, wie gemischte Anhydride mit Carbonsäuren oder Mineralsäuren, in Amide der Formel I ($R_1$ = amidiertes Carboxy) überführen.

Die Ammono- bzw. Aminolyse erfolgt in üblicher Weise, erforderlichenfalls in Gegenwart eines Kondensationsmittels, in einem Lösungsmittel, unter Kühlen oder Erwärmen, z.B. im Temperaturbereich von etwa 0 bis 150°C. In einem geschlossenen Gefäss und/oder unter Inertgas, wie Stickstoff. Kondensationsmittel sind beispielsweise basische Kondensationsmittel, wie Alkalimetallhydroxide- oder carbonate, z.B. Natrium- oder Kaliumhydroxid oder Kaliumcarbonat, oder tertiäre organische Stickstoffbasen, wie heteroaromatische Stickstoffbasen, z.B. Pyridin, oder Triniederalkylamine, z.B. Triäthylamin. Als Lösungsmittel kommen neben einem Ueberschuss eines gegebenenfalls verwendeten organischen Amins beispielsweise Aether, wie Diniederalkyl- oder Niederalkylenäther, z.B. Diäthyläther, Tetrahydrofuran oder Dioxan, Kohlenwasserstoffe, wie aromatische oder araliphatische Kohlenwasserstoffe, z.B. Benzol, Toluol oder Xylole, N,N-Diniederalkylniederalkansäureamide bzw. N-Niederalkylniederalkansäurelactame, z.B. Dimethylformamid oder N-Methylpyrrolidon, oder Diniederalkylsulfoxide, z.B. Dimethylsulfoxid, in Betracht.

Cyano $R_1$ kann ferner durch Umsetzung mit einem entsprechenden Alkohol oder einem mindestens ein Wasserstoffatom aufweisenden Amin in Gegenwart einer starken Säure, z.B. von Chlor- oder Bromwasserstoffsäure, in eine entsprechende Iminoether- bzw. Amidinogruppierung und anschliessend durch milde Hydrolyse in verestertes Carboxy bzw. substituiertes Carbamyl $R_1$ überführt werden. Die Bildung und Weiterreaktion von Iminoethergruppierungen kann aber auch <u>in situ</u>

erfolgen, beispielsweise indem man das Nitril in Gegenwart von Ammoniumchlorid mit einem geringe Mengen (etwa 1 bis 5 Vol %) Wasser enthaltenden Alkohol erhitzt.

Weitere in gegebenenfalls verestertes Carboxy überführbare Reste $R_1'$ sind beispielsweise oxidativ in dieses überführbare Reste, wie zu Carboxy $R_1$ oxidierbare, von organischen Carbonsäuren abgeleitete Acylgruppen oder die Hydroxymethylgruppe, ferner gegebenenfalls in 5-Stellung substituiertes, wie Diniederalkoxymethyl aufweisendes, 2-Furyl. Zu Carboxy oxidierbare, von organischen Carbonsäuren abgeleitetes Acyl $R_1'$ ist beispielsweise gegebenenfalls substituiertes, z.B. in 2-Stellung durch freies oder funktionell abgewandeltes Carboxy, Halogen oder Oxo und/oder in 2- und/oder 3-Stellung Hydroxy aufweisendes, in höherer als der 2-Stellung gegebenenfalls zusätzlich durch gegebenenfalls verestertes Carboxy oder eine Phenylgruppe substituiertes Niederalkanoyl, bzw. Niederalkenoyl, wie Niederalkanoyl, insbesondere gegebenenfalls in Hydratform, Enolätherform, z.B. als Enoläther mit einem Alkohol der Formel $R_o$-OH, worin $R_o$ einen Kohlenwasserstoffrest, wie Niederalkyl, bedeutet, als Acetal, z.B. Diniederalkyl- oder Niederalkylenacetal, oder Acylal, z.B. als Diniederalkanoyloxy- oder Dihalogenmethylgruppe vorliegendes Formyl, ferner Acetyl, Propionyl, Pivaloyl und dergleichen, gegebenenfalls funktionell abgewandeltes, wie verestertes oder amidiertes Oxalo, z.B. Oxalo oder Niederalkoxyoxalyl, 2-Mono-, 2,2-Di- oder 2,2,2-Trihalogenniederalkanoyl, z.B. Mono-, Di- oder Trichloracetyl, Mono-, Di- oder Tribromacetyl oder Mono-, Di- oder Trijodacetyl, 2-Oxoniederalkanoyl oder 2-Oxo-phenylniederalkanoyl, wie Pyruvoyl oder Benzoylcarbonyl, 2-Hydroxyniederalkanoyl, z.B. Glykoloyl, oder gegebenenfalls funktionell abgewandeltes, z.B. verestertes oder amidiertes 3-Carboxy-2,3-dihydroxypropionyl, wie Tartronoyl oder Niederalkoxytartronoyl, oder gegebenenfalls durch eine Phenylgruppe substituiertes Niederalkenoyl, z.B. Acryloyl, Methacryloyl, Crotonyl, Cinnamoyl und dergleichen. Weitere Gruppen $R_1$ sind veretherte Hydroxymethylgruppen, wie Niederalkoxymethyl, die zu verestertem Carboxy, wie Niederalkoxycarbonyl, $R_1$ oxidiert werden können.

Die Oxidation erfolgt in üblicher Weise durch Umsetzung mit einem geeigneten Oxidationsmittel, vorteilhaft in einem Lösungs- oder Verdünnungsmittel, erforderlichenfalls unter Kühlen oder Erwärmen z.B. bei etwa 0 bis 100°C, in einem geschlossenen Gefäss und/oder unter Inertgas, wie Stickstoff. Geeignete Oxidationsmittel sind beispielsweise Sauerstoff, vorzugsweise in Gegenwart eines Katalysators, wie einer Silber-, Mangan-, Eisen- oder Kobaltverbindung, Perverbindungen, wie Wasserstoffperoxid, Metallperoxide, z.B. Nickelperoxid, Perkohlensäure und ihre Salze oder organische Persäuren, z.B. m-Chlorperbenzoesäure, Phthalmonopersäure oder Peressigsäure, oder ihre Salze, oxidierende Sauerstoffsäuren oder deren Salze oder Anhydride, wie unterhalogenige Säuren und ihre Salze, z.B. Natriumhypochlorit, Halogensäuren und ihre Salze, z.B. Jodsäuren, Perjodsäuren, Kaliumjodat, Natriumperjodat oder Kaliumchlorat, Salpetersäure bzw. salpetrige Säure und deren Salze und Anhydride, z.B. Kaliumnitrat, Natriumnitrit, Stickoxid, Distickstofftrioxid oder Stickstoffdioxid, oder oxidierende Schwermetallverbindungen, wie Chrom-VI-, Chrom-IV, Mangan-IV-, Mangan-VII-, Silber-II-, Kupfer-II-, Quecksilber-II-, Vanadium-V- oder Wismut-II-verbindungen, z.B. Natriumdichromat, Kaliumdichromat, Chromtrioxid, Mangandioxid, Kaliumpermanganat, Silber-II-oxid, Kupfer-II-oxid, Quecksilberoxid oder Wismutoxid. Inerte Lösungsmittel sind beispielsweise gegenüber dem jeweils zur Anwendung gelangenden Oxidationsmittel inerte Lösungsmittel, wie Wasser, Ketone, z.B. Aceton, Carbonsäuren und ihre Anhydride, z.B. Essigsäure oder Acetanhydrid, Halogenkohlenwasserstoffe, z.B. Tetrachlormethan, oder Aromaten oder Heteroaromaten, z.B. Benzol, oder Pyridin, oder deren Gemische.

Gegebenenfalls verestertes Oxalo $R_1'$ der Formel $-C(=O)-R_1$ kann ferner unter Decarbonylierung, beispielsweise in Gegenwart eines geeigneten Oxidationsmittels, erforderlichenfalls unter Kühlen oder Erwärmen, z.B. bei etwa 0 bis 100°C, in einem inerten Lösungsmittel, in einem geschlossenen Gefäss und/oder unter Inertgas, wie Stickstoff, oxidiert werden. Geeignete Oxidationsmittel sind beispielsweise Perverbindungen, wie Wasserstoffperoxid oder Percarbonsäuren, z.B. Perkohlensäure oder organische Percarbonsäuren, u.a. Peressigsäure, Perameisensäure, Perbenzoesäure und dergleichen, ferner Halogensauerstoffsäuren und ihre Salze, wie Chlorsäure bzw. Alkalimetallchlorite, oder oxidierende Metallverbindungen, wie Silberoxid, Kaliumpermanganat oder Chromtrioxid. Geeignete Lösungsmittel sind beispielsweise gegebenenfalls wasserhaltige Ketone, wie Aceton, oder Carbonsäuren, wie Essigsäure.

Die als Ausgangsstoffe genannten Verbindungen der Formel VIII können beispielsweise hergestellt werden, indem man Verbindungen der Formeln

$R - Z - X_3$ (V) und 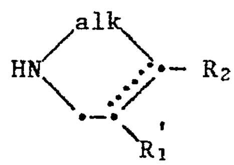 (VIb),

worin $X_3$ reaktionsfähiges verestertes Hydroxy, z.B. Halogen, wie Chlor, Brom oder Jod, bedeutet miteinander kondensiert oder eine Verbindung der Formel

R' - Z - 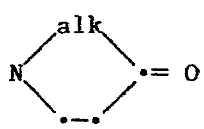 (XXIV),

worin R' einen in 1-Stellung substituierten oder intermediär geschützten 3-Indolylrest R bedeutet, nach Umsetzung mit einer Metallbase, wie einem Alkalimetallalkanolat, -hydrid oder -amid, z.B. mit Natriummethanolat, Natriumhydrid, Natriumamid oder Lithiumdiisopropylamid, mit Phosgen, einem Niederalkanoyloxycarbonylchlorid, Tetrachlormethan, Niederalkoxytrihalogenmethan, Triniederalkoxyhalogenmethan, Dicyan bzw. einem Halogencyan, einem Acylhalogenid oder Diacyloxid der Formel $R_1$'-Halogen (XXVa) bzw. $R_1$'-O-$R_1$' (XXVb), mit Formaldehyd oder einem Halogenmethoxyniederalkan zur Reaktion bringt. Intermediär geschützte Reste R' sind beispielsweise an sich 1-unsubstituierte und/oder in 4- bis 7-Stellung Hydroxy aufweisende, in 1-Stellung bzw. an der Hydroxygruppe

durch übliche Schutzgruppen, beispielsweise durch veresterte oder veretherte Hydroxymethylgruppen, wie Pivaloyloxymethyl, Methoxymethyl, 2-Chlorethoxymethyl oder Benzyloxymethyl, Tetrahydropyranyl oder Triniederalkylsilyl, wie Trimethylsilyl, intermediär geschützte 3-Indolylreste R. Die Schutzgruppe wird beispielsweise durch Umsetzung der zu schützenden Verbindung VI mit einem entsprechenden Halogenderivat bzw. mit Chlorjodmethan ($Cl-CH_2I$), einem Alkalimetall-, z.B. Natriumpivalat, -methanolat, -1,2-dichlorethanolat oder -benzylalkoholat und bzw. mit Dihydropyran, eingeführt.

Man kann aber auch Verbindungen der Formeln

$R' - X_5$ (XXVI) und $X_6 - Z -$ 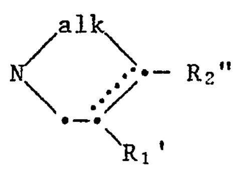 (XXVII),

worin einer der Reste $X_5$ und $X_6$ einen metallischen Rest, z.B. der Formel $-M^{I}$, $-M^{II/2}$ oder $-M^{II}$-Hal, worin $M^{I}$ ein Alkalimetallradikal, z.B. Lithium, $M^{II}$ ein Erdalkalimetallradikal, z.B. Magnesium, Cadmium oder Zink und Hal für Halogen, wie Chlor, Brom oder Jod steht, der andere für reaktionsfähiges verestertes Hydroxy, z.B. für Halogen, wie Chlor, Brom oder Iod, oder für organisches Sulfonyloxy, wie Niederalkan- bzw. gegebenenfalls substituiertes Benzolsulfonyloxy, z.B. Methan-, Benzol- oder p-Toluolsulfonyloxy, steht, $R_2''$ verethertes oder geschütztes Hydroxy oder geschütztes Amino, z.B. verethertes Hydroxy $R_2$, Silyloxy, wie Triniederalkylsilyloxy, z.B. Trimethylsilyloxy, aber auch Triphenylniederalkoxy, z.B. Trityloxy, oder Silylamino, wie Triniederalkylsilylamino, z.B. Trimethylsilylamino, kann aber auch Phenyl-, Diphenyl- oder Triphenylniederalkylamino, wie Benzylamino, Diphenylmethylamino oder Tritylamino, und $R_1'$ insbesondere verethertes Trihydroxymethyl, z.B. Triniederalkoxymethyl, eine 2-Oxazinylgruppe oder gegebenenfalls in 5-Stellung substituiertes 2-Furyl, bedeutet, miteinander umsetzen und die Schutzgruppe(n) abspalten.

0187122

Verbindungen VIII, worin $R_1$' Cyano bedeutet, können ferner hergestellt werden, indem man eine Verbindung der Formel

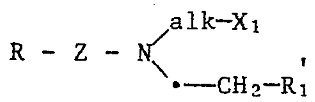

(XXVIII),

in der $X_1$ eine Gruppe der Formeln $-CH=R_2'$ oder $-C(X_2)=R_2'$, wobei $X_2$ einen abspaltbaren Rest, z.B. Niederalkoxy oder Halogen bedeutet, darstellt oder für Cyano steht, in Gegenwart einer Metallbase, z.B. eines Alkalimetallalkoholates, wie von Natriummethanolat bzw. -ethanolat, cyclisiert. Dieses Verfahren ist besonders geeignet zur Herstellung von Verbindung VIII, worin der azacyclische Ring eine Doppelbindung aufweist, $R_1$' Cyano und $R_2$ Amino ist, wozu man von Verbindungen XXVIII ausgeht, worin $X_1$ und $R_1$' beide für Cyano stehen, sowie zur Herstellung von Verbindungen VIII, worin der azacycloaliphatische Rest keine Doppelbindung aufweist, $R_1$' Formyl oder Cyano und $R_2$ Hydroxy bedeutet, wozu man von Verbindungen XXVIII ausgeht, worin $X_1$ für Formyl und $R_1$' für Formyl oder Cyano steht. Aus nach der erstgenannten Bildungweise erhältlichen Verbindungen VIII, worin $R_2$ Amino und $R_1$' Cyano bedeutet, können durch milde Hydrolyse die entsprechenden Verbindungen VIII, worin $R_2$ Hydroxy und $R_1$' Cyano ist, und aus diesen durch Umsetzung mit Ammonchlorid oder Chlorwasserstoff und einem Alkohol die entsprechenden Verbindungen VIII, worin $R_2$ gegebenenfalls verethertes Hydroxy und $R_1$' veretherte C-Hydroxyiminomethyl ist, hergestellt werden, die in situ unter Hydrolyse von $R_1$' zu verestertem Carboxy $R_1$ weiterreagieren.

In Ausgangsstoffen VIa gemäss der Verfahrensvariante g) sind abspaltbare Reste beispielsweise Halogenatome oder Sulfonyloxygruppen, z.B. Benzol- oder p-Toluolsulfonyloxy. Als Verbindungen VIa kommen beispielsweise Halogenkohlensäureester bzw. Carbamoylhalogenide der Formel $R_1$-Halogen in Betracht. Geschütztes Hydroxy $R_2$"' hat beispielsweise die unter der Verfahrensvariante a) angegebene Bedeutung, ebenso in 1-Stellung geschütztes 3-Indolyl.

Die Umsetzung erfolgt beispielsweise in Gegenwart eines basischen Kondensationsmittels, wie eines Alkoholates, Amides oder Hydrides eines Alkalimetalls oder Erdalkalimetalls, z.B eines Alkalimetall- oder Erdalkalimetallalkoholates, z.B. von Natriummethanolat, Magnesiummethanolat, Natriumethanolat oder Kaliumtertiärbutanolat, eines Alkalimetallamides, z.B. von Natriumamid, Lithiumdiisopropylamid, Natrium-N-methylanilid oder Natriumdiphenylamid, oder eines Alkalimetall- oder Erdalkalimetallhydrides, z.B. von Natriumhydrid oder Calciumhydrid, erforderlichenfalls unter Kühlen oder Erwärmen, z.B. bei etwa -10°C bis +120°C, und/oder unter Inertgas, wie Stickstoff.

Die Ausgangsstoffe IX können beispielsweise erhalten werden, indem man eine Verbindung der Formel $R-Z-X_3$ (V; $X_3$ = Halogen), mit einer Verbindung der Formel

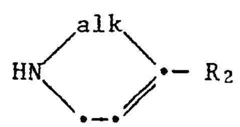

(XXIX)

oder einem Tautomeren davon, z.B. mit 4-Piperidon, kondensiert. Dabei kann der Rest R und/oder Hydroxy $R_2$ in geschützter Form vorliegen oder vor Umsetzung mit der Reaktionskomponente geschützt, z.B. zu Trimethylsilyloxy silyliert, werden.

Verfahrensvariante h)

In $R_2$ überführbare Rest $X_4$ sind beispielsweise durch Solvolyse, d.h. Umsetzung mit einer Verbindung der Formel $R_2H$ (XIII) oder einem Salz davon, in eine Gruppe $R_2$ überführbare Reste, beispielsweise reaktionsfähige veresterte Hydroxygruppen, wie Halogenatome, z.B. Chlor, Brom oder Jod. In Hydroxy überführbare Reste $X_4$ sind ferner Diazoniumgruppen, z.B. der Formel $-N_2^{\oplus}A^{\ominus}$, worin $A^{\ominus}$ das Anion einer starken Säure, wie einer Mineralsäure, z.B. das Chlorid- oder Sulfation, bedeutet, oder veretherte bzw. acylierte Hydroxygruppen $R_2$ bzw. gegebenenfalls acylierte Aminogruppen $R_2$.

Die Solvolyse erfolgt in üblicher Weise, beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triethylamin, oder einer heteroaromatischen Stickstoffbase, wie Pyridin, bzw. eines quaternären Ammoniumhydroxides, wie Benzyl-trimethyl-ammoniumhydroxid, oder indem man die Verbindung XIII in Form eines Metallsalzes, z.B. der Formel $R_2^{\ominus}M^{\oplus}$ (XIIIa) einsetzt, worin $M^{\oplus}$ ein Alkalimetallkation, wie das Natriumion, bedeutet. Vorteilhaft arbeitet man in Gegenwart eines Lösungs- oder Verdauungsmittels, z.B. in einem Ueberschuss der Reaktionskomponente XIII und/oder einem mit diesen mischbaren inerten Lösungsmittel, erforderlichenfalls unter Kühlen oder Erwärmen, z.B. im Temperaturbereich von etwa 0° bis 120°C, und/oder unter Inertgas, wie Stickstoff.

Verbindungen X können beispielsweise durch Umsetzungen von Verbindungen der Formeln

$R - Z - X_3$ (V) und 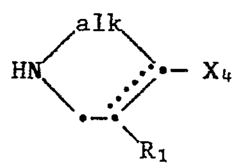 (XXX),

worin $X_3$ reaktionsfähiges verestertes Hydroxy, z.B. Halogen bedeutet, in üblicher Weise, beispielsweise in Gegenwart eines basischen Kondensationsmittels, wie einer tertiären organischen Stickstoffbase, z.B. von Pyridin oder eines Triniederalkylamins, wie von Triethylamin, hergestellt werden.

Verbindungen X, worin $X_4$ Halogen bedeutet, erhält man ferner, indem man an eine Verbindung der Formel

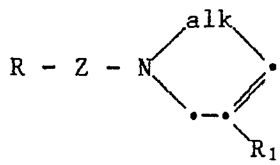

(XII)

Halogen oder Halogenwasserstoff anlagert und im erstgenannten Fall wieder Halogenwasserstoff abspaltet.

Verbindungen X, worin $X_4$ eine Diazoniumgruppe bedeutet, werden z.B. hergestellt, indem man eine Verbindung der Formel I, worin $R_2$ Amino ist, diazotiert, z.B. durch Umsetzung mit salzpetriger Säure oder mit Ammonium- oder einem Alkalimetallnitrit, z.B. mit Natriumnitrit, in Gegenwart einer Mineralsäure der Formel HA.

Verfahrensvariante i)

Die Umlagerung von Verbindungen XI zu Verbindungen der Formel I, worin der azacycloaliphatische Ring eine Doppelbindung aufweist und $R_2$ Hydroxy ist, bzw. zu deren Tautomeren der Formel I', worin $R_2'$ für Oxo steht, erfolgt beispielsweise durch Säurebehandlung, insbesondere Behandlung mit einer Lewissäure, d.h. einem koordinativ ungesättigten Halogenid eines Elementes der Gruppen IIb, IIIb oder Va des Periodischen Systems der Elemente, z.B. mit Zinkchlorid, Aluminiumtrichlorid, Bortrifluorid oder Antimonpentachlorid, vorzugsweise mit einem Bortrifluoridetherat, z.B. dem Komplex von Bortrifluorid mit Diethylether (Bortrifluorid-Etherat), kann aber auch durch Behandeln mit einer starken Protonensäure, wie einer Mineralsäure, z.B. mit Schwefel- oder Polyphosphorsäure, oder mit p-Toluolsulfonsäure in Benzol, bewirkt werden. Erforderlichenfalls arbeitet man in Gegenwart eines inerten Lösungsmittels und/oder unter Kühlen oder Erwärmen, z.B. im Temperaturbereich von etwa 0° bis 120°C, und/oder unter Inertgas, wie Stickstoff.

Verbindungen XI werden beispielsweise hergestellt, indem man eine Verbindung der Formel

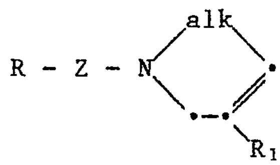

(XII),

in üblicher Weise, beispielsweise durch Umsetzung mit einer Peroxyverbindung, wie mit Wasserstoffperoxid oder insbesondere mit einer organischen Hydroperoxidverbindung, z.B. mit Tertiärbutylhydroperoxid, Peressigsäure, Perbenzoesäure, m-Chlorperbenzoesäure, Permonophthalsäure oder dergl., epoxidiert.

<u>Verfahrensvariante j)</u>

Die Umsetzung von Verbindungen XII und XIII erfolgt in üblicher Weise beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triethylamin, oder einer heteroaromatischen Stickstoffbase, wie Pyridin, bzw. eines quaternären Ammoniumhydroxides, wie Benzyl-trimethyl-ammoniumhydroxid, oder indem man die Verbindung XIII in Form eines Metallsalzes, z.B. der Formel $R_2^{\ominus}M^{\oplus}$ (XIIIa) einsetzt, worin $M^{\oplus}$ ein Alkalimetallkation, wie das Natriumion, bedeutet. Vorteilhaft arbeitet man in Gegenwart eines Lösungs- oder Verdünnungsmittels, z.B. in einem Ueberschuss der Reaktionskomponente XIII und/oder einem mit diesen mischbaren inerten Lösungsmittel, erforderlichenfalls unter Kühlen oder Erwärmen, z.B. im Temperaturbereich von etwa 0° bis 120°C, und/oder unter Inertgas, wie Stickstoff.

Ausgangsstoffe XII werden beispielsweise erhalten, indem man Verbindungen der Formeln

$R - Z - X_3$ (V) und 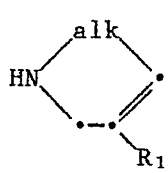 (XXXI),

worin $X_3$ reaktionsfähiges verestertes Hydroxy, z.B. Halogen, bedeutet, in üblicher Weise untereinander kondensiert, beispielsweise in Gegenwart eines basischen Kondensationsmittels, wie einer tertiären organischen Stickstoffbase, z.B. von Pyridin oder eines Triniederalkylamins, wie von Triethylamin.

Verfahrensvariante k)

Oxoniederalkane, die sich für die Einführung des Restes Z eignen, sind beispielsweise Niederalkanale, d.h. 1-Oxoniederalkane, können aber auch Diniederalkylketone, z.B. 2-Oxoniederalkane, sein. Die Umsetzung derselben mit Verbindungen der Formeln L und VI erfolgt in üblicher Weise, beispielsweise in Gegenwart einer Protonensäure, wie einer Mineralsäure, z.B. von Salzsäure, oder einer Carbonsäure, z.B. von Essigsäure. Dabei wird intermediär wahrscheinlich die entsprechende Verbindung der Formel R-Z-$X_3$ (V), worin $X_3$ Hydroxy bedeutet, gebildet, die dann ohne Isolierung erfindungsgemäss mit der Reaktionskomponente VI weiterreagiert. Die Verfahrensvariante ist somit als bevorzugte Ausführungsform der Verfahrensvariante d) zu betrachten.

In Ausgangsstoffen können, wie erwähnt, Gruppen R, aber auch Gruppen $R_1$ und/oder $R_2$ bzw. $R_2'$ in intermediär geschützer Form vorliegen.

Intermediär geschützte Reste R sind beispielsweise an sich 1-unsubstituierte und/oder in 4- bis 7-Stellung Hydroxy aufweisende, in 1-Stellung bzw. an der Hydroxygruppe durch übliche Schutzgruppen, beispielsweise durch gegebenenfalls substituierte Benzyl- oder Benzyloxycarbonylgruppen, veresterte oder veretherte Hydroxymethylgruppen, wie Pivaloyloxymethyl, Methoxymethyl, 2-Chlorethoxymethyl oder Benzyloxymethyl, Tetrahydropyranyl oder Triniederalkylsilyl, wie Trimethylsilyl, intermediär geschützte 3-Indolylreste R. Die Schutzgruppe wird beispielsweise durch Umsetzung der zu schützenden Verbindung mit einem entsprechenden Halogenderivat bzw. mit Chlorjodmethan ($Cl-CH_2I$), einem Alkalimetall-, z.B. Natriumpivalat, -methanolat, -1,2-dichlorethanolat oder -benzylalkoholat und bzw. mit Dihydropyran, eingeführt. Analog können Hydroxy- bzw. Amino-

gruppen $R_2$ oder die primäre Aminogruppe in Ausgangsstoffen, z.B. in Verbindungen XVIII, beispielsweise durch gegebenenfalls substituierte Benzyl- oder Benzyloxycarbonylgruppen, veresterte oder veretherte Hydroxymethylgruppen, wie Pivaloyloxymethyl, Methoxymethyl, 2-Chlorethoxymethyl oder Benzyloxymethyl, Tetrahydropyranyl oder Triniederalkylsilyl, wie Trimethylsilyl, intermediär geschützt sein. Geschütztes Hydroxy ist beispielsweise Silyloxy, wie Triniederalkylsilyloxy, z.B. Trimethylsilyloxy, kann aber auch Triphenylniederalkoxy, z.B. Trityloxy sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylmethylamino oder Tritylamino sein. Intermediär geschützte Reste $R_1$ sind beispielsweise als cyclische Iminoether geschützte Carboxygruppen, insbesondere gegebenenfalls niederalkyliertes 4,5-Dihydro-oxazolyl(2) bzw. 5,6-Dihydro-oxazinyl(2), wie 4,4- oder 5,5-Dimethyl-4,5-dihydro-oxazolyl-(2), bzw. 4,4,6-Trimethyl-5,6-dihydro-oxazinyl-(2). Die in einer dieser Formen geschützte Gruppe $R_1$ wird in üblicher Weise gebildet, ausgehend von Carboxy, z.B. durch Umsetzung mit Aminoisobutanol (3-Amino-2-methylpropan-2-ol) oder 4-Amino-4-methyl-pentan-2-ol oder durch Umsetzung mit 2,2-Dimethylaziridin und anschliessende Säurebehandlung und ausgehend von Cyano durch Umsetzung mit 2-Methyl-propan-1,2-diol, 4-Amino-2-methyl-pentan-2-ol oder 2-Methyl-pentan-2,4-diol.

Die Freisetzung intermediär geschützter Reste, d.h. Abspaltung der intermediären Schutzgruppen, erfolgt in üblicher Weise, beispielsweise durch Hydrogenolyse, z.B. in Gegenwart von Platin- oder Palladiumkatalysatoren, bzw. Solvolyse, wie milde Hydrolyse, z.B. Behandlung mit Wasser unter neutralen oder schwach sauren Bedingungen, z.B. durch Einwirkung von verdünnt-wässriger Mineral- oder Carbonsäuren, z.B. von verdünnter Salz- oder Essigsäure. Bei der Hydrolyse von Dihydro-oxazolyl- und Dihydro-oxazinylgruppen werden dabei freie Carboxygruppen $R_1$ gebildet.

Verfahrensgemäss erhältliche Verbindungen können in üblicher Weise in andere Verbindungen der Formel I überführt werden.

So kann man veresterte oder amidierte Carboxygruppen in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy hydrolysieren. Veresterte Carboxygruppen können ferner durch Umesterung, d.h. Behandlung mit einem Alkohol in Gegenwart eines sauren oder basischen Solvolysemittels, wie einer Mineralsäure, z.B. von Schwefelsäure, bzw. eines entsprechenden Alkalimetallalkoholates oder eines Alkalimetallhydroxides, in andere veresterte Carboxygruppen $R_1$ oder durch Umsetzung mit Ammoniak oder einem mindestens ein Wasserstoffatom aufweisenden Amin in amidiertes Carboxy überführt werden.

Freies Carboxy $R_1$ kann in üblicher Weise, beispielsweise durch Behandlung mit einem entsprechenden Alkohol in Gegenwart einer Mineralsäure, z.B. von Schwefelsäure, oder durch Ueberführung in ein Halogenid und anschliessender Umsetzung mit einem entsprechenden Alkohol, z.B. in Gegenwart von Pyridin oder Triethylamin, oder durch Ueberführung in ein Alkalimetallsalz und anschliessender Umsetzung mit einem reaktionsfähigen Ester des entsprechenden Alkohols, wie einem entsprechenden Halogenid, in verestertes Carboxy überführt werden. Freies oder verestertes Carboxy kann auch durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels N,N-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy überführt werden.

Ferner kann man veretherte oder acylierte Hydroxygruppen $R_2$ in Hydroxy und acyliertes Amino $R_2$ in freies Amino überführen, beispielsweise durch Hydrolyse, vorteilhaft in Gegenwart eines geeigneten Hydrolysemittels. Solche sind beispielsweise saure Hydrolysemittel, wie Protonensäuren, z.B. Mineralsäuren, wie Chlorwasserstoffsäure oder Schwefelsäure, für die Hydrolyse einer acylierten Hydroxy- bzw. Aminogruppe ebenfalls basische Hydrolysemittel, wie Alkalimetallhydroxide oder -carbonate, z.B. Natriumhydroxid oder Kaliumcarbonat. In analoger Weise kann man auch in Verbindungen I, worin der azacycloaliphatische Rest eine Doppelbindung aufweist und $R_2$ Amino ist, bzw. in deren Tautomeren (I', $R_2$ = Imino) die Amino- bzw. Iminogruppe hydrolytisch oder durch Behandeln mit einem Alkalimetallnitrit durch Hydroxy bzw. Oxo ersetzen, insbesondere unter sauren Bedingungen. Die Freisetzung von Hydroxy aus acyliertem Hydroxy $R_2$ kann aber auch durch Umesterung, z.B. durch Behandeln mit einem Alkoholat, z.B. Alkalimetallniederalkanolat, oder einem Alkohol, z.B. Niederalkanol, in Gegenwart einer der genannten Protonensäuren oder eines entsprechenden Alkalimetallalkoholates, erfolgen. α-Phenylniederalkoxygruppen $R_2$ können auch hydrogenolytisch, z.B. durch Behandlen mit Wasserstoff in Gegenwart eines Hydrierungskatalysators, wie von Palladiumkohle, in Hydroxy überführt werden.

Umgekehrt kann man Hydroxy $R_2$ verethern bzw. Hydroxy oder Amino $R_2$ acylieren. Die Veretherung erfolgt beispielsweise mit einem Veretherungsmittel, wie einem reaktionsfähigen Ester, z.B. einem Halogenwasserstoffsäureester, des entsprechenden Alkohols der Formel $R_2H$ (XIII), z.B. einem Niederalkylhalogenid oder Diniederalkylsulfat, oder einem entsprechenden Oxoniumsalz, z.B. einem Triniederalkyl-oxoniumtetrafluorborat oder -pentachloroantimonat. Die Acylierung erfolgt beispielsweise durch Umsetzung mit einem von der entsprechenden Carbonsäure der Formel $R_2H$ (XIII) abgeleiteten Anhydrid, z.B. der Formel $R_2$-O-$R_2$ (XIIIb) oder $R_2$-Halogen (XIIIc), vorteilhaft in Gegenwart eines basischen Kondensationsmittels, z.B. eines Alkalimetallhydroxides oder -carbonates oder einer tertiären Stickstoffbase, z.B. von Pyridin oder eines Triniederalkylamins, wie Triethylamin.

Weiterhin kann man in Verbindungen I, worin der azacycloaliphatische Ring eine Doppelbindung aufweist, diese zur Einfachbindung reduzieren, beispielsweise durch Behandeln mit Wasserstoff in Gegenwart eines Hydrierungskatalysators, wie eines Platin-, Palladium- oder Nickelkatalysators, z.B. von Platinoxid, oder durch Umsetzung mit einem Dileichtmetallhydrid, z.B. mit Natriumborhydrid oder Natriumcyanoborhydrid. Ausgehend von einer entsprechenden Hydroxyverbindung kann die Reduktion der Doppelbindung auch durch Umsetzung mit einem sekundären Alkohol, z.B. mit Isopropanol oder Cyclohexanol, in Gegenwart eines Aluminiumalkoholates, z.B. von Aluminiumsopropanolat, bewirkt werden. Dabei reagiert die Verbindung I aus der tautomeren Oxoform (I',$R_2$' = Oxo). Umgekehrt kann man eine Verbindung I, worin $R_2$ Hydroxy ist und der azacycloaliphatische Ring keine Doppelbindung aufweist, durch Umsetzung mit einem Keton, z.B. mit Aceton oder Cyclohexanon, in Gegenwart eines entsprechenden Aluminiumalkoholates, zu der entsprechenden ungesättigten Verbindung bzw. deren Tautomeren (I',$R_2$ = Oxo) oxidieren.

Auch kann man Oxo $R_2$' durch Umsetzung mit Ammoniak, z.B. in Methanol, in Amino überführen.

Ferner kann man in 1-Stellung unsubstituierte 3-Indolylreste R in üblicher Weise durch einen der eingangs genannten 1-Substituenten substituieren, beispielsweise durch Umsetzung mit einem reaktionsfähigen Ester eines aliphatischen Alkohols, vorzugsweise in Gegenwart einer Base, wie eines Alkalimetallhydroxides, -niederalkanolats oder -hydrides. In analoger Weise kann man auch Hydroxy $R_2$ verethern. Hydroxy bzw. Amino $R_2$ kann ferner acyliert werden z.B. durch Umsetzung mit einem Anhydrid oder Halogenid einer organischen Carbonsäure, vorzugsweise in Gegenwart einer Base, wie eines Alkalimetallhydroxides, -niederalkanolats oder -hydrides, organischer Carbonsäuren auch in Gegenwart einer organischen Stickstoffbase. In analoger Weise kann ferner auch Carbamyl N-acylieren.

In analoger Weise wie R kann man auch Carbamyl $R_1$ aliphatisch N-substituieren, ferner aliphatisch N-monosubstituiertes Carbamyl in aliphatisch N,N-disubstituiertes Carbamyl überführen.

Ferner kann man im Rest R vorhandene Hydroxygruppen verestern, z.B. durch Behandeln mit einem Niederalkancarbonsäureanhydrid bzw. -halogenid in Niederalkanoyloxy oder durch Umsetzung mit einem reaktionsfähigen Ester, insbesondere Brom- oder Chlorwasserstoffsäureester, eines Niederalkanols bzw. Niederalkenols in verethertes Hydroxy überführen. Zwei an vicinale C-Atome gebundene Hydroxygruppen können auch durch Umsetzung mit einem reaktiven Diester eines Niederalkandiols, z.B. einem Dihalogenniederalkan, in Niederalkylendioxygruppierungen bzw. durch Umsetzung mit einem Oxoniederalkan, d.h. Niederalkanals oder Diniederalkylketon, in Niederalkylidendioxygruppierungen überführt werden. Umgekehrt kann man in veresterten oder veretherten Hydroxygruppen, wie Niederalkanoyloxy, Niederalkoxy oder Niederalkyl(id)endioxy, die Hydroxygruppe(n) solvolytisch freisetzen, vorzugsweise unter sauren Bedingungen.

Die neuen Verbindungen können, je nach Wahl der Ausgangsstoffe und Arbeitsweisen, in Form eines der möglichen Isomeren, z.B. je nach der Anzahl der asymmetrischen Kohlenstoffatome als optische Isomere, wie in Form eines Enantiomeren, wie Antipoden bzw. Diastereomeren oder als Gemische derselben, wie Enantiomerengemische, z.B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines in 1-Stellung und/oder an der Hydroxy- bzw. Aminogruppe $R_2$ unsubstituierten oder in mindestens einer der Stellungen 4 bis 7 durch Hydroxy substituierten Endstoffes I mit einer optisch aktiven Säure oder einem Anhydrid davon bzw. einem reaktionsfähigen Ester eines optisch aktiven Alkohols oder durch Umsetzung eines Esters

oder einer Säure I ($R_1$ = gegebenenfalls verestertes Carboxy) mit einem mit der racemischen Säure Ester bildenden optisch aktiven Alkohol und jeweils Trennung des erhaltenen diastereomeren Substitutionsproduktes bzw. Esters, z.B. auf Grund ihrer verschiedenen Löslichkeiten, in die Diastereomeren, aus denen die Enantiomeren durch Einwirkung geeigneter Mittel freigesetzt werden können. Racemate salzbildender Verbindungen der Formel I können auch durch Umsetzung mit einer optisch aktiven Hilfsverbindung in Diastereomerengemische, z.B. mit einer optisch aktiven Säure bzw. Base in Gemische diastereomerer Salze, und Trennung derselben in die Diastereomeren, aus denen die Enantiomeren in der jeweils üblichen Weise freigesetzt werden könne, gespalten werden.

Für diesen Zweck übliche optisch aktive Basen bzw. Säuren sind z.B. Strichnin, Cinchonin, optisch aktive Alkaloidbasen, wie Brucin, oder l-Phenylethylamin, 3-Pipecolin, Ephedrin, Amphetamin und ähnliche synthetisch zugängliche, optisch aktive Basen, bzw. optisch aktive Carbon- oder Sulfonsäuren, wie D- oder L-Weinsäure, Di-o-Toluylweinsäure, Aepfelsäure, Mandelsäure, Camphersulfonsäure oder Chinasäure.

Ferner können erhaltene freie salzbildende Verbindungen in an sich bekannter Weise in Salze überführt werden, z.B. durch Umsetzen einer Lösung der freien Verbindung in einem geeigneten Lösungsmittel oder Lösungsmittelgemisch mit einer entsprechenden Base bzw. Säure oder mit einem geeigneten Ionenaustauscher.

Erhaltene Salze können in an sich bekannter Weise in die freien Verbindungen umgewandelt werden, Säureadditionssalze z.B. durch Behandeln mit einer Base, wie einem Alkalimetallhydroxid, einem Metallcarbonat oder -hydrogencarbonat, oder Ammoniak, und Basesalze z.B. durch Umsetzung mit einer Säure, z.B. mit Salzsäure.

Erhaltene Salze können in an sich bekannter Weise in andere Salze überführt werden, Säureadditionssalze z.B. durch Behandlung eines Salzes einer organischen Säure mit einem geeigneten Metallsalz, wie einem Natrium-, Barium- oder Silbersalz, einer Säure in einem

geeigneten Lösungsmittel, in welchen ein sich bildendes anorganisches Salz unlöslich ist und damit aus dem Reaktionsgemisch ausscheidet.

Die Verbindungen, einschliesslich ihre Salze, können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter der Reaktionsbedingungen bildet.

So können Zwischenprodukte der Formeln III, VII bzw. VIII gemäss der Verfahrensvariante e) bzw. f) <u>in situ</u> gebildet und ohne Isolierung weiterumgesetzt werden.

Neue Ausgangsstoffe, z.B. der Formeln II, III, VIII, IX, X, XII, XVI, XXVII und XXVIII, die speziell für die Herstellung der erfindungsgemässen Verbindungen entwickelt wurden, insbesondere die zu den eingangs als bevorzugt gekennzeichneten Verbindungen der Formeln I führende Ausgangsstoffauswahl, die Verfahren zu ihrer Herstellung sowie ihre Verwendung als Zwischenprodukte, bilden ebenfalls einen Gegenstand der Erfindung.

In diesem Zusammenhang sind insbesondere Verbindungen der Formel XVI zu nennen, die ähnliche nootrope Eigenschaften aufweisen, wenn auch in etwas geringerer Wirkungsstärke, wie die Verbindungen der

Formel I und neben ihrer Verwendung als Zwischenprodukte zur Herstellung derselben als Arzneimittelwirkstoffe zur Behandlung zerebraler Leistungsinsuffizienz, insbesondere von Gedächtnisstörungen unterschiedlicher Genese, wie senile Demenz oder Demenz vom Alzheimer-Typ, ferner von Folgezuständen von Hirntraumata und Apoplexien, Verwendung finden können.

Die Erfindung betrifft demgemäss als weiteren Erfindungsgegenstand Verbindungen der Formel

$$R - Z - \overset{R_6}{\overset{|}{N}} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1$"', wobei $R_1$"' eine der für $R_1$ angegebenen Bedeutungen hat oder für Cyano, Hydroxymethyl oder gegebenenfalls acetalisiertes Formyl, wie Diniederalkoxy- oder Niederalkylendioxymethyl, z.B. Dimethoxy- oder Diethoxymethyl, Dioxolan-2-yl oder 1,3-Dioxan-2-yl, steht und alk Methylen, Ethylen oder 1,3-Propylen darstellt, und ihre pharmazeutisch verwendbaren Salze zur Anwendung in einem Verfahren zur Behandlung des menschlichen oder tierischen Körpers, diese enthaltende pharmazeutische Präparate und ihre Verwendung als Arzneimittelwirkstoffe, ebenso Verbindungen der Formel XVI, worin R, Z, $R_1$ und $R_6$ vorstehende Bedeutung haben, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze sowie Verfahren zu ihrer Herstellung.

Als Substituenten des 3-Indolylrestes kommen dabei beispielsweise die für Verbindungen der Formel I genannten in Betracht. Ebenso haben Z, alk und $R_1$ beispielsweise die für Verbindungen der Formel I

angegebenen bevorzugten Bedeutungen und sind Salze beispielsweise Säureadditions- bzw. Basesalze des gleichen Typs wie für Verbindungen der Formel I angegeben.

Die Erfindung betrifft beispielsweise Verbindungen der Formel XVI, worin R, Z und $R_1$ die angegebenen Bedeutungen haben und $R_6$ für Wasserstoff oder eine Gruppe der Formel $-(CH_2)_n-R_1'''$ steht, worin n 2 darstellt und $R_1'''$ Formyl oder Iminomethyl bedeutet oder, sofern $R_1$ freies oder verestertes Carboxy darstellt, ebenfalls freies oder verestertes Carboxy bedeutet bzw., sofern $R_1$ Niederalkoxycarbonyl darstellt, Formyl, Hydroxymethyl oder Cyano bedeutet, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze.

Die Erfindung betrifft beispielsweise ferner Verbindungen der Formel XVI, worin R unsubstituiertes oder in 5-Stellung durch Methoxy substituiertes 3-Indolyl bedeutet, Z für Ethylen steht, $R_1$ Methoxycarbonyl bedeutet und $R_6$ Wasserstoff oder eine Gruppe der Formel $-(CH_2)_n-R_1'''$ darstellt, in der n für 2 und $R_1'''$ für Methoxycarbonyl oder Formyl steht, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze.

Die Erfindung betrifft in erster Linie solche Verbindungen der Formel XVI, worin R unsubstituiertes oder in mindestens einer der Stellungen 1, 2 und 4 bis 7 substituiertes 3-Indolyl bedeutet, wobei als Substituent in 1-Stellung Niederalkyl oder Niederalkenyl, in 2-Stellung Niederalkyl und als Substituent(en) in 4- bis 7-Stellung Niederalkyl, Niederalkenyl, Niederalkoxy, Niederalkenyloxy, Hydroxy, Halogen, Trifluormethyl und/oder an benachbarte C-Atome gebundenes Niederalkyl(id)endioxy in Betracht kommen, Z Niederalkylen darstellt, welches den Rest R vom Stickstoffatom durch 1 bis 3, vorzugsweise 2 C-Atome trennt, $R_1$ für Carboxy, Niederalkoxycarbonyl, gegebenenfalls durch Niederalkyl, Niederalkoxy, Halogen, Nitro

und/oder Trifluormethyl substituiertes Phenylniederalkoxycarbonyl, 3- bis 8-gliedriges Cycloalkoxycarbonyl, Carbamyl, Mono- oder Diniederalkylencarbamyl, Niederalkylen- bzw. Aza-, Oxa- oder Thianiederalkylencarbamyl, N-Niederalkanoylcarbamyl, N-Niederalkoxycarbonylcarbamyl, Ureidocarbonyl, N'-Mono- oder N',N'-Diniederalkylureidocarbonyl, N',N'-Niederalkylen- bzw. N',N'-(Aza-, Oxa-oder Thia)niederalkylenureidocarbonyl steht und $R_6$ für Wasserstoff oder eine Gruppe der Formel $-(CH_2)_n-R_1'''$ bedeutet, worin n 1, 2 oder 3 ist und $R_1'''$ für Carboxy, Niederalkoxycarbonyl, gegebenenfalls durch Niederalkyl, Niederalkoxy, Halogen, Nitro und/oder Trifluormethyl substituiertes Phenylniederalkoxycarbonyl, 3- bis 8-gliedriges Cycloalkoxycarbonyl, Carbamyl, Mono- oder Diniederalkylcarbamyl, Niederalkylen- bzw. Aza-, Oxa-oder Thianiederalkylencarbamyl, N-Niederalkanoylcarbamyl, N-Niederalkoxycarbonylcarbamyl, Ureidocarbonyl, N'-Mono- oder N',N'-Diniederalkylureidocarbonyl, N',N'-Niederalkylen- bzw. N',N'-(Aza-, Oxa-oder Thia)niederalkylenureidocarbonyl steht oder in zweiter Linie Cyano, Hydroxymethyl, Diniederalkoxymethyl oder Niederalkylendioxymethyl bedeutet, wobei Niederalkylen alk bis und mit 4, die übrigen niederen Reste insgesamt bis und mit 7 und in jeder niederen Teilstruktur bis und mit 4 C-Atome aufweisen, und ihre pharmazeutisch verwendbaren Salze zur Anwendung in einem Verfahren zur Behandlung des menschlichen oder tierischen Körpers, diese enthaltende pharmazeutische Präparate und ihre Verwendung als Arzneimittelwirkstoffe, ebenso Verbindungen der Formel XVI, worin R, Z, $R_1$ und $R_6$ vorstehende Bedeutung haben, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze sowie Verfahren zu ihrer Herstellung.

Die Erfindung betrifft vor allem Verbindungen der Formel XVI, worin R unsubstituiertes oder in einer oder zwei der Stellungen 4 bis 7 durch Niederalkyl mit bis und mit 4 C-Atomen, wie Methyl, Niederalkoxy mit bis und mit 4 C-Atomen, wie Methoxy, Halogen der Atomnummer bis und mit 35, wie Chlor, und/oder Trifluormethyl substituiertes 3-Indolyl bedeutet, Z Niederalkylen mit bis und mit

4 C-Atomen, welches den Rest R vom Stickstoffatom durch 1 bis 3, vorzugsweise 2, C-Atome trennt, z.B. Methylen, 1,3-Propylen oder vorzugsweise Ethylen, darstellt, $R_1$ für Carboxy, Niederalkoxycarbonyl mit bis und mit 4 C-Atomen im Alkoxyteil, wie Methoxy- oder Ethoxycarbonyl, 3- bis 8-, z.B. 5- oder 6-gliedriges Cycloalkoxycarbonyl, wie Cyclohexyloxycarbonyl, Carbamyl oder N-Mono- oder N,N-Diniederalkylcarbamyl mit jeweils bis und mit 4 C-Atomen, wie Methyl-, Ethyl- oder Dimethylcarbamyl, bedeutet und $R_6$ für Wasserstoff oder eine Gruppe der Formel $-(CH_2)_n-R''$' steht, in der n 1 oder 2 ist und $R_1$"' Carboxy, Niederalkoxycarbonyl mit bis und mit 4 C-Atomen im Alkoxyteil, wie Methoxy- oder Ethoxycarbonyl, 3- bis 8-, z.B. 5- oder 6-gliedriges Cycloalkoxycarbonyl, wie Cyclohexyloxycarbonyl, Carbamyl, N-Mono- oder N,N-Diniederalkylcarbamyl mit jeweils bis und mit 4 C-Atomen, wie Methyl-, Ethyl- oder Dimethylcarbamyl, Cyano, Hydroxymethyl, Formyl oder Diniederalkoxymethyl bzw. Niederalkylendioxymethyl mit jeweils bis und mit 4 C-Atomen in jedem Alkoxy- bzw. Alkylendioxyteil, wie Dimethoxy- oder Diethoxymethyl bzw. Ethylendioxymethyl, bedeutet, und ihre pharmazeutisch verwendbaren Salze zur Anwendung in einem Verfahren zur Behandlung des menschlichen oder tierischen Körpers, diese enthaltende pharmazeutische Präparate und ihre Verwendung als Arzneimittelwirkstoffe, ebenso Verbindungen der Formel XVI, worin R, Z, $R_1$ und $R_6$ vorstehende Bedeutung haben, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze.

Die Erfindung betrifft vorzugsweise solche Verbindungen der Formel XVI, worin R unsubstituiertes oder in einer oder zwei der Stellungen 4 bis 7 durch Halogen der Atomnummer bis und mit 35, wie Chlor, und/oder Niederalkyl mit bis und mit 4 C-Atomen, wie Methyl, substituiertes 3-Indolyl bedeutet, Z geradkettige 1,1-, 1,3- oder vorzugsweise 1,2-Niederalkylen mit bis und mit 4 C-Atomen, wie Ethylen, ferner Methylen oder 1,3-Propylen, darstellt, $R_1$ Niederalkoxycarbonyl mit bis und mit 4 C-Atomen im Alkoxyteil, wie Methoxycarbonyl oder Ethoxycarbonyl, darstellt und

$R_6$ für Wasserstoff oder eine Gruppe der Formel $-(CH_2)_n-R_1'''$ bedeutet, worin n 1 oder 2 ist und R''' die gleiche Bedeutung wie $R_1$ hat, und ihre pharmazeutisch verwendbaren Salze zur Anwendung in einem Verfahren zur Behandlung des menschlichen oder tierischen Körpers, diese enthaltende pharmazeutische Präparate und ihre Verwendung als Arzneimittelwirkstoffe, ebenso Verbindungen der Formel XVI, worin R, Z, $R_1$ und $R_6$ vorstehende Bedeutung haben, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze sowie Verfahren zu ihrer Herstellung.

Die Erfindung betrifft vor allem solche Verbindungen der Formel XVI, worin R unsubstituiertes oder in zweiter Linie in 4- und 7-Stellung durch Niederalkyl mit bis und mit 4 C-Atomen, wie Methyl, bzw. in 5-Stellung durch Halogen der Atomnummer bis und mit 35, wie Chlor substituiertes 3-Indolyl bedeutet, Z Ethylen darstellt, $R_1$ Niederalkoxycarbonyl mit bis und mit 4 C-Atomen im Niederalkoxyteil, wie Methoxycarbonyl oder Ethoxycarbonyl, bedeutet und $R_6$ Wasserstoff oder eine Gruppe der Formel $-(CH_2)_n-R_1'''$ bedeutet, worin n 1 oder 2 ist und $R_1'''$ die gleiche Bedeutung wie $R_1$ hat oder Cyano, Diniederalkoxymethyl mit je bis und mit 4 Alkoxy-C-atomen, wie Dimethoxy- oder Diethoxymethyl, Niederalkylendioxymethyl mit 2 bis und mit 4 Alkylendioxy-C-atomen, wie 1,3-Dioxolan-2-yl oder 1,3-Dioxan-2-yl, oder N,N-Diniederalkylcarbamyl mit je bis und mit 4 Alkyl-C-atomen, wie Dimethylcarbamyl, darstellt, und ihre pharmazeutisch verwendbaren Salze zur Anwendung in einem Verfahren zur Behandlung des menschlichen oder tierischen Körpers, diese enthaltende pharmazeutische Präparate und ihre Verwendung als Arzneimittelwirkstoffe, ebenso Verbindungen der Formel XVI, worin R, Z, $R_1$ und $R_6$ vorstehende Bedeutung haben, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze sowie Verfahren zu ihrer Herstellung.

Die Erfindung betrifft jeweils vorzugsweise solche Verbindungen der Formel XVI, worin $R_6$ Wasserstoff ist.

Die Erfindung betrifft namentlich die in den Beispielen genannten Verbindungen der Formel XVI und ihre Salze, insbesondere pharmazeutisch verwendbaren Salze.

Das erfindungsgemäss Verfahren zur Herstellung von Verbindungen der Formel XVI und ihrer Salze ist dadurch gekennzeichnet, dass man

l) Verbindungen der Formeln

$$R - Z - X_5 \quad (XXXII) \quad \text{und} \quad X_6 - CH_2 - CH_2 - R_1 \quad (XXXIII),$$

worin einer der Reste $X_5$ und $X_6$ eine Gruppe der Formel $-N(R_6)-H$ und der andere eine nukleofuge Abgangsgruppe bedeutet, oder deren Salze miteinander umsetzt, oder

m) Verbindungen der Formeln

$$R - Z - N(R_6)-H \quad (XXXVII) \quad \text{und} \quad CH_2 = CH - R_1 \quad (XXXIV)$$

oder deren Salze miteinander umsetzt, oder

n) eine Verbindung der Formel

$$R_3 - \underset{R_4}{\underset{|}{N}} - N = \underset{R_5}{\underset{|}{C}} - CH_2 - Z - \underset{R_6}{\underset{|}{N}} - CH_2CH_2 - R_1 \quad (XXXV)$$

worin $R_6$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel XVI ringschliesst, oder

o) in einer Verbindung der Formel

$$R - Z - \overset{R_6'}{\underset{|}{N}} - CH_2CH_2 - R_1' \quad (XXXVI)$$

worin $R_6'$ Wasserstoff oder eine Gruppe der Formel alk-$R_1'$ und $R_1'$ einen in gegebenenfalls verestertes oder amidiertes Carboxy $R_1$ überführbaren Rest bedeutet, $R_1'$ in gegebenenfalls veresteres oder amidiertes Carboxy überführt und gewünschtenfalls die verfahrensgemäss erhältliche Verbindung in eine andere Verbindung der Formel XVI überführt, ein verfahrensgemäss erhältliches Isomerengemisch in die Komponenten auftrennt und das Isomere der Formel XVI isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere isoliert und/oder eine verfahrensgemäss erhältliche freie Verbindung in ein Salz oder ein verfahrensgemäss erhältliches Salz in die freie Verbindung oder in ein anderes Salz umwandelt.

Verfahrensvariante l)

Nukleofuge Abgangsgruppen $X_5$ sind beispielsweise reaktionsfähige veresterte Hydroxygruppen $X_3$, wie Halogen, z.B. Chlor, Brom oder Iod, oder von Sulfonsäuren, wie Niederalkan- oder gegebenenfalls substituierten Benzolsulfonsäuren, oder Halogensulfonsäuren abgeleitete Sulfonyloxygruppen, z.B. Methansulfonyloxy, Benzolsulfonyloxy, p-Toluolsulfonyloxy, p-Brombenzolsulfonyloxy oder Fluorsulfonyloxy.

Die Umsetzung von Verbindungen XXXII und XXXIII erfolgt in üblicher Weise, beispielsweise in Gegenwart eines basischen Kondensationsmittels, wie einer tertiären organischen Stickstoffbase, z.B. von Pyridin oder eines Triniederalkylamins, wie von Triethylamin. Durch Umsetzungen equimolarer Mengen beider Reaktanden erhält man, ausgehend von Verbindungen XXXII, worin $R_6$ Wasserstoff ist, Verbindungen XVI, worin $R_6$ Wasserstoff ist. Verwendet man die Reaktionskomponente XXXII in mindest doppeltmolarem Ueberschuss, so gelangt man, ausgehend von Verbindungen XXXII, worin $R_6$ Wasserstoff ist, zu Verbindungen XVI, worin $R_6$ eine Gruppe $-(CH_2)_n-R_1'''$ bedeutet, in der n für 2 steht und $R_1'''$ mit $R_1'$ identisch ist.

Die Herstellung von Verbindungen XXXII, worin $X_5$ eine Gruppe $X_3$ bedeutet, d.h. von Verbindungen V, ist bereits unter der Verfahrensvariante d) beschrieben. Verbindungen XXXVII, worin $X_5$ eine Gruppe $-N(R_6)-H$ und $R_6$ Wasserstoff ist, d.h. Verbindungen XVIII, sind weitgehend bekannt, sie können auch aus den entsprechenden Verbindungen V durch Umsetzung mit Ammoniak erhalten werden. Verbindungen XXXII, worin $X_5$ eine Gruppe $-N(R_6)-H$ und $R_6$ eine Gruppe der Formel alk-$R_1'''$ bedeutet, können hergestellt werden durch Umsetzung von Verbindungen der Formeln $R-Z-NH_2$ (XVIII) und $X_3$-alk-$R_1'''$ (XXXIIIa) bzw. $CH_2=CH-R_1'''$ (XXXIVa) oder Umsetzung von Verbindungen $R-Z-X_3$ (V) und $H_2N$-alk-$R_1'''$ (XXXIIIb).

Nach dieser Verfahrensvariante kann man beispielsweise Verbindungen der Formel XVI, worin R, Z und $R_1$ die angegebenen Bedeutungen haben und $R_6$ für Wasserstoff oder eine Gruppe der Formel $-(CH_2)_n-R_1'''$ steht, worin n 2 darstellt und $R_1'''$ Formyl oder Iminomethyl bedeutet oder, sofern $R_1$ freies oder verestertes Carboxy darstellt, ebenfalls freies oder verestertes Carboxy bedeutet bzw., sofern $R_1$ Niederalkoxycarbonyl darstellt, Formyl, Hydroxymethyl oder Cyano bedeutet, oder Verbindungen der Formel XVI, worin R unsubstituiertes oder in 5-Stellung durch Methoxy substituiertes 3-Indolyl bedeutet, Z für Ethylen steht, $R_1$ Methoxycarbonyl bedeutet und $R_6$ Wasserstoff oder eine Gruppe der Formel $-(CH_2)_n-R_1'''$ darstellt, in der n für 2 und $R_1'''$ für Methoxycarbonyl oder Formyl steht, und ihre Salze herstellen, indem man eine Verbindung der Formel

$R-Z-X_3$ (V),

worin $X_3$ reaktionsfähig verestertes Hydroxy bedeutet, mit einer Verbindung der Formel

$H_2N-CH_2CH_2-R_1$ (XVa)

umsetzt und die erhaltene Verbindung der Formel XVI, worin $R_6$ Wasserstoff ist, gewünschtenfalls mit Acrolein oder einem Aldehyd der Formel $Y_1-CH_2CH_2-CH=R_2'$ (XVIIa), worin $Y_1$ reaktionsfähiges verestertes Hydroxy und $R_2'$ Oxo bedeutet, weiter umsetzt und gewünschtenfalls eine verfahrensgemäss erhältliche freie Verbindung in ein Salz oder ein verfahrensgemäss erhältliches Salz in die freie Verbindung oder in ein anderes Salz umwandelt.

Verfahrensvariante m)

Die Umsetzung von Verbindungen XXXVII mit Verbindungen XXXIV erfolgt in üblicher Weise, beispielsweise in einem Niederalkanol, wie Ethanol, bei etwa 0° bis etwa 60°C.

Durch Umsetzungen equimolarer Mengen beider Reaktanden erhält man, ausgehend von Verbindungen XXXVII, worin $R_6$ Wasserstoff ist, Verbindungen XVI, worin $R_6$ Wasserstoff ist. Verwendet man die Reaktionskomponente XXXIV in mindest doppeltmolarem Ueberschuss, so gelangt man, ausgehend von Verbindungen XXXVII, worin $R_6$ Wasserstoff ist, zu Verbindungen XVI, worin $R_6$ eine Gruppe $-(CH_2)_n-R_1'''$ bedeutet, in der n 2 und $R_1'''$ mit $R_1$ identisch ist.

Nach diesen Verfahrensvarianten kann man beispielsweise Verbindungen der Formel XVI, worin R und Z die angegebenen Bedeutungen haben und $R_6$ für Wasserstoff oder eine Gruppe der Formel $-(CH_2)_n-R_1'''$ steht, worin n 2 darstellt, wobei $R_1$ und $R_1'''$ freie oder gleiche veresterte Carboxygruppen darstellen, oder Verbindungen der Formel XVI, worin R unsubstituiertes oder in 5-Stellung durch Methoxy substituiertes 3-Indolyl bedeutet, alk für Ethylen steht, $R_1$ Methoxycarbonyl bedeutet und $R_6$ Wasserstoff oder eine Gruppe der Formel $-(CH_2)_n-R_1'''$ darstellt, in der n für 2 und $R_1'''$ für Methoxycarbonyl steht, und ihre Salze herstellen, indem man eine Verbindung der Formel

$R-Z-NH_2$ (XVIII)

mit der annähernd doppeltmolaren Menge einer Verbindung der Formel $Y_1-CH_2CH_2-R_1''$ (XIXa) bzw. $CH_2=CH-R_1''$ (XIXb), worin $Y_1$ reaktionsfähiges verestertes Hydroxy und $R_1''$ freies oder verestertes Carboxy, z.B. Methoxycarbonyl, bedeutet, umsetzt und gewünschtenfalls eine verfahrensgemäss erhältliche freie Verbindung in ein Salz oder ein verfahrensgemäss erhältliches Salz in die freie Verbindung oder in ein anderes Salz umwandelt

Verfahrensvariante n)

Die Umlagerung, bei der im Sinne der Fischer'schen Indolsynthese Ammoniak abgespalten wird, kann in üblicher Weise erfolgen, beispielsweise durch Säurebehandlung, d.h. Einwirkung einer geeigneten Protonen- oder Lewissäure. Als Protonensäuren kommen beispielsweise Mineralsäuren, wie Schwefelsäure, Phosphorsäure, ebenso Chlorwasserstoff in einem Niederalkanol, wie ethanolische Salzsäure, und organische Sulfonsäuren, wie Niederalkan-, z.B. Methansulfonsäure, oder gegebenenfalls substituierte Benzolsulfonsäuren, wie Benzol- oder p-Toluolsulfonsäure, aber auch organische Carbonsäuren, wie Niederalkansäuren, z.B. Essigsäure, in Betracht. Als Lewissäuren sind beispielsweise koordinativ ungesättigte Schwermetallverbindungen, wie koordinativ ungesättigte Halogenide des Bors, Aluminium, Antimons, Zinks, Kupfers, Nickels, Eisens, Chroms oder Zinns, z.B. Zinkchlorid oder Kupfer-I-chlorid bzw. -bromid, geeignet. Die Umsetzung wird vorteilhaft unter Erwärmen, z.B. im Temperaturbereich von etwa 60 bis 170°C, erforderlichenfalls unter Inertgas, durchgeführt.

Zwischenprodukte XXXV können z.B. hergestellt werden, indem man ein Hydrazon der Formel $R_3-NH-N=C(CR_5)-CH_2-Z-NH_2$ (XXXVa) mit einer Verbindung $X_6-CH_2CH_2-R_1$ (XXXIII; $X_6$ = Halogen) bzw. $CH_2=CH_2-R_1$ (XXXIV) und gewünschtenfalls anschliessend mit einer Verbindung $X_3$-alk-$R_1'''$ (XXXIIIa; $X_3$ = Halogen) bzw. $CH_2=CH-R_1'''$ (XXXIVa)

kondensiert und gewünschtenfalls das erhaltene Hydrazon am sekundären N-Atom durch einen von Wasserstoff verschiedenen Rest $R_4$ substituiert.

Verfahrensvariante o)

In gegebenenfalls verestertes oder amidiertes Carboxy überführbare Reste $R_1'$ sind beispielsweise die für die Verfahrensvariante f) angegebenen zu $R_1$ solvolysierbaren bzw. oxidierbaren Gruppen. Auch die Durchführung der Solvolyse bzw. Oxidation erfolgt beispielsweise in analoger Weise wie dort angegeben.

Die Ausgangsstoffe XXXVI können beispielsweise hergestellt werden, indem man eine Verbindung der Formel $R-Z-NH_2$ (XVIII) oder ein Salz davon mit einer Verbindung der Formel $X_3-CH_2CH_2-R_1'$ (XXXVIII) bzw. $CH_2=CH-R_1'$ (IXL), worin $X_3$ eine nukleofuge Abgangsgruppe, wie Halogen, z.B. Chlor oder Brom bedeutet, und gewünschtenfalls anschliessend mit einer Verbindung der Formel $X_3$-alk-$R_1'$ (XXXVIIIa) umsetzt, beispielsweise in analoger Weise wie für die Verfahrensvariante l) angegeben. Zur Herstellung von Verbindungen XXXVI, worin $R_1'$ Cyano ist, kann man aber auch die Verbindung XVIII mit einem 1,2-difunktionellen Ethanderivat, wie einem 1,2-Dihalogenethan, z.B. 1,2-Dibromethan, umsetzen und das Reaktionsprodukt mit einem Alkalimetallcyanid zur Reaktion bringen.

Als fakultative Folgereaktionen im Zusammenhang mit Verbindungen XVI sind z.B. die für Verbindungen I angegebenen gegenseiten Umwandlungen von freien, veresterten und amidierten Carboxygruppen $R_1$, Einführungs- bzw. Abwandlungsreaktionen von Substituenten des Restes R, gegenseitige Umwandlungen freier Verbindungen und Salze sowie Auftrennungsmassnahmen erhaltener Isomeren zu nennen, die sämtlichst in analoger Weise durchgeführt werden, wie für Verbindungen I angegeben. Zu erwähnen ist ferner die Möglichkeit der Oxidation von Hydroxymethyl bzw. Formyl $R_1'''$ zu Carboxy sowie die Hydrolyse von Cyano $R_1'''$ zu Carbamyl oder Carboxy, die ebenfalls in üblicher Weise durchgeführt werden können.

Insbesondere kann man ferner eine verfahrensgemäss erhältliche Verbindung XVI, worin $R_6$ Wasserstoff ist, in analoger Weise wie für die Verfahrensvariante l) bzw. m) angegeben durch Umsetzung mit einer Verbindung der Formel $X_3$-alk-$R_1'''$ (XXXIIIa) bzw. $CH_2$=$CH_2$-$R_1'''$ (XXXIVa) in die entsprechende Verbindung XVI überführen, worin $R_1'''$ von $R_1$ verschieden ist.

Die neuen Verbindungen der Formel I und XVI können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oer Gelatinekapseln, welche den Wirkstoff zusammen mit Verdünnungsmitteln, z.B. Laktose, Dextrose, Saccharose, Mannitol, Sorbitol, Cellulose und/oder Schmiermittel, z.B. Kieselerde, Talk, Stearinsäure oder Salze davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel Farbstoffe, Geschmackstoffe und Süssmittel aufweisen. Ferner kann man die neue Verbindung der Formel I bzw. XVI in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame

Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Erfindung betrifft ebenfalls die Verwendung der Verbindungen der Formel I bzw. XVI, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Gewicht von etwa 70 kg vorzugsweise zwischen etwa 20 mg und etwa 500 mg.

Die nachfolgenden Beispiele dienen zur Illustration der Erfindung; Temperaturen sind in Celciusgraden, Drucke in mbar angegeben.

Beispiel 1: 22,4 g 1-Brom-2-(1H-indol-3-yl)-ethan, 15,7 g Piperid-4-on-3-carbonsäuremethylester und 39 g N-Ethyl-N,N-diisopropylamin werden unter Stickstoff in 750 ml Dimethylformamid gelöst und 16 Stunden gerührt. Dann engt man unter vermindertem Druck auf etwa 300 ml ein, fügt 500 ml Wasser hinzu und schüttelt dreimal mit je 250 ml Dichlormethan aus. Die organischen Phasen werden vereinigt und dreimal mit je 80 ml n-Salzsäure ausgeschüttelt. Die wässrigen Phasen werden vereinigt, mit 40%iger Natronlauge basisch gestellt und erneut dreimal mit je 100 ml Dichlormethan ausgeschüttelt. Die neuen organischen Phasen werden vereinigt, mit Wasser und anschliessend mit gesättigter Kochsalzlösung gewaschen, über Natriumsulfat getrocknet und zur Trockene eingedampft. Der Eindampfrückstand wird in 100 ml warmen Diethylethers gelöst und mit 150 ml n-Hexan versetzt. Nach dem Abkühlen kristallisiert der 4-Hydroxy-1-[2-(1H-indol-3-yl)ethyl]-1,2,5,6-tetrahydro-pyridin-3-carbonsäuremethylester bzw. 1-[2-(1H-Indol-3-yl)ethyl]-piperidin-4-on-3-carbonsäuremethylester aus, welcher abfiltriert und getrocknet wird; das Hydrochlorid desselben schmilzt bei 187° (Zers.)

Beispiel 2: 320,4 g (2 Mol) Tryptamin werden unter Rühren in 550 ml Methanol suspendiert und innerhalb einer Stunde tropfenweise mit 378,5 g (4,4 Mol) Acrylsäuremethylester versetzt. Man lässt 6 Stunden bei 50° und über Nacht bei Raumtemperatur nachrühren, dampft bei 40° unter vermindertem Druck zur Trockne ein, nimmt in 1,5 l Toluol auf, fügt 50 g Aktivkohle hinzu, lässt 30 Minuten rühren, filtriert über Diatomerenerde und dampft unter vermindertem Druck zur Trockne ein. Man erhält N-[2-(1H-Indol-3-yl)ethyl]-imino-di(3-propionsäure)dimethylester als rötliches Oel, der in analoger Weise wie in Beispiel 10 beschrieben in das Oxalat vom Smp. 98-103° überführt werden kann.

153 g (2,83 Mol) Natriummethanolat werden unter Rühren in 1000 ml Dimethylformamid gelöst. Dann tropft man innerhalb von 90 Minuten eine Lösung von 665 g (2 Mol) N-[2-(1H-Indol-3-yl)ethyl]-imino-di(3-propionsäure)dimethylester in 680 ml Dimethylformamid hinzu, erwärmt auf 40°, lässt 3 Stunden bei Raumtemperatur nachrühren und destilliert das Lösungsmittel bei 40° unter vermindertem Druck ab. Der Rückstand wird bei 20 bis 35° in ein Gemisch von 2,1 l 5n-Salzsäure und 500 ml Toluol eingegossen. Das ausgefallene 4-Hydroxy-1-[2-(1H-indol-3-yl)ethyl]-1,2,5,6-tetrahydropyridin-3-carbonsäuremethylester-hydrochlorid bzw. 1-[2-(1H-Indol-3-yl)ethyl]-piperidin-4-on-3-carbonsäuremethylester-hydrochlorid wird abgenutscht, mit kaltem Methanol gewaschen und bei 40° unter vermindertem Druck getrocknet. Es schmilzt bei 185-187° (Zers.).

Beispiel 3: In analoger Weise wie in Beispiel 2 beschrieben erhält man ausgehend von 80 g (0,5 Mol) Tryptamin und 112 g (1,1 Mol) Acrylsäureethylester in 150 ml Ethanol N-[2-(1H-Indol-3-yl)ethyl]-imino-di(3-propionsäure)diethylester und durch Umsetzung von 180 g derselben mit 37,4 g (0,55 Mol) Natriumethanolat das 4-Hydroxy-1-[2-(1H-indol-3-yl)ethyl]-1,2,5,6-tetrahydro-pyridin-3-carbonsäureethylester-hydro chlorid bzw. 1-[2-(1H-Indol-3-yl)ethyl]-piperidin-4-on-3-carbonsäureethylester-hydrochlorid von Smp. 170-173° (Zers.).

Beispiel 4: 52,6 g (0,15 Mol) 4-Hydroxy-1-[2-(1H-indol-3-yl)-ethyl]-1,2,5,6-tetrahydro-pyridin-3-carbonsäureethylester-hydrochlorid werden in 2 l Wasser mit 5 g Platinoxid bei Raumtemperatur und Normaldruck bis zur Aufnahme von 3,8 l Wasserstoff hydriert. Man filtriert vom Katalysator ab und dampft bei 30° unter vermindertem Druck zur Trockne ein. Der Rückstand wird mit 50 ml Aceton versetzt. Das alsbald auskristallisierende Rohprodukt wird abgesaugt und aus Ethanol umkristallisiert. Man erhält cis-4-Hydroxy-1-[2-(1H-indol-3-yl)ethyl]-piperidin-3-carbonsäureethylester-hydrochlorid vom Smp. 207-210°.

In analoger Weise erhält man durch Hydrierung von 33,7 g (100 mMol) 4-Hydroxy-1-[2-(1H-indol-3-yl)ethyl]-1,2,5,6-tetrahydro-pyridin-3-carbonsäuremethylester-hydrochlorid in Gegenwart von 1,7 g Platinoxid das cis-4-Hydroxy-1-[2-(indol-3-yl)ethyl)-piperidin-3-carbonsäuremethylester-hydrochlorid vom Smp. 187° (Zers.).

Beispiel 5: 337 g (1 Mol) 4-Hydroxy-1-[2-(1H-indol-3-yl)ethyl]-1,2,5,6-tetrahydro-pyridin-3-carbonsäuremethylester-hydrochlorid werden in 3,3 l Methanol gelöst und unter Rühren bei -15 bis 5° innerhalb von 2 Stunden portionsweise mit 68 g (1,1 Mol) Natriumborhydrid versetzt. Man lässt 1 Stunde nachrühren, erwärmt langsam auf 40° und destilliert unter vermindertem Druck das Lösungsmittel ab. Der Rückstand wird in einem Gemisch von 1,5 l Essigester und 1 l Eiswasser aufgenommen. Man gibt 50 g Kaliumcarbonat hinzu, lässt 30 Minuten rühren, trennt die organische Phase ab, wäscht mit Wasser neutral, trocknet über Magnesiumsulfat und dampft unter vermindertem Druck zur Trockne ein. Man erhält ein Gemisch aus etwa gleichen Teilen cis- und trans-4-Hydroxy-1-[2-(1H-indol-3-yl)ethyl]-piperidin-3-carbonsäuremethylester als Oel. Dieses kann durch Flash-Chromatographie an Kieselgel mit Dichlormethan/Methanol (95:5) in die Komponenten aufgetrennt werden, wobei das trans-Isomere zuerst eluiert wird. Es kann durch Ueberführung in das Semifumarat vom Smp. 208-209° (vgl. Beispiel 6) charakterisiert werden. Das später

isolierte cis-Isomere kann durch Ueberführung in das Hydrochlorid vom Smp. 187° (Zers.) in analoger Weise wie in Beispiel 8 beschrieben charakterisiert werden.

Beispiel 6: 3,16 g (10 mMol) trans-4-Hydroxy-1-[2-(indol-3-yl)-ethyl]-piperidin-3-carbonsäuremethylester werden in 50 ml Aceton gelöst und mit 0,6 g (50 mMol) Fumarsäure, gelöst in 10 ml Aceton, versetzt. Nach einiger Zeit kristallisiert das trans-4-Hydroxy-1-[2-(indol-3-yl)ethyl]-piperidin-3-carbonsäuremethylester-semifumarat aus. Es wird abfiltriert und unter vermindertem Druck getrocknet; Smp. 208-209°.

Beispiel 7: In analoger Weise wie in Beispiel 5 beschrieben erhält man durch Reduktion von 35,3 g (100 Mol) 4-Hydroxy-1-[2-(1H-indol-3-yl)ethyl]-1,2,5,6-tetrahydro-pyridin-3-carbonsäureethylester-hydrochlorid mit 3,8 g (100 mMol) Natriumborhydrid in 300 ml Ethanol ein Gemisch aus etwa gleichen Teilen cis- und trans-4-Hydroxy-1-[2-(1H-indol-3-yl)ethyl]-piperidin-3-carbonsäureethylester, das chromatographisch aufgetrennt und zur Charakterisierung in das trans-4-Hydroxy-1-[2-(1H-indol-3-yl)ethyl]-piperidin-3-carbonsäureethylester-hydrochlorid, Smp. 109-112°, bzw. cis-4-Hydroxy-1-[2-(1H-indol-3-yl)ethyl]-piperidin-3-carbonsäureethylester-hydrochlorid, Smp. 210-213°, (vgl. Beispiel 8) überführt werden kann.

Beispiel 8: 6,32 g (20 mMol) trans-4-Hydroxy-1-[2-(1H-indol-3-yl)-ethyl]-piperidin-3-carbonsäureethylester werden in 100 ml Dichlormethan gelöst und mit etherischer Chlorwasserstofflösung kongosauer gestellt. Der kristalline Niederschlag wird abgenutscht und unter vermindertem Druck getrocknet. Man erhält das trans-4-Hydroxy-1-[2-(1H-indol-3-yl)ethyl]-piperidin-3-carbonsäureethylester-hydrochlorid vom Smp. 109-112°. In analoger Weise erhält man auch das cis-4-Hydroxy-1-[2-(1H-indol-3-yl)ethyl]-piperidin-3-carbonsäureethylester-hydrochlorid vom Smp. 210-213° (Zers.).

Beispiel 9: 30,2 g (100 mMol) cis-4-Hydroxy-1-[2-(1H-indol-3-yl)-ethyl]-piperidin-3-carbonsäuremethylester werden in 150 ml Pyridin gelöst, mit 75 ml Acetanhydrid versetzt und 15 Stunden bei Raumtemperatur gerührt. Man dampft unter vermindertem Druck zur Trockne ein, nimmt mit einem Gemisch von Eiswasser, gesättigte Kaliumcarbonatlösung und Diethylether auf, trennt die organische Phase ab, wäscht mit Wasser neutral, trocknet über Magnesiumsulfat und dampft zur Trockne ein. Man erhält den cis-4-Acetoxy-1-[2-(1H-indol-3-yl)-ethyl]-piperidin-3-carbonsäuremethylester vom Smp. 93-95°.

In genau analoger Weise erhält man auch den trans-4-Acetoxy-1-[2-(1H-indol-3-yl)ethyl]-piperidin-3-carbonsäuremethylester als farbloses Oel, Smp. des Oxalates (vgl. Beispiel 10) 189°.

Die beiden vorstehend genannten Verbindungen können auch erhalten werden, indem man ein Gemisch von etwa gleichen Teilen cis- und trans-4-Hydroxy-1-[2-(1H-indol-3-yl)ethyl]-piperidin-3-carbonsäuremethylester in genau analoge Weise wie vorstehend beschrieben acyliert und das erhaltene ölige Gemisch etwa gleicher Teile cis- und trans-4-Acetoxy-1-[2-(1H-indol-3-yl)ethyl]-piperidin-3-carbonsäuremethylester chromatographisch in die Komponenten auftrennt. Dazu unterwirft man 2,5 g des Gemisches einer Flash-Chromatographie an 150 g Kieselgel (60, Merck) mit Trichlormethan/Methanol 98:2) als Laufmittel. Das trans-Isomere wird zuerst, das cis-Isomere zuletzt isoliert.

Beispiel 10: 0,38 g (2 mMol) trans-4-Acetoxy-1-[2-(1H-indol-3-yl)-ethyl]-piperidin-3-carbonsäuremethylester werden in 10 ml Aceton gelöst und mit 0,2 g (2 mMol) Oxalsäure, gelöst in 3 ml Aceton, versetzt. Das alsbald auskristallisierende trans-4-Acetoxy-1-[2-(1H-indol-3-yl)ethyl]-piperidin-3-carbonsäuremethylester-oxalat wird abgesaugt und getrocknet; Smp. 189° (Zers.)

Beispiel 11: 16,9 g (50 mMol) 4-Hydroxy-1-[2-(1H-indol-3-yl)ethyl]-1,2,5,6-tetrahydro-pyridin-3-carbonsäuremethylester-hydrochlorid werden in 100 ml Methanol suspendiert und mit 25 ml einer konzentrierten wässrigen Ammoniaklösung versetzt. Man lässt 2 Stunden bei 40° rühren, dampft unter vermindertem Druck zur Trockne ein, schüttelt mit 300 ml Dichlormethan und 100 ml Wasser aus, trennt die organische Phase ab, wäscht mit Wasser neutral, trocknet über Magnesiumsulfat, dampft zur Trockne ein und kristallisiert aus Diethylether/Hexan. Man erhält den 4-Amino-2-[2-(1H-indol-3-yl)-ethyl]-1,2,5,6-tetrahydro-pyridin-3-carbonsäuremethylester vom Smp. 112-113°.

Beispiel 12: Eine Lösung von 14,2 g (50 mMol) 1-[2-(1H-Indol-3-yl)-ethyl]-1,2,5,6-tetrahydro-pyridin-3-carbonsäuremethylester in 150 ml Methanol wird mit 40 g Ammoniak versetzt und im Autoklaven 12 Stunden auf 100° erwärmt, wobei der Druck auf 22 bar ansteigt. Man lässt abkühlen, dampft an der Wasserstrahlpumpe zur Trockne ein, kristallisiert aus einem Gemisch von 50 ml Dichlormethan und 100 ml Diethylether und kristallisiert aus Ethanol um. Man erhält das 4-Amino-1-[2-(1H-indol-3-yl)ethyl]-piperidin-3-carbonsäureamid vom Smp. 210-212°.

Das Ausgangsmaterial kann folgendermassen hergestellt werden:

22,4 g 1-Brom-2-(1H-indol-3-yl)-ethan, 22,2 g 1,2,5,6-Tetrahydropyridin-3-carbonsäuremethylester-hydrobromid (Guvacolinhydrobromid) und 39 g N-Ethyl-N,N-diisopropyl-amin werden unter Stickstoff in 750 ml Dimethylformamid gelöst und 16 Stunden gerührt. Dann engt man unter vermindertem Druck auf etwa 300 ml ein, fügt 500 ml Wasser hinzu und schüttelt dreimal mit je 250 ml Dichlormethan aus. Die wässrige Phase wird mit 40%iger Natronlauge basisch gestellt und erneut dreimal mit je 100 ml Dichlormethan ausgeschüttelt. Die neuen organischen Phasen werden vereinigt, mit Wasser und anschliessend mit gesättigter Kochsalzlösung gewaschen, über Natriumsulfat getrocknet und zur Trockene eingedampft. Der Eindampfrückstand wird in 100 ml warmen Diethylethers gelöst und mit 150 ml n-Hexan

versetzt. Nach dem Abkühlen kristallisiert der 1-[2-(1H-Indolyl-3-yl)ethyl]-1,2,5,6-tetrahydro-pyridin-3-carbonsäureethylester aus, welcher abfiltriert und getrocknet wird; Smp. 76-77°.

Beispiel 13: 2,82 g (10 mMol) 4-Amino-1-[2-(1H-indol-3-yl)ethyl]-piperidin-3-carbonsäureamid werden in 35 ml Ethanol suspendiert und bis zur kongosauren Reaktion mit einer diethyletherischen Chlorwasserstoffsäurelösung versetzt. Durch Versetzen mit Diethylether wird das 4-Amino-1-[2-(1H-indol-3-yl)ethyl]-piperidin-3-carbonsäureamid-bishydrochlorid ausgefällt. Es wird abgenutscht und getrocknet; Smp. 170-180°.

Beispiel 14: 33 g (175 mMol) 4,6-Dimethyltryptamin und 40 ml (368 mMol) Acrylsäureethylester werden in 1000 ml Ethanol 72 Stunden zum Rückfluss erhitzt. Man dampft zur Trockne ein und chromatographiert an Kieselgel (0,063-0,02 mm) mit Toluol/Isopropanol (99:1) als Laufmittel. Die das gewünschte Produkt enthaltenden Fraktionen werden eingedampft und unter vermindertem Druck getrocknet; man erhält N-[2-(4,6-Dimethyl-1H-indol-3-yl)ethyl]-imino-di(3-propionsäure)diethylester als bräunliches Oel.

3,8 g (165 mMol) Natrium werden in 180 ml Ethanol gelöst und zum Rückfluss erhitzt. Man destilliert 120 ml Ethanol ab, fügt 180 ml Toluol hinzu und destilliert solange, bis die Destillationstemperatur 110° erreicht hat. Dann lässt man auf 20° abkühlen, gibt 64,1 g (165 mMol) N-[2-(4,6-Dimethyl-1H-indol-3-yl)ethyl]-imino-di(3-propionsäure)diethylester, gelöst in 270 ml Toluol, hinzu und lässt 2 Stunden bei 100° rühren. Man lässt auf Raumtemperatur abkühlen, säuert mit 130 ml konzentrierter Salzsäure an, saugt den ausgefallenen Niederschlag ab und kristallisiert zweimal aus 90%-igem Ethanol um. Man erhält so 4-Hydroxy-1-[2-(4,6-dimethyl-1H-indol-3 -yl)ethyl]-1,2,5,6-tetrahydro-pyridin-3-carbonsäureethylester-hydrochlorid bzw. 1-[2-(4,6-Dimethyl-1H-indol-3-yl]-ethyl]-piperidin-4-on-3-carbonsäureethylester-hydrochlorid vom Smp. 207-209°.

In analoger Weise erhält man aus 19 g 4,6-Dimethyltryptamin und 19 g Acrylsäuremethylester N-[2-(4,6-Dimethyl-1H-indol-3-yl)-ethyl]-imino-di(3-propionsäure)dimethylester (Oel) und ausgehend von 35,6 g desselben und 8,44 g Natriummethanolat 4-Hydroxy-1-[2-(4,6-dimethyl-1H-indol-3-yl)ethyl]-1,2,5,6-tetrahydropyridin-3-carbonsäuremethylester-hydrochlorid bzw. 1-[2-(4,6-Dimethyl-1H-indol-3-yl)-ethyl]-piperidin-4-on-3-carbonsäuremethylesterhydrochlorid vom Smp. 185-188°.

Beispiel 15: In analoger Weise wie in Beispiel 14 beschrieben erhält man ausgehend von 58,4 g (300 mMol) 5-Chlortryptamin und 65 ml (600 mMol) Acrylsäureethylester N-[2-(5-Chlor-1H-indol-3-yl)-ethyl]-imino-di(3-propionsäure)diethylester (Oel) und durch Cyclisierung desselben 4-Hydroxy-1-[2-(5-chlor-1H-indol-3-yl)ethyl]-1,2,5,6-tetrahydropyridin-3-carbonsäureethylester-hydrochlorid bzw. 1-[2-(5-Chlor-1H-indol-3-yl)ethyl]-piperidin-4-on-3-carbonsäureethylester-hydrochlorid vom Smp. 172-174°C.

In analoger Weise erhält man ausgehend von 5,7 g 5-Chlortryptamin und 5,7 g Acrylsäuremethylester N-[2-(5-Chlor-1H-indol-3-yl)ethyl]-imino-di(3-propionsäure)dimethylester (Oel) und durch Behandlung von 12,4 g desselben mit 2,9 g Natriummethanolat in 120 ml Dimethylformamid 4-Hydroxy-1-[2-(5-chlor-1H-indol-3-yl)ethyl]-1,2,5,6-tetrahydro-pyridin-3-carbonsäuremethylester-hydrochlorid bzw. 1-[2-5-Chlor-1H-indol-3-yl)ethyl]-piperidin-4-on-3-carbonsäuremethylesterhydrochlorid vom Smp. 195-197°C sowie ausgehend von 5-Brom- bzw. 5-Fluortryptamin und Acrylsäureethylester N-[2-(5-Brom-1H-indol-3-yl)ethyl] bzw. N-[2-(5-Fluor-1H-indol-3-yl)-ethyl]-imino-di(3-propionsäure)diethylester und 4-Hydroxy-1-[2-(5-brom-1H-indol-3-yl)ethyl]- und 4-Hydroxy-1-[2-(5-fluor-1H-indol-3-yl)ethyl]-1,2,5,6-tetrahydro-pyridin-3-carbonsäureethylester bzw. 1-[2-(5-Brom-1H-indol-3-yl)ethyl]- und 1-[2(5-Fluor-1H-indol-3-yl)-ethyl]-piperidin-4-on-3-carbonsäureethylester, analog auch die entsprechenden Methylester.

Beispiel 16: In eine Suspension von 9,5 g (25 mMol) 4-Hydroxy-1-[2-(4,6-dimethyl-1H-indol-3-yl)ethyl]-1,2,5,6-tetrahydro-pyridin-3-carbonsäureethylester-hydrochlorid in 72 ml Methanol und 18 ml Wasser werden bei 5 bis 10° innerhalb von 20 Minuten portionsweise 1,42 g (37,5 mMol) Natriumborhydrid eingetragen. Man lässt 20 Minuten bei 5 bis 10° nachrühren, versetzt mit Methylenchlorid und Wasser, schüttelt durch, trennt die Methylenchloridphase ab und schüttelt mit Methylenchlorid nach. Die vereinigten Methylenchloridauszüge werden über Magnesiumsulfat getrocknet und zur Trockne eingedampft. Der Eindampfrückstand wird über Kieselgel (0,063-0,2 mm) mit Essigsäureethylester/Isopropanol (7:1) als Laufmittel chromatographiert. Zunächst wird trans-4-Hydroxy-1-[2-(4,6-dimethyl-1H-indol-3-yl)ethyl]-piperidin-3-carbonsäureethylester, Rf = 0,56, und dann cis-4-Hydroxy-1-[2-(4,6-dimethyl-1H-indol-3-yl)ethyl]-piperidin-3-carbonsäureethylester, Rf = 0,49, eluiert. Die jeweiligen Fraktionen werden vereinigt, eingedampft und durch Behandeln mit Chlorwasserstoffsäure in Diethylether in das Hydrochlorid-hydrat vom Smp. 126-129° (trans) bzw. Hydrochlorid-hydrat vom Smp. 181-183° (cis) überführt.

Beispiel 17: In analoger Weise wie in Beispiel 16 beschrieben werden ausgehend von 10,8 g (28 mMol) 4-Hydroxy-1-[2-(5-chlor-1H-indol-3-yl)ethyl]-1,2,5,6-tetrahydropyridin-3-carbonsäureethylester durch Reduktion mit 1,6 g (42 mMol) Natriumborhydrid, chromatographische Trennung der diastereomeren trans-4-Hydroxy-1-[2-(5-chlor-1H-indol-3-yl)ethyl]-piperidin-3-carbonsäureethylester, Rf = 0,42, und cis-4-Hydroxy-1-[2-(5-chlor-1H-indol-3-yl)ethyl]-piperidin-3-carbonsäureethylester, Rf = 0,27, sowie durch anschliessende Behandlung mit Fumarsäure deren Semifumarate vom Smp. 108-110° (trans) bzw. 209-211° (cis) erhalten.

Analog erhält man durch Umsetzung von 6,6 g 4-Hydroxy-1-[2-(5-chlor-1H-indol-3-yl)ethyl]-1,2,5,6-tetrahydropyridin-3-carbonsäuremethylester mit 3,0 g Natriumborhydrid ein Gemisch von cis- und trans-4-Hydroxy-1-[2-(5-chlor-1H-indol-3-yl)ethyl]-piperidin-3-carbonsäuremethylester, dass chromatographisch in die Komponenten vom $R_f$-Wert = 0,38 (trans) bzw. 0,26 (cis) aufgetrennt werden kann.

In analoger Weise erhält man auch cis- und trans-4-Hydroxy-1-[2-(5-brom-1H-indol-3-yl)ethyl]- und cis- und trans-4-Hydroxy-1-[2-(5-fluor-1H-indol-3-yl)ethyl]-piperidin-3-carbonsäureethylester sowie die entsprechenden Methylester.

Beispiel 18: 2,3 g (6,7 mMol) 4-Hydroxy-1-[2-(4,6-dimethyl-1H-indol-3-yl)-ethyl]-1,2,5,6-tetrahydro-pyridin-3-carbonsäureethylester werden mit 0,4 g Platinoxid und 46 ml Ethanol vermischt und in eine Parr-Apparatur 18 Stunden bei Raumtemperatur und einem Wasserstoffüberdruck von 4 bar bis zur Aufnahme von 160 ml Wasserstoff hydriert. Das Reaktionsgemisch wird über Diatomeenerde filtriert und zur Trockne eingedampft. Chromatographische Reinigung an Kieselgel (0,063-0,2 mm) mit Essigester/Isopropanol (7:2) als Laufmittel ergibt 4-Hydroxy-1-[2-(4,6-dimethyl-1H-indol-3-yl)ethyl]-piperidin-3-carbonsäureethylester als gelbes Oel, welches gemäss $^1H$-NMR-Spektrum überwiegend das cis-Isomere enthält.

In analoger Weise erhält man ausgehend von 7,3 g 4-Hydroxy-1-[2-(4,6-dimethyl-1H-indol-3-yl)ethyl]-1,2,5,6-tetrahydro-pyridin-3-carbonsäuremethylester ein Gemisch von cis- und trans-4-Hydroxy-1-[2-(4,6-dimethyl-1H-indol-3-yl)ethyl]-piperidin-3-carbonsäuremethylester, welches ebenfalls überwiegend in der cis-Form vorliegt; Smp. (nach Umkristallisation aus Isopropanol) 138-140°.

Beispiel 19: Man versetzt eine Lösung von 19,3 g (0,1 Mol) 4-Oxo-piperidin-3-carbonsäuremethylester-hydrochlorid in 60 ml Eisessig mit 8,1 ml 37 %-iger wässriger Formaldehydlösung und 11 g (0,1 Mol) Indol. Nach 2-stündigem Rühren bei Raumtemperatur wird mit Eis gekühlt, mit 300 ml Dichlormethan versetzt und mit n-Natronlauge

neutralisiert. Die organische Phase wird abgetrennt, mit Wasser gewaschen, getrocknet und das Lösungsmittel abgedampft. Der Rückstand wird durch Flash-Chromatographie an Kieselgel mit Toluol/Essigsäureäthylester (3:2) gereinigt. Man erhält 1-(1H-Indol-3-ylmethyl)-piperidin-4-on-3-carbonsäuremethylester bzw. 4-Hydroxy-1-(1H-indol-3-yl-methyl)-1,2,5,6-tetrahydro-pyridin-3-carbonsäuremethylester, der zur Identifizierung ausgehend von 14,3 g durch Lösen in Aceton und Versetzen mit 5,8 g Fumarsäure, gelöst in 30 ml Aceton, Abkühlen und Absaugen in das Fumarat von Smp. 140-142° überführt werden kann.

Beispiel 20: In eine Lösung 11,59 g (40,5 mMol) 4-Hydroxy-1-(1H-indol-3-ylmethyl)-1,2,5,6-tetrahydro-pyridin-3-carbonsäuremethylester in 150 ml Methanol wird unter Kühlen auf 0-5° portionsweise 1,53 g (4,05 mMol) Natriumborhydrid eingetragen. Nach 2 Stunden wird das Lösungsmittel abgedampft, der Rückstand in Dichlormethan aufgenommen, mit Wasser extrahiert, getrocknet und zur Trockne eingedampft. Der Rückstand wird über Kieselgel mit Essigsäureäthylester/Methanol (9:1) als Laufmittel chromatographiert. Zunächst wird trans-4-Hydroxy-1-(1H-indol-3-ylmethyl)-piperidin-3-carbonsäuremethylester und dann cis-4-Hydroxy-1-(1H-indol-3-ylmethyl)-piperidin-3-carbonsäuremethylester eluiert. Die einheitlichen Fraktionen werden vereinigt und eingedampft. Das Hemifumarathydrat der trans-Verbindung hat einen Smp. 140-142°, während das Cyclamat der cis-Verbindung bei 170-172° schmilzt.

Beispiel 21: Eine Lösung von 18,1 g (104 mMol) 3-(1H-Indol-3-yl)-aminopropan in 150 ml Methanol wird tropfenweise mit 22 ml (230 mMol) Acrylsäuremethylester versetzt. Nach 15-stündigem Rühren bei Raumtemperatur dampft man unter vermindertem Druck ein und erhält so N-[3-(1H-Indol-3-yl)propyl]-imino-di(3-propionsäure)-dimethylester als rötliches Oel.

Beispiel 22: Man versetzt unter Kühlen eine Lösung von 12,1 g (35 mMol) N-[3-(1H-Indol-3-yl)propyl]-imino-di(3-propionsäure)-dimethylester in 50 ml Dimethylformamid portionsweise mit 1,85 g

(38,5 mMol) Natriumhydrid (50%-ige Suspension in Mineralöl). Nach 1 Stunde Rühren bei Raumtemperatur giesst man die Lösung auf Eiswasser, säuert mit 2n-Salzsäure an und extrahiert 3-mal mit je 100 ml Diethylether. Hierauf wird die wässrige Lösung 4-mal mit je 500 ml Chloroform extrahiert, die organische Phase abgetrennt, über Natriumsulfat getrocknet und zur Trockne eingedampft. Aus Aceton/Ether kristallisieren das 1-[3-(1H-Indol-3-yl)propyl]-piperidin-4-on-3-carbonsäuremethylester-hydrochlorid bzw. 4-Hydroxy-1-[3-(1H-indol-3-yl)propyl]-1,2,5,6-tetrahydro-pyridin-3-carbonsäuremethylester-hydrochlorid vom Smp. 158-159° aus.

Beispiel 23: In eine Lösung von 10,5 g (30 mMol) 4-Hydroxy-1-[3-(1H-indol-3-yl)propyl]-1,2,5,6-tetrahydro-pyridin-3-carbonsäuremethylester-hydrochlorid in 140 ml Methanol werden unter Kühlen auf 0-5° portionsweise 2,28 g (60 mMol) Natriumborhydrid eingetragen. Nach 1 Stunde Nachrühren wird die Lösung eingedampft und der Rückstand über Kieselgel mit Essigsäureäthylester/Methanol (95:5) als Laufmittel chromatographiert. Zunächst wird trans-4-Hydroxy-1-[3-(1H-indol-3-yl)propyl]-piperidin-3-carbonsäuremethylester (Smp. des Fumarat-Hydrats: 98-100°; Zers.) und dann cis-4-Hydroxy-1-[3-(1H-indol-3-yl)propyl]-piperidin-3-carbonsäuremethylester, (Smp. des Hydrochlorides: 217-219°; Zers.) eluiert.

Beispiel 24: Man tropft zu einer Lösung von 3 g Natriummethanolat in 100 ml Dimethylformamid eine Lösung von 15,72 g 2-[2-(1H-Indol-3-yl)ethyl]-aminomethyl-3-methoxy-penta-2,4-diencarbonsäuremethylester in 150 ml Dimethylformamid und rührt 3 Stunden bei Raumtemperatur. Man engt unter vermindertem Druck auf etwa 150 ml ein und giesst den Rückstand in ein Gemisch von 100 ml 2n-Salzsäure und 50 ml Toluol ein. Das langsam auskristallisierende 4-Hydroxy-1-[2-(1H-indol-3-yl)ethyl]-1,2,5,6-tetrahydropyridin-3-carbonsäuremethylester-hydrochlorid bzw. 1-[2-(1H-Indol-3-yl)ethyl]-piperidin-4-on-3-carbonsäuremethylesterhydrochlorid wird abgenutscht und im Vakuum getrocknet. Es schmilzt bei 185-187° (Zers.). Das Ausgangsmaterial kann z.B. folgendermassen hergestellt werden:

Man lässt eine Lösung von 53,3 g (0,375 Mol) 2 Methyl-3-oxo-pent-4-en-carbonsäuremethylester, hergestellt nach J.A.M. van den Goorbergh und A. van der Gen: Rec. Trav. Chim. Pays-Bas 103, 90 (1984)), 38,5 g Orthoameisensäuretrimethylester und 300 ml p-Toluolsulfonsäurehydrat in 150 Methanol 65 Stunden stehen, neutralisiert hierauf mit 84 mg Natriummethanolat in 3 ml Methanol und destilliert das Lösungsmittel im Vakuum ab. Der Rückstand wird mit 2,5 g Ammoniumdihydrogenphosphat versetzt und auf 190° erhitzt, wobei das freigesetzte Methanol abdestilliert wird. Destillation im Kugelrohr liefert 2-Methyl-3-methoxy-penta-2,4-diencarbonsäuremethylester vom Kp = 65-75° (bei 20 mbar).

Eine gut gerührte Lösung von 39,5 g (0,25 Mol) 2 Methyl-3-methoxy-penta-2,4-diencarbonsäuremethylester, 44,5 g N-Bromsuccinimid und 200 mg Azobisisobutyronitil in 200 ml Tetrachlormethan wird 12 Stunden zum Rückfluss erhitzt, wobei die Lösung mit einer UV-Lampe bestrahlt wird. Nach dem Erkalten filtriert man vom Succinimid ab, wäscht den Filterrückstand 2-mal mit je 30 ml Tetrachlormethan nach, wäscht die vereinigten organischen Phasen mit Natriumcarbonatlösung und Wasser, trocknet über Natriumsulfat und dampft das Lösungsmittel im Vakuum ab. Man erhält 2-Brommethyl-3-methoxy-penta-2,4-diencarbonsäuremethylester als orangerotes Oel.

Eine Lösung von 16 g 1-Amino-2-(1H-indol-3-yl)-ethan, 23,5 g Brommethyl-3-methoxy-penta-2,4-diencarbonsäuremethylester und 39 g N-Ethyl-N,N-diisopropylamin in 500 ml Dimethylformamid wird unter Stickstoff 16 Stunden gerührt. Man engt unter vermindertem Druck auf etwa 250 ml ein, fügt 500 ml Wasser hinzu und schüttelt dreimal mit je 250 ml Dichlormethan aus. Die organischen Phasen werden vereinigt über Natriumsulfat getrocknet und eingedampft. Nach Reinigung durch Chromatographie an Kieselgel mit Toluol/Ethylacetat 9:1 als Laufmittel erhält man 2-[2-(1H-indol-3-yl)ethyl]aminomethyl-3-methoxy-penta-2,4-diencarbonsäuremethylester als gelbes Oel.

Beispiel 25: Man versetzt eine Lösung von 3,8 g (40 mMol) Chlorameisensäuremethylester in 100 ml trockenem Dichlormethan mit 100 mg (4 mMol) wasserfreiem Zinkbromid. Die auf 0° gekühlte Lösung versetzt man tropfenweise mit einer Lösung von 13,5 g (35 mMol) 4-Trimethylsilyloxy-1-[2-(1-Trimethylsilylindol-3-yl)ethyl]-1,2,5,6-tetrahydro-pyridin in 100 ml Dichlormethan. Nach Erwärmen auf Raumtemperatur wird 1 Stunde nachgerührt, hierauf auf 300 ml gesättigte Natriumbicarbonatlösung gegossen und mit Dichlormethan extrahiert. Die vereinigten organischen Phasen werden über Magnesiumsulfat getrocknet und eingedampft. Der Rückstand wird in 150 ml Aethanol gelöst und mit äthanolischer Salzsäurelösung angesäuert. Nach Versetzen mit Diäthylether und Abkühlen kristallisiert 4-Hydroxy-1-[2-(1H-indol-3-yl)ethyl]-1,2,5,6-tetrahydropyridin-3-carbonsäuremethylester-hydrochlorid vom Smp. 187° (Zers.) aus.
Das Ausgangsmaterial kann z.B. folgendermassen hergestellt werden:

Eine Lösung von 10,12 g Diisopropylamin in 100 ml trockenem Tetrahydrofuran wird bei Eisbadtemperatur mit 62,5 ml n-Butyllithium in n-Hexan versetzt. Nachdem man 30 Minuten bei Raumtemperatur gerührt hat, wird die Lösung erneut auf -15° gekühlt und mit einer Lösung von 10,9 g (45 mMol) 1-[2-(Indol-3-yl)ethyl]-piperidin-4-on, hergestellt nach E. Winterfeldt und P. Strehlke, Chem. Ber. 98, 2579 (1965) in 100 ml Tetrahydrofuran versetzt. Nach 15 Minuten tropft man eine Lösung von 10,9 g (100 mMol) Trimethylchlorilan in 50 ml Tetrahydrofuran hinzu. Man lässt über Nacht bei Raumtemperatur rühren, filtriert und dampft unter vermindertem Druck zur Trockne ein. Man erhält so 4-Trimethylsilyloxy-1-[2-(1-trimethylsilyl-indol-3-yl)ethyl]-1,2,5,6 -tetrahydropyridin als hellgelbes Oel.

Beispiel 26: Eine Lösung von 18,3 g eines Gemisches von cis- und trans-4-Brom-1-[2-(1H-indol-3-yl)ethyl]-piperidin-3-carbonsäuremethylester und 8,2 g wasserfreiem Natriumacetat in 100 ml Eisessig wird 16 Stunden im Rückfluss erhitzt. Nach dem Abkühlen wird unter vermindertem Druck eingeengt, zwischen Dichlormethan und wässriger Natriumcarbonatlösung verteilt, die organische Phase getrocknet und

eingedampft. Das Gemisch von cis und trans-4-Acetoxy-1-[2-1H-indol-3-yl)ethyl]-piperidin-3-carbonsäuremethylester wird chromatographisch in seine Komponenten aufgetrennt (vergl. Beispiel 9).

Das Ausgangsmaterial kann man z.B. folgendermassen herstellen: Unter Rühren werden 28,4 g (0,1 Mol) 1-[2-(1H-Indol-3-yl)ethyl)-1,2,5,6-tetrahydro-pyridin-3-carbonsäuremethylester portionsweise in 150 ml einer 33%igen Lösung von Bromwasserstoff in Eisessig eingetragen. Nach 18 Stunden Rühren bei Raumtemperatur wird unter vermindertem Druck eingeengt und zwischen Dichlormethan und wässriger Natriumcarbonatlösung verteilt. Die organische Phase wird über Natriumsulfat getrocknet und das Lösungsmittel im Vakuum abgedampft. Man erhält ein Gemisch von cis und trans 4-Brom-1-[2-(1H-indol-3-yl)ethyl]-piperidin-3-carbonsäuremethylester als gelbes Oel.

Beispiel 27: Man versetzt eine Suspension von 1,2 g Natriumhydrid (50%ige Suspension in Mineralöl) in 25 ml trockenen Tetrahydrofuran mit einer Lösung von 2,7 g (25 mMol) Benzylalkohol in 25 ml Tetrahydrofuran und erhitzt nach Abklingen der Gasentwicklung zum Rückfluss. Nach Erkalten fügt man tropfenweise eine Lösung von 8,2 g (25 mMol) 1-[2-(5-Methoxy-1H-indol-3-yl)ethyl]-1,2,5,6-tetrahydropyridin-3-carbonsäureethylester in 50 ml Tetrahydrofuran hinzu und erhitzt erneut 5 Stunden zum Rückfluss. Nach Erkalten wird das Lösungsmittel eingedampft. Man erhält ein Gemisch von cis- und trans-4-Benzyloxy-1-[2-(5-methoxy-1H-indol-3-yl)ethyl]-piperidin-3-carbonsäureethylester als Oel.

Das Ausgangsmaterial kann wie folgt hergestellt werden: 25,4 g 1-Brom-2-(5-Methoxy-1H-indol-3-yl)ethan, 19,2 g 1,2,5,6-Tetrahydropyridin-3-carbonsäureethylester-hydrochlorid (Guvacinethylester-hydrochlorid) und 39 g N-Ethyl-N,N-diisopropyl-amin werden unter Stickstoff in 750 ml Dimethylformamid gelöst und 16 Stunden gerührt. Dann engt man unter vermindertem Druck auf etwa 300 ml ein, fügt 500 ml Wasser hinzu und schüttelt dreimal mit je 250 ml Dichlormethan aus. Die organischen Phasen werden vereinigt und dreimal mit je 80 ml n-Salzsäure ausgeschüttelt. Die vereinigten

wässrigen Phasen werden mit 40%iger Natronlauge basisch gestellt und erneut dreimal mit je 100 ml Dichlormethan ausgeschüttelt. Die organischen Phasen werden vereinigt, mit Wasser gewaschen, über Natriumsulfat getrocknet und zur Trockne eingedampft. Der Rückstand wird in 100 ml Methanol gelöst und mit methanolischer Salzsäurelösung versetzt. Nach Zusatz von Diäthylether und dem Abkühlen kristallisiert 1-[2-(5-Methoxy-1Hindol-3-yl)ethyl]-1,2,5,6-tetrahydro-pyridin-3-carbonsäureethylester-hydrochlorid vom Smp. 184-6° aus.

Beispiel 28: 5,2 g des Gemisches von cis- und trans-4-Benzyloxy-1-[2-(5-methoxy-1H-indol-3-yl)ethyl]-piperidin-3-carbonsäureethylester werden in 100 ml Methanol gelöst, mit 2 g 10 % Palladium auf Kohle versetzt und in einer Parr-Apparatur 12 Stunden bei Raumtemperatur hydriert. Das Reaktionsgemisch wird über Diatomeenerde filtriert und zur Trockne eingedampft. Der Eindampfrückstand wird über Kieselgel mit Toluol/Essigsäureethylester 9:1 als Laufmittel chromatographiert. Zunächst wird trans-4-Hydroxy-1-[2-(5-Methoxy-1H-indol-3-yl)-ethyl]-piperidin-3-carbonsäureethylester und dann cis-4-Hydroxy-1-[2-(5-Methoxy-1H-indol-3-yl)ethyl]-piperidin-3-carbonsäureethylester eluiert. Die gereinigten Fraktionen werden vereinigt, eingedampft und durch Behandeln mit Fumarsäure in Aceton in die Hemifumarate vom Smp. 104-108° (trans) bzw. Smp. 104-107° (cis) überführt.

Beispiel 29: In analoger Weise wie im Beispiel 14 beschrieben erhält man aus 3,8 g (20 mMol) 5-Methoxytryptamin und 46 ml (42 mMol) Acrylsäureethylester N-[2-(5-Methoxy-1H-indol-3-yl)ethyl]-imino-di(3-propionsäure)diethylester; rötliches Oel, $R_f$ = 0,7 (Essigsäureethylester) und durch Cyclisierung desselben 4-Hydroxy-1-[2-(5-Methoxy-1H-indol-3-yl)ethyl]-1,2,5,6-tetrahydro-pyridin-3-carbonsäureethylester-hydrochlorid-hemihydrat bzw. 1-[2-(5-Methoxy-1H-indol-3-yl)ethyl]-piperidin-4-on-3-carbonsäureethylester-hydrochlorid-hemihydrat vom Smp. 149-152°.

Beispiel 30: In analoger Weise wie im Beispiel 16 beschrieben werden ausgehend von 16,2 g (47 mMol) 4-Hydroxy-1[2-(5-Methoxy-1H-indol-3-yl)ethyl]-1,2,5,6-tetrahydro-pyridin-3-carbonsäureethylester durch Reduktion mit 2,7 g (70,5 mMol) Natriumborhydrid und chromatographische Diastereomerentrennung trans-4-Hydroxy-1-[2-(5-Methoxy-1H-indol-3-yl)ethyl]-piperidin-3-carbonsäureethylester, $R_f$ = 0,53 (Essigsäureethylester/Isopropanol 7:2) und cis-4-Hydroxy-1-[2-(5-Methoxy-1H-indol-3-yl)ethyl]-piperidin-3-carbonsäureethylester, $R_f$ = 0,34, sowie durch anschliessende Behandlung mit Fumarsäure deren partiell hydratisierte Semifumarate vom Smp. 104-108° (0,27 x $H_2O$, trans) bzw. 104-107° (0,8 x $H_2O$, cis) erhalten.

Beispiel 31: Man versetzt eine siedende Lösung von 11 g Phenylhydrazin in 280 ml Methanol und 20 ml Wasser mit einer Lösung von 24,3 g (0,1 Mol) 4-(cis-3-Methoxycarbonyl-4-methoxy-piperidino)-butanal in 50 ml Methanol und erhitzt unter Rühren 20 Stunden zum Rückfluss. Das Lösungsmittel wird am Vakuum eingedampft und der Rückstand auf 500 g Kieselgel chromatographisch mit Toluol/ Ethylacetat (9:1) gereinigt. Man erhält so cis-4-Methoxy-1-[2-(1H-indol-3-yl)ethyl]-piperidin-3-carbonsäuremethylester; Smp. des Cyclamates: 144-145°.

Das Ausgangsmaterial kann z.B. folgendermassen hergestellt werden: Eine Lösung von 83,6 g (0,5 Mol) 4-Methoxy-nikotinsäuremethylester in 200 ml absolutem Ethanol wird mit 50 ml Eisessig und 2 g Platinoxid versetzt und bei etwa 3 bar Ueberdruck 12 Stunden hydriert. Das Reaktionsgemisch wird über Diatomeenerde filtriert und eingedampft. Nach Zusatz von 200 ml n-Natronlauge wird 4-mal mit je 250 ml Diethylether extrahiert, die organischen Phasen vereinigt, über Natriumsulfat getrocknet und eingedampft. Man erhält von cis-4-Methoxy-piperidin-3-carbonsäuremethylester als gelbes Oel.

55 g 4-Chlor-1,1-dimethoxybutan, 73,4 g (0,35 Mol) cis-4-Methoxy-piperidin-3-carbonsäuremethylester-hydrochlorid und 136,5 g N-Ethyl-N,N-diisopropyl-amin werden unter Stickstoff in 1,2 Liter Dimethylformamid gelöst und 16 Stunden bei 50° gerührt. Man engt unter vermindertem Druck auf etwa 300 ml ein, fügt 500 ml Wasser hinzu und schüttelt

3-mal mit je 250 ml Dichlormethan aus. Die organischen Phasen werden vereinigt, über Natriumsulfat getrocknet und im Vakuum eingedampft. Nach Reinigung über 1 kg Kieselgel mit Toluol/Ethylacetat (9:1) als Laufmittel erhält man 4-(cis-3-Methoxycarbonyl-4-methoxy-piperidino)-1,1-dimethoxybutan als gelbes Oel.

Eine Lösung von 58 g (0,2 Mol) 4-(cis-3-Methoxycarbonyl-4-methoxy-piperidino)-1,1-dimethoxy-butan in 500 ml Dichlormethan wird mit 250 g Kieselgel, welches nach J.M. Conia et al. Synthesis 1978, 63 mit 10 % wässriger Oxalsäurelösung imprägniert ist, versetzt und die Suspension 3 Stunden bei Raumtemperatur gerührt. Nach Abfiltrieren wäscht man mit 5%iger wässriger Natriumbicarbonatlösung, trocknet die organische Phase mit Natriumsulfat und dampft unter vermindertem Druck ein. Man erhält so 4-(cis-3-Methoxycarbonyl-4-methoxy-piperidino) butanal als gelbes Oel.

Beispiel 32: Eine Lösung von 28,5 g (0,1 Mol) 1-[2-(1H-indol-3-yl)ethyl]-1,2,5,6-tetrahydro-pyridin-3-carbonsäuremethylester in 350 ml Dichlormethan wird portionsweise mit insgesamt 52 g 3,5-Dinitroperbenzosäure versetzt und 30 Minuten unter Rühren zum Rückfluss erhitzt. Nach dem Abkühlen wird durch eine Glasfilternutsche filtriert und die Lösung unter Eiskühlung und gutem Rühren tropfenweise mit 25 ml Bortrifluorid-diethyletherat versetzt. Nach 5 Minuten versetzt man mit 10 g wasserfreiem Natriumsulfat und tropfenweise mit 30 ml Schwefelkohlenstoff, und rührt 2 Stunden bei 0° und 1 Stunde bei Raumtemperatur nach. Nach Filtration wird mit wässriger Natriumcarbonat- und Natriumbisulfitlösung gewaschen, die organische Phase getrocknet und eingedampft. Nach Behandeln mit methanolischer Salzsäure der Lösung des Eindampfrückstandes in Methanol wird kristallines 4-Hydroxy-1-[2-(1H-indol-3-yl)ethyl]-1,2,5,6-tetrahydro-pyridin-3-carbonsäuremethylester-hydrochlorid bzw. 1-[2-(1H-indol-3-yl)ethyl]-piperidin-4-on-3-carbonsäuremethylester-hydrochlorid vom Smp. 185-187° (Zers.) erhalten.

Beispiel 33: Man versetzt eine siedende Lösung von 11 g Phenylhydrazin in 280 ml Methanol und 20 ml Wasser mit einer Lösung von 26 g (0,1 Mol) N-(4-Oxobutyl)-imino-di(3-propionsäure)dimethylester in 50 ml Methanol und erhitzt 20 Stunden zum Rückfluss. Das Lösungsmittel wird im Vakuum abgedampft und der Rückstand auf 500 g Kieselgel mit Toluol/Ethylacetat (9:1) als Laufmittel chromatographiert. Man erhält so N-[2-(1H-Indol-3-yl)ethyl]-imino-di(3-propionsäure)dimethylester als rötliches Oel. Sein Oxalat hat einen Smp. von 98-103°. Das Ausgangsmaterial kann folgendermassen hergestellt werden:

55 g 4-Chlor-1,1-dimethoxybutan, 66,2 g (0.35 Mol) Imino-di(3-propionsäure)dimethylester und 56,5 g N-Ethyl-N,N-diisopropylamin werden unter Stickstoff in 1 Liter Dimethylformamid gelöst und 16 Stunden bei 50° gerührt. Man engt unter vermindertem Druck auf etwa 250 ml ein, fügt 500 ml Wasser hinzu und schüttelt 3x mit je 250 ml Dichlormethan aus. Die vereinigten organischen Phasen werden über Natriumsulfat getrocknet und im Vakuum eingedampft. Man erhält so N-(4,4-Dimethoxybutyl)-imino-di(3-propionsäure)dimethylester als orangerotes Oel.

Eine Lösung von 61,1 g (0,2 Mol) N-(4,4-Dimethoxybutyl)-imino-di(3-propionsäure)dimethylester in 500 ml Dichlormethan wird mit 250 g Kieselgel, welches nach J.M. Conia et al.: Synthesis 1978, 63 mit 10 % wässriger Oxalsäurelösung imprägniert ist, versetzt und die Suspension 3 Stunden bei Raumtemperatur gerührt. Nach Abfiltrieren wäscht man mit 5 % wässriger Natriumbicarbonatlösung, trocknet die organische Phase über Natriumsulfat und dampft zur Trockne ein. Man erhält so N-(4-Oxobutyl)-imino-di(3-propionsäure)dimethylester als gelbes Oel.

Beispiel 34: 40.1 g (0.25 Mol) Tryptamin werden in 50 ml Methanol gelöst und unter Rühren bei Raumtemperatur tropfenweise mit 21.52 g (0.25 Mol) Acrylsäuremethylester versetzt, wobei man die Reaktionstemperatur unter 25° hält. Anschliessend lässt man das Reaktionsgemisch 15 Stunden bei Raumtemperatur stehen, kühlt dann auf 5° ab

und versetzt mit etherischer Chlorwasserstofflösung bis zur schwach kongosauren Reaktion, wobei das N-[2-(1H-Indol-3-yl)ethyl]-β-alaninmethylester-hydrochlorid ausfällt. Zur Reinigung wird einmal aus Methanol umkristallisiert, das Produkt schmilzt bei 168-170°. In analoger Weise erhält man ausgehend vom 40.1 g (0.25 Mol) Tryptamin und 25 g (0.25 Mol) Acrylsäureethylester in 50 ml Methanol und anschliessender Versetzung mit Chlorwasserstoff N-[2-(1H-Indol-3-yl)ethyl]-β-alaninethylester-hydrochlorid vom Smp. 130-133°. In analoger Weise erhält man ausgehend von 5,7 g 4,6-Dimethyltryptamin und 2,6 g Acrylsäuremethylester N-[2-(4,6-Dimethyl-1H-indol-3-yl)ethyl]-β-alaninmethylester, der durch Behandeln mit etherischer Chlorwasserstoffsäure in das Hydrochlorid vom Smp. 151-155° überführt werden kann.

Beispiel 35: 28.3 g (0.1 Mol) N-[2-(1H-Indol-3-yl)ethyl]-β-alaninmethylester-hydrochlorid werden in 100 ml Methanol mit 10.5 g (0.1 Mol) Triethylamin und 5.84 g (0.11 Mol) Acrylnitril versetzt und 15 Stunden bei Raumtemperatur gerührt. Hierauf wird im Wasserstrahlvakuum eingeengt, der Rückstand in Ether aufgenommen und mit Eiswasser neutral gewaschen. Die etherische Lösung wird über Kaliumcarbonat getrocknet und eingedampft. Der als Rückstand anfallende N-(2-Cyanoethyl)-[2-(1H-indol-3-yl)ethyl]-β-alaninsäuremethylester wird zur Ueberführung in das Oxalat in 50 ml Aceton gelöst und mit einer Lösung von 9 g Oxalsäure in 50 ml Aceton versetzt. Das ausgefallene Oxalat wird mit wenig Aceton gewaschen, abgesaugt und getrocknet; Smp. 146-147°.

Beispiel 36: In analoger Weise wie in Beispiel 35 beschrieben erhält man ausgehend von 28.3 g (0.1 Mol) N-[2-(1H-Indol-3-yl)ethyl]-β-alaninmethylester-hydrochlorid, 10.5 g (0.1 Mol) Triethylamin und 10.9 g (0.11 Mol) Acrylsäure-(N,N-dimethyl)amid in 100 ml Methanol und unter 6 stündigem Erhitzen zum Rückfluss und anschliessender Ueberführung ins Oxalat N-[2-(1H-Indol-3-yl)ethyl]-N-[2-(N,N-dimethylaminocarbamyl)ethyl]-β-alaninmethylester-oxalat vom Smp. 153°-155° (Zers.).

Beispiel 37: In analoger Weise wie in Beispiel 35 beschrieben erhält man ausgehend von 2.83 (0.01 Mol) N-[2-(1H-Indol-3-yl)ethyl]-β-alaninmethylester-hydrochlorid, 2.77 g (0.002 Mol) Kaliumcarbonat und 1.99 g (0.0011 Mol) 2-(2-Bromethyl)-1,3-dioxolan in 20 ml Dioxan, wobei man 20 Stunden bei einer Reaktionstemperatur von 80° belässt, und nach der Aufarbeitung mit Oxalsäure ins Oxalat überführt, N-[2-(1,3-Dioxolan-2-yl)ethyl]-N-[2-(1H-indol-3-yl)ethyl]-β-alaninmethylester-oxalat vom Smp. 159-161°.

Beispiel 38: In analoger Weise wie in Beispiel 35 beschrieben erhält man ausgehend von 28.3 g (0.1 Mol) N-[2-(1H-Indol-3-yl)ethyl]-β-alaninmethylester-hydrochlorid, 16.8 g (0.11 Mol) Bromessigsäuremethylester und 29 g (0.21 Mol) Kaliumcarbonat in 140 ml Methanol und anschliessender Ueberführung in Hydrochlorid N-(Methoxycarbonylmethyl)-N-[2-(1H-indol-3-yl)ethyl]-β-alaninmethylester-hydrochlorid vom Smp. 168° (Zers.).

Beispiel 39: In analoger Weise wie in Beispiel 35 beschrieben erhält man ausgehend von 29.7 g (0.1 Mol) N-[2-(1H-Indol-3-yl)ethyl]-β-alanin-ethylester-hydrochlorid, 18.4 g (0.11 Mol) Bromessigsäureethylester und 29 g (0.21 Mol) Kaliumcarbonat in 150 ml Ethanol N-(Ethoxycarbonylmethyl)-N-[2-(1H-indol-3-yl)ethyl]-β-alaninethylester und dessen Hydrochlorid vom Smp. 119-121°.

Beispiel 40: Eine Lösung von 6,9 g (20 mMol) N-[2-(1,3-Dioxolan-2-yl)ethyl]-N-[2-(1H-indol-3-yl)ethyl]-β-alaninmethylester in 100 ml Dichlormethan wird mit 30 g Kieselgel, welcher nach J.M. Conia et al.: Synthesis 1978, 63 mit 10% wässriger Oxalsäurelösung imprägniert wurde, versetzt und die Suspension 3 Stunden bei Raumtemperatur gerührt. Nach Abfiltrieren wäscht man mit 5%iger wässriger Natriumbicarbonatlösung, trocknet die organische Phase mit Natriumsulfat und dampft unter vermindertem Druck ein. Der Eindampfrückstand wird zur Reinigung über 300 g Kieselgel mit Toluol/Ethylacetat (9:1) als Laufmittel chromatographiert. Man erhält N-(3-Oxopropyl)-N-[2-(1H-indol 3-yl)ethyl]-β-alaninmethylester als gelbes Oel.

Beispiel 41: Man versetzt eine Lösung von 0,81 g (15 mMol) Natriummethanolat in 5 ml Dimethylformamid mit einer Lösung von 3,3 g (11 mMol) N-(3-Oxopropyl)-N-[2-(1H-indol-3-yl)ethyl]-β-alaninmethylester in 15 ml N,N-Dimethylformamid und rührt 3 Stunden bei 40°. Hierauf wird das Lösungsmittel unter vermindertem Druck abgedampft und der Rückstand durch Chromatographie an 200 g Kieselgel mit Dichlormethan/Methanol (95:5) in die Komponenten aufgetrennt. Zuerst wird trans-4-Hydroxy-1-[2-(1H-indol-3-yl)ethyl]-piperidin-3-carbonsäuremethylester (Semifumarat Smp. 208-209°) und dann cis-4-Hydroxy-1-[2-(1H-indol-3-yl)ethyl]-piperidin-3-carbonsäuremethylester (Hydrochlorid Smp. 187°, Zers.) eluiert.

Beispiel 42: In analoger Weise wie in Beispiel 4 beschrieben erhält man durch Hydrierung von 4-Amino-1-[2-(1H-indol-3-yl)ethyl]-1,2,5,6-tetrahydro-pyridin-3-carbonsäuremethylester cis- und trans-4-Amino-1-[2-(1H-indol-3-yl)ethyl]-piperidin-3-carbonsäuremethylester, die in analoger Weise wie in Beispiel 8 beschrieben in die Bishydrochloride überführt werden können.

Beispiel 43: In eine Suspension von 19,6 g (50 mMol) 1-[2-(1H-Indol-3-yl)ethyl]-3-methoxycarbonyl-4-methoxy-pyridiniumbromid in 250 ml Methanol wird in einer Stickstoffatmosphäre bei 0° innerhalb von 90 Minuten 3,3 g Natriumborhydrid eingetragen. Nach 1 Stunde Rühren bei 0° und 2 Stunden bei Raumtemperatur wird die Suspension unter vermindertem Druck eingedampft, der Rückstand mit 70 ml Wasser versetzt und dreimal mit je 100 ml Dichlormethan extrahiert. Die organischen Phasen werden vereinigt, über Magnesiumsulfat getrocknet und zur Trockne eingedampft. Der Rückstand wird mit einem Gemisch von 80 ml 5n Salzsäure und 20 ml Toluol versetzt, worauf beim Rühren und Abkühlen 4-Hydroxy-1-[2-(1H-indol-3-yl)ethyl]-1,2,5,6-tetra-hydro-pyridin-3-carbonsäuremethylesterhydrochlorid bzw. 1-[2-(1H-Indol-3-yl)ethyl]-piperidin-4-on-3-carbonsäuremethylester-hydrochlorid vom Smp. 184-187° (Zers.) auskristallisiert.

Das Ausgangsmaterial kann z.B. folgendermassen hergestellt werden:

Man versetzt eine Lösung von 22,4 g (0.1 Mol) 1-Brom-2-(1H-indol-3-yl)-ethan in 100 ml Methanol mit 20,8 g 4-Methoxynikotinsäuremethylester und lässt 3 Tage bei Raumtemperatur stehen, worauf 1-[2-(1H-Indol-3-yl)ethyl]-3-methoxycarbonyl-4-methoxy-pryridiniumbromid als orangegelbe Kristalle vom Smp. 133-137° auskristallisieren.

Beispiel 44: Eine Lösung von 4,55 g (0.015 Mol) von 4-Hydroxy-1-[2-(1H-indol-3-yl)ethyl]-1,2,5,6-tetrahydro-pyridin-3-carbonsäurenitril-hydrochlorid in 100 ml 95 % Methanol wird mit 1,5 ml konzentrierter Salzsäure versetzt und 15 Stunden zum Rückfluss erhitzt. Nach dem Erkalten wird unter vermindertem Druck auf ca. 30 ml eingeengt und diese Lösung in ein Gemisch von 80 ml 5n-Salzsäure und 20 ml Toluol eingegossen, worauf beim Rühren und Abkühlen 4-Hydroxy-1-[2-(1H-indol-3-yl)ethyl]-1,2,5,6-tetrahydro-pyridin-3-carbonsäuremethylesterhydrochlorid bzw. 1-[2-(1H-Indol-3-yl)ethyl]-piperidin-4-on-3-carbonsäuremethylesterhydrochlorid vom Smp. 185-187° (Zers.) auskristallisiert.

Das Ausgangsmaterial kann man z.B. folgendermassen herstellen:

Man versetzt eine Suspension von 5,73 g ca. 55 % Natriumhydrid in 100 ml Tetrahydrofuran in einer Stickstoffatmosphäre tropfenweise mit einer Lösung von 12,35 g (41,3 mMol) N-(2-Cyanoethyl)-N-[2-(1H-Indol-3-yl)ethyl]-β-alaninmethylester in 200 ml Tetrahydrofuran und rührt 16 Stunden bei Raumtemperatur. Nach Zusatz von 70 ml 2 normaler wässriger Schwefelsäurelösung erhält man eine gelbe Lösung. Man versetzt diese mit 300 ml Ether und 100 ml Wasser, wobei 2 Schichten entstehen. Die wässrige Schicht wird dreimal mit je 100 ml Ether extrahiert. Die vereinigten organischen Phasen werden über Natriumsulfat getrocknet, unter vermindertem Druck auf etwa 100 ml eingeengt und in ein Gemisch von 80 ml 5n-Salzsäure und 20 ml Toluol eingegossen, worauf beim Rühren und Abkühlen 4-Hydroxy-1-[2-(1H-indol-3-yl)ethyl]-1,2,5,6-tetrahydropyridin-3-carbonsäurenitril-hydrochlorid bzw. 1-[2-(1H-indol-3-yl)ethyl]-piperidin-4-on-3-carbonsäurenitril-hydrochlorid auskristallisiert.

Beispiel 45: Eine Suspension von 10 g Platinoxid in 100 ml Wasser wird mit Wasserstoff reduziert, worauf die Wasserstoffatmosphäre durch reinem Sauerstoff ersetzt wird. Nun fügt man eine Lösung von 5,5 g (20 mMol) cis-4-Hydroxy-3-hydroxymethyl-1-[2-(1H-indol-3-yl)ethyl]-piperidin in 250 ml Aceton, welche mit 3 g Natriumhydrogencarbonat in 50 ml Wasser versetzt wurde, hinzu und leitet 16 Stunden bei 58° Sauerstoff ein. Nach dem Erkalten wird vom Katalysator abfiltriert und das Aceton im Vakuum eingedampft. Die wässrige Phase wird 3 mal mit je 70 ml Dichlormethan extrahiert und mit 1n-Salzsäure angesäuert. Nach Eindampfen unter vermindertem Druck wird der Rückstand mit methanolischer Salzsäure (4n) versetzt, filtriert und die Lösung 3 Stunden zum Rückfluss erhitzt. Nach Erkalten wird das Lösungsmittel unter vermindertem Druck abgedampft, der Rückstand in 50 ml Wasser aufgenommen und mit gesättigter Natriumbicarbonatlösung behandelt. Die so neutralisierte Lösung wird dreimal mit je 70 ml Dichlormethan extrahiert, die vereinigten organischen Phasen über Natriumsulfat getrocknet und eingedampft. Der Rückstand wird durch Chromatographie auf 250 g Kieselgel mit Dichlormethan/Methanol (95:5) als Laufmittel gereinigt, wobei cis-4-Hydroxy-1-[2-(1H-indol-3-yl)ethyl]-piperidin-3-carbonsäuremethylester als Oel erhalten wird; Smp. des Hydrochlorids 187° (Zers.).

Das Ausgangsmaterial kann man z.B. folgendermassen herstellen:

Man versetzt eine Lösung von 31,9 g Quecksilber(II)acetat in 100 ml Wasser und 100 ml Tetrahydrofuran in einer Stickstoffatmospäre bei 5° mit einer Lösung von 25,6 g (0,1 Mol) 3-Hydroxymethyl-1-[2-(1H-Indol-3-yl)ethyl]-1,2,5,6-tetrahydro-pyridin in 100 ml Tetrahydrofuran und rührt 1 Stunde bei 5°. Nach Zusatz von 100 ml einer 3-molaren wässrigen Natriumhydroxidlösung tropft man langsam 100 ml einer 0,5-molaren Natriumborhydridlösung in einer 3-molaren Natriumhydroxidlösung zu, rührt 1 Stunde bei 5° und filtriert. Man dampft das Tetrahydrofuran unter vermindertem Druck ab und extrahiert die wässrige Phase 3-mal mit je 100 ml Diethylether. Die

vereinigten organischen Phasen werden über Natriumsulfat getrocknet und zur Trockne eingedampft. Man erhält ein Gemisch von cis- und trans 4-Hydroxy-3-hydroxymethyl-1-[2-1H-indol-3-yl)ethyl]-piperidin, welches durch Flash-Chromatographie an 1,5 kg Kieselgel mit Dichlormethan/Methanol (9:1) als Laufmittel in die Komponenten aufgetrennt wird. Das aus Acetonitril umkristallisierte cis-Isomere weist einen Smp. von 159,5-161° auf.

Beispiel 46: 22,4 g (0,1 Mol) 1-Brom-2-(1H-Indol-3-yl)-ethan, 15,5 g β-Alaninethylester-hydrochlorid und 39 g N-Ethyl-N,N-diisopropylamin werden unter Stickstoff in 750 ml Dimethylformamid gelöst und 16 Stunden bei Raumtemperatur gerührt. Hierauf wird unter vermindertem Druck auf ca. 200 ml eingeengt, mit 500 ml Wasser versetzt und dreimal mit je 150 ml Dichlormethan ausgeschüttelt. Die vereinigten organischen Phasen werden über Natriumsulfat getrocknet und zur Trockne eingedampft. Durch Versetzen mit äthanolischer Salzsäurelösung und Abkühlen erhält man N-[2-(1H-Indol-3-yl)ethyl]-β-alaninethylester-hydrochlorid vom Smp. 130-133°.

Beispiel 47: 22,4 g (0,1 Mol) 1-Brom-2-(1H-indol-3-yl)-ethan, 19 g Imino-di(3-propionsäure)dimethylester und 39 g N-Ethyl-N,N-diisopropylamin werden unter Stickstoff in 500 ml Dimethylformamid gelöst und 16 Stunden bei 40° gerührt. Man engt unter vermindertem Druck auf ca. 200 ml ein, fügt 500 ml Wasser hinzu und schüttelt dreimal mit je 250 ml Dichlormethan aus. Die organischen Phasen werden vereinigt, über Natriumsulfat getrocknet und zur Trockne eingedampft. Man erhält N-[2-(1H-Indol-3-yl)ethyl]-imino-di(3-propionsäure)dimethylester als rötliches Oel. Sein Oxalat hat einen Smp. von 98-103°.

Beispiel 48: 22,4 g (0,1 Mol) 1-Brom-2-(1H-indol-3-yl)-ethan, 12,5 g Imino-di(3-propionsäure)dinitril und 25 g N-Ethyl-N,N-Diisopropylamin werden unter Stickstoff im 500 ml Dimethylformamid gelöst und 16 Stunden bei 60° gerührt. Man engt unter vermindertem Druck auf ca. 200 ml ein, fügt 500 ml Wasser hinzu und schüttelt dreimal mit je 250 ml Dichlormethan aus. Die organischen Phasen werden

vereinigt, über Natriumsulfat getrocknet und zur Trockne eingedampft. Man erhält N-[2-(1H-indol-3-yl)ethyl]imino-di-(3-propionsäure)dinitril als rötliches Oel.

Eine Lösung von 13,3 g (0,05 Mol) von N-[2-(1H-indol-3-yl)ethyl]-imino-di-(3-propionsäure)dinitril in 250 ml 95 % Methanol wird mit 5 ml konzentrierter Schwefelsäure versetzt und 16 Stunden zum Rückfluss erhitzt. Nach dem Erkalten wird unter vermindertem Druck auf etwa 75 ml eingeengt, mit 200 ml Dichlormethan und 100 ml Wasser versetzt und mit gesättigter Natriumbicarbonatlösung neutralisiert. Die organische Phase wird abgetrennt und die wässrige Phase noch zweimal mit je 100 ml Dichlormethan extrahiert. Die vereinigten organischen Phasen werden über Natriumsulfat getrocknet und eingedampft. Man erhält N-[2-(1H-Indol-3-yl)ethyl]-imino-di(3-propionsäure)dimethylester. Sein Oxalat schmilzt bei 98-103°.

Beispiel 49: 18,2 g 4-{N-Benzyl-N-[2-(1H-indol-3-yl)ethyl]-amino}-buttersäureethylester werden in 500 ml Ethanol gelöst, mit 1,2 g Platinoxid versetzt und in einer Parr-Apparatur bei 5 bar Ueberdruck 18 Stunden bei Raumtemperatur und 3 Stunden bei 40-50° hydriert. Man filtriert durch Diatomeenerde und versetzt die erhaltene enthanolische Lösung von 4-{N-[2-(1H-indol-3-yl)ethyl]-amino}-buttersäureethylester unter Rühren tropfenweise mit 4,2 g Acrylsäureethylester in 25 ml Ethanol. Man lässt 12 Stunden bei Raumtemperatur nachrühren, engt unter vermindertem Druck auf 300 ml ein, kühlt auf 5° ab und säuert mit etherischer Salzsäure auf pH = 5,5 an. Der ausgefallene kristalline Niederschlag wird abgesaugt und getrocknet. Man erhält N-[2-(1H-Indol-3-yl)ethyl]-N-(3-ethoxycarbonylpropyl)-ß-alaninethylester in Form des Hydrochlorides. In analoger Weise erhält man auch N-[2-(1H-Indol-3-yl)ethyl]-N-(3-methoxycarbonylpropyl)-ß-alaninmethylester und sein Hydrochlorid.

Der als Ausgangsmaterial verwendete 4-{N-Benzyl-N-[2-(1H-indol-3-yl)ethyl]-amino}-buttersäureethylester wird durch Benzylierung von 7,5 g Tryptamin mit 8,5 g Benzylbromid und Umsetzung von 12 g des gebildeten 1-Benzylamino-2-(1H-indol-3-yl)-ethan mit 9,7 g 4-Brombuttersäureethylester hergestellt.

Beispiel 50: 18,2 g N-[2-(1H-Indol-3-yl)ethyl]-N-(ethoxycarbonylmethyl)-β-alaninethylester werden in 20 ml Dimethylformamid gelöst und unter Rühren innerhalb von 10 Minuten zu einer Suspension von 4,7 g Natriumethanolat in 25 ml Dimethylformamid getropft. Man rührt 15 Stunden bei Raumtemperatur, dampft unter stark vermindertem Druck bei einer Badtemmperatur von 40° ein, versetzt den Rückstand mit 150 ml Dichlormethan, etwas Eis und 80 ml n-Salzsäure und rührt 5 Minuten. Dann gibt man vorsichtig 7 g Natriumhydrogencarbonat hinzu, trennt im Scheidetrichter und dampft die Dichlormethan-Lösung bei vermindertem Druck ein. Der Rückstand wird aus 25 ml Ethanol umkristallisiert. Man erhält so den 4-Hydroxy-1-[2-(1H-indol-3-yl)-ethyl]-2,5-dihydro-1H-pyrrol-3-carbonsäureethylester bzw. 1-[2-(1H-Indol-3-yl)-ethyl]-pyrrolidin-4-on-3-carbonsäureethylester von Smp. 122-123°.

In analoger Weise erhält man ausgehend von 19,7 g N-[2(1H-Indol-3-yl)ethyl]-N-(3-ethoxycarbonylpropyl)-β-alaninethylester den 4-Hydroxy-1-[2-(1H-indol-3-yl)ethyl]-1,2,5,6-tetrahydro-7H-azepin-3-carbonsäureethylester bzw. 1-[2-(1H-Indol-3-yl)ethyl]-hexahydroazepin-4-on-3-carbonsäureethylester.

Beispiel 51: 12,1 g N-[2-(1H-Indol-3-yl)ethyl]-N-(methoxycarbonylmethyl)-β-alaninmethylester werden in 10 ml Dimethylformamid gelöst und unter Rühren bei 20-30° innerhalb von 15 Minuten zu einer Suspension von 2,8 g Natriummethanolat in 20 ml Dimethylformamid getropft. Man lässt 3 Stunden bei Raumtemperatur rühren, dampft unter vermindertem Druck bei einer Badtemperatur von 40° ein, löst den Rückstand in 150 ml Methanol und gibt bei -5° eine 10%ige Lösung von Salzsäuregas in Ether zu bis pH=5 hinzu. Der gebildete 4-Hydroxy-1-[2-(1H-indol-3-yl)ethyl]-2,5-dihydro-1H-pyrrol-3-carbonsäuremethylester bzw. 1-[2-(1H-Indolyl-3-yl)-ethyl]-pyrrolidin-4-on-3-carbonsäuremethylester kann ohne weitere Reinigung durch Zugabe von 7 Portionen von je 0,4 g Natriumborhydrid (insgesamt 2,8 g) unter Rühren bei -30° innerhalb einer Stunde reduziert werden. Nach beendetem Eintragen lässt man 3 Stunden bei Raumtemperatur rühren, dampft dann bei vermindertem Druck ein, nimmt den Rückstand in Ether auf, versetzt mit Wasser und stellt mit konzentrierter Natronlauge auf pH=9. Die abgetrennte organische Phase wird noch zweimal mit Wasser gewaschen, über Kaliumcarbonat getrocknet und bei vermindertem Druck eingedampft. Der Rückstand wird in 50 ml Aceton gelöst und unter Rühren mit etherischer Salzsäure auf pH=4 gestellt, wobei das Hydrochlorid ausfällt. Nach Umkristallisation aus Methanol erhält man das 4-Hydroxy-1-[2-(1H-indolyl-3-yl)ethyl]-pyrrolidin-3-carbonsäuremethylester-hydrochlorid als cis/trans-Gemisch (ca. 66 % cis) mit einem Smp. von 192-194° (Zers.), das chromatographisch in die Komponenten aufgetrennt werden kann.

Beispiel 52: 4,7 g 1-[2-(1H-Indol-3-yl)-ethyl]-pyrrolidin-4-on-3-carbonsäureethylester werden in 50 ml Ethanol suspendiert und unter Rühren mit etherischer Salzsäure auf pH=5 gestellt. Dann gibt man innerhalb von 4 Stunden bei -10° 5 Portionen von je 0,2 g Natriumborhydrid (insgesamt 1,0 g) zu. Dann lässt man 3 Stunden bei 0-3° rühren, dampft bei vermindertem Druck ein, nimmt den Rückstand in Dichlormethan auf und schüttelt mit Natriumhydrogencarbonat-Lösung aus. Nach dem Trocknen über Magnesiumsulfat wird eingedampft und das cis-trans-Gemisch mittels Flash-Chromatographie über Kieselgel mit Essigester als Laufmittel getrennt. Die trans-Verbindung wird in wenig Ethanol gelöst und mit alkoholischer Salzsäure auf pH=4 gestellt, wobei das trans-4-Hydroxy-1-[2-(1H-indol-3-yl)ethyl]-pyrrolidin-3-carbonsäureethylester-hydrochlorid auskristallisiert; Smp. 152-153°.

Die cis-Verbindung wird als Oxalat isoliert; Smp. 151-152°.

In analoger Weise erhält man ausgehend von 5,3 g 4-Hydroxy-1-[2-(1H-indol-3-yl)ethyl]-1,2,5,6-tetrahydro-7H-azepin-3-carbonsäureethylester den 4-Hydroxy-1-[2-(1H-indol-3-yl)ethyl]-hexahydroazepin-3-carbonsäureethylester als cis/trans-Gemisch.

Beispiel 53: In analoger Weise wie in den Beispielen 1-52 beschrieben kann man ferner herstellen:
4-Amino-1-[2-(1H-indol-3-yl)ethyl]-1,2,5,6-tetrahydro-pyridin-3-carbonsäureethylester oder ein Salz, z.B. das Bishydrochlorid, davon;
cis-4-Amino-1-[2-(1H-indol-3-yl)ethyl]-piperidin-3-carbonsäureethylester oder ein Salz, z.B. das Bishydrochlorid, davon;
trans-4-Amino-1-[2-(1H-indol-3-yl)ethyl]-piperidin-3-carbonsäureethylester oder ein Salz, z.B. das Bishydrochlorid, davon;
N-[2-(5-Methoxy-1H-indol-3-yl)ethyl]-imino-di(3-propionsäure)-dimethylester oder ein Salz, z.B. das Hydrochlorid, davon;
4-Hydroxy-1-[2-(5-methoxy-1H-indol-3-yl)ethyl]-1,2,5,6-tetrahydropyridin-3-carbonsäuremethylester bzw. 1-[2-(5-Methoxy-1H-indol-3-yl)ethyl]-piperidin-3-carbonsäuremethylester oder ein Salz, z.B. das Hydrochlorid, davon;
cis-Hydroxy-1-[2-(5-methoxy-1H-indol-3-yl)ethyl]-piperidin-3-carbonsäuremethylester oder ein Salz, z.B. das Hydrochlorid, davon;
trans-Hydroxy-1-[2-(5-methoxy-1H-indol-3-yl)ethyl]-piperidin-3-carbonsäuremethylester oder ein Salz, z.B. das Hydrochlorid, davon.

Beispiel 54: In analoger Weise wie in den Beispielen 6, 8 und 10 beschrieben kann man auch Fumarate (bzw. Semifumarate), Hydrochloride und Oxalate (bzw. Hydrogenoxalate) anderer Verbindungen gemäss einem der Beispiele 1-53 sowie Hydrobromide und Cyclamate von Verbindungen gemäss einem der Beispiele 1-53 herstellen.

Beispiel 55: Tabletten, enthaltend je 50 mg 4-Hydroxy-1-[2-(4,6-dimethyl-1H-indol-3-yl)ethyl]-1,2,5,6-tetrahydro-pyridin-3-carbonsäuremethylester bzw. dessen Hydrochlorid, können wie folgt hergestellt werden:

Zusammensetzung (1000 Tabletten)

| | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 56: Lacktabletten, enthaltend 100 mg 4-Hydroxy-1-[2-(4,6-dimethyl-1H-indol-3-yl)ethyl]-1,2,5,6-tetrahydro-pyridin-3-carbonsäuremethylester bzw. dessen Hydrochlorid können wie folgt hergestellt werden:

Zusammensetzung (für 1000 Tabletten)

| | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 g |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 57: In analoger Weise wie in den Beispielen 55 und 56 beschrieben kann man auch pharmazeutische Präparate, enthaltend eine andere Verbindung der Formel I gemäss Beispiel 1 bis 53 herstellen.

Beispiel 58: 20 g Rohrzucker und 19 g Bäckerhefe werden in 160 ml Trinkwasser 1 Stunde bei 30° gerührt. Dann gibt man 3,5 g (10 mMol) feinpulverisiertes 4-Hydroxy-1-[2-(1M-indol-3-yl)ethyl]-1,2,5,6-tetrahydro-pyridin-3-carbonsäureethylester hinzu. Man lässt 24 Stunden bei Raumtemperatur rühren, fügt eine 40° warme Lösung von 13 g Rohrzucker in 65 ml Trinkwasser und weitere 4 g Bäckerhefe hinzu und lässt weitere 48 Stunden bei Raumtemperatur rühren. Dann wird über Diatomeenerde filtriert, mit Kaliumcarbonat alkalisch gemacht und zweimal mit je 50 ml Dichlormethan ausgeschüttet. Die organischen Phasen werden vereinigt, über Kaliumcarbonat getrocknet und unter vermindertem Druck zur Trockne eingedampft. Der Rückstand wird chromatographisch (Kieselgel/Essigester) gereinigt. Man erhält 3R,4S-cis-4-Hydroxy-1-[2-(1M-indol-3-yl)ethyl]-piperidin-3-carbonsäureethylester, der mittels etherischer Salzsäure in das Hydrochlorid vom Smp. 108°, $[\alpha]_D^{20}$ = + 46,2±0,6° (C = 1,586 in Ethanol), überführt werden kann.

0187122

Patentansprüche (für alle benannten Vertragsstaaten ausser AT)

1. 1,3,4-trisubstituierte Azacycloalkane bzw. Azacycloalkene der Formel

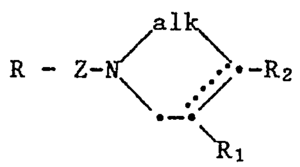

(I),

worin R einen gegebenenfalls substituierten 3-Indolylrest bedeutet, Z Niederalkylen darstellt, welches den Rest R durch 1 bis 3 Kohlenstoffatome vom Stickstoffatom trennt, alk Methylen, Ethylen oder 1,3-Propylen bedeutet, $R_1$ gegebenenfalls verestertes oder amidiertes Carboxy bedeutet und $R_2$ eine gegebenenfalls veretherte oder acylierte Hydroxygruppe oder für eine gegebenenfalls acylierte Aminogruppe steht, und ihre Tautomeren und/oder Salze davon.

2. Eine Verbindung gemäss Anspruch 1, worin R unsubstituiertes oder in mindestens einer der Stellungen 1, 2 sowie 4 bis 7 substituiertes 3-Indolyl bedeutet, wobei als Substituent in 1-Stellung Niederalkyl oder Niederalkenyl, in 2-Stellung Niederalkyl und als Substituent(en) in 4- bis 7-Stellung Niederalkyl, Niederalkenyl, Niederalkoxy, Niederalkenyloxy, Hydroxy, Halogen, Trifluormethyl und/oder an benachbarte C-Atome gebundenes Niederalkyl(id)endioxy in Betracht kommen, Z Niederalkylen darstellt, welches den Rest R vom Stickstoffatom durch 1 bis 3 C-Atome trennt, alk Methylen, Ethylen oder 1,3-Propylen bedeutet, $R_1$ für Carboxy, Niederalkoxycarbonyl, gegebenenfalls durch Niederalkyl, Niederalkoxy, Halogen, Nitro und/oder Trifluormethyl substituiertes Phenylniederalkoxycarbonyl, 3- bis 8-gliedriges Cycloalkoxycarbonyl, Carbamyl, Mono- oder Diniederalkylcarbamyl, Niederalkylen- bzw. Aza-, Oxa- oder Thianiederalkylencarbamyl, N-Niederalkanoylcarbamyl, N-Niederalkoxycarbonylcarbamyl, Ureidocarbonyl, N'-Mono- oder N',N'-Dinie-

deralkylureidocarbonyl, N',N'-Niederalkylen- bzw. N',N'-(Aza-, Oxa- oder Thia)niederalkylenureidocarbonyl steht, und $R_2$ für Hydroxy, Niederalkoxy, gegebenenfalls durch Niederalkyl, Niederalkoxy, Halogen, Trifluormethyl und/oder Nitro substituiertes Phenylniederalkoxy, Amino, Niederalkanoylamino, Niederalkoxycarbonylamino oder gegebenenfalls durch Niederalkyl, Niederalkoxy, Halogen, Trifluormethyl und/oder Nitro substituiertes Phenylniederalkoxycarbonylamino steht, wobei Niederalkylen alk bis und mit 4, die übrigen niederen Reste insgesamt bis und mit 7 und in jeder niederen Teilstruktur bis und mit 4 C-Atome aufweisen, oder ein Tautomeres und/oder Salz davon.

3. Eine Verbindung gemäss Anspruch 1 oder 2, worin alk Ethylen bedeutet.

4. Eine Verbindung gemäss Anspruch 1, worin R unsubstituiertes oder in einer oder zwei der Stellungen 4 bis 7 durch Niederalkyl mit bis und mit 4 C-Atomen, Niederalkoxy mit bis und mit 4 C-Atomen, Halogen der Atomnummer bis und mit 35 und/oder Trifluormethyl substituiertes 3-Indolyl bedeutet, Z Niederalkylen mit bis und mit 4 C-Atomen darstellt, welches den Rest R vom Stickstoffatom durch 1 bis 3 C-Atome trennt, alk Methylen, Ethylen oder 1,3-Propylen bedeutet, $R_1$ für Carboxy, Phenylniederalkoxycarbonyl mit 7 bis und mit 10 C-Atomen Niederalkoxycarbonyl mit bis und mit 4 C-Atomen im Alkoxyteil, 3-bis 8-gliedriges Cycloalkoxycarbonyl, Carbamyl oder N-Mono- oder N,N-Diniederalkylcarbamyl mit bis und mit 4 C-Atomen in jedem Alkylteil steht und $R_2$ Hydroxy, Niederalkoxy mit bis und mit 4 C-Atomen, Niederalkanoyloxy mit bis und mit 4 C-Atomen, Amino oder Niederalkanoylamino mit bis und mit 4 C-Atomen bedeutet, oder ein Tautomeres und/oder Salz davon.

5. Eine Verbindung gemäss Anspruch 1, worin R unsubstituiertes oder in einer oder zwei der Stellungen 1 und 4 bis 7 substituiertes 3-Indolyl bedeutet, wobei als Substituent in 1-Stellung Niederalkyl, und als Substituent(en) in 4- bis 7-Stellung Niederalkoxy mit bis und mit 4 C-Atomen, Halogen der Atomnummer bis und mit 35

und/oder Niederalkyl mit bis und mit 4 C-Atomen in Betracht kommen, alk Ethylen darstellt, Z 1,1-, 1,2- oder 1,3-Niederalkylen mit bis und mit 4 C-Atomen darstellt und $R_1$ Niederalkoxycarbonyl mit bis und mit 4 C-Atomen im Niederalkoxyteil darstellt und $R_2$ Hydroxy oder Amino bedeutet, oder ein Tautomeres und/oder Salz davon.

6. Eine Verbindung gemäss Anspruch 1, worin R unsubstituiertes oder in einer oder zwei der Stellungen 4 bis 7 durch Niederalkyl mit bis und mit 4 C-Atomen und/oder Halogen der Atomnummer bis und mit 35 substituiertes 3-Indolyl bedeutet, Z geradkettiges 1,1-, 1,2- oder 1,3-Niederalkylen mit bis und mit 4 C-Atomen bedeutet, alk Methylen oder Ethylen bedeutet, $R_1$ Niederalkoxycarbonyl mit bis und mit 4 C-Atomen Alkoxyteil darstellt und $R_2$ Hydroxy bedeutet, oder ein Tautomeres und/oder Salz davon.

7. Eine Verbindung gemäss Anspruch 1, worin R unsubstituiertes oder in 4- und 7-Stellung durch Niederalkyl mit bis und mit 4 C-Atomen substituiertes 3-Indolyl bedeutet, Z und alk Ethylen darstellen, $R_1$ Niederalkoxycarbonyl mit bis und mit 4 C-Atomen im Niederalkoxyteil bedeutet und $R_2$ für Hydroxy steht, oder ein Tautomeres und/oder Salz davon.

8. Eine Verbindung gemäss Anspruch 1, worin R unsubstituiertes oder in 5-Stellung durch Methoxy substituiertes 3-Indolyl bedeutet, Z und alk Ethylen darstellen, $R_1$ Methoxycarbonyl bedeutet und $R_2$ für Hydroxy oder Amino bedeutet, oder, sofern der azacyclische Ring keine Doppelbindung aufweist, Niederalkanoyloxy mit bis und mit 4 C-Atomen, z.B. Acetoxy, darstellt, oder ein Tautomeres und/oder ein Salz davon.

9. Eine Verbindung gemäss einem der Ansprüche 3, 5 und 7, worin der azacycloaliphatische Ring keine Doppelbindung aufweist und $R_1$ und $R_2$ zueinander cis-ständig sind.

10. Eine Verbindung gemäss einem der Ansprüche 1, 2, 4, 6 und 8, worin der azacycloaliphatische Ring keine Doppelbindung aufweist und $R_1$ und $R_2$ zueinander cis-ständig sind.

11. 4-Hydroxy-1-[2-(1H-indol-3-yl)ethyl]-1,2,5,6-pyridin-3-carbonsäuremethylester bzw. 1-[2-(1H-Indol-3-yl)ethyl]-piperidin-4-on-3-carbonsäuremethylester, 4-Hydroxy-1-[2-(5-Methoxy-1H-indol-3-yl)-ethyl]-1,2,5,6-tetrahydro-pyridin-3-carbonsäuremethylester bzw. 1-[2-(5-Methoxy-1H-indol-3-yl)ethyl]-piperidin-4-on-3-carbonsäuremethylester oder ein Isomerengemisch von cis- und trans-4-Hydroxy-1-[2-(1H-indol-3-yl)ethyl]-piperidin-3-carbonsäuremethylester oder ihrer Salze bzw. cis- und trans-Hydroxy-1-[2-(5-methoxy-1H-indol-3-yl)ethyl]-piperidin-3-carbonsäuremethylester oder ihrer Salze.

12. cis-4-Hydroxy-1-[2-(1H-indol-3-yl)ethyl]-piperidin-3-carbonsäureethylester oder ein Salze davon.

13. trans-4-Hydroxy-1-[2-(1H-indol-3-yl)ethyl]-piperidin-3-carbonsäureethylester,
cis-4-Hydroxy-1-[2-(1H-indol-3-yl)ethyl]-piperidin-3-carbonsäuremethylester,
4-Hydroxy-1-[2-(1H-indol-3-yl)ethyl]-1,2,5,6-pyridin-3-carbonsäureethylester bzw. 1-[2-(1H-Indol-3-yl)ethyl]-piperidin-4-on-3-carbonsäureethylester,
trans-4-Hydroxy-1-[2-(1H-indol-3-yl)ethyl]-piperidin-3-carbonsäuremethylester,
cis-4-Acetoxy-1-[2-(1H-indol-3-yl)ethyl]-piperidin-3-carbonsäuremethylester,
trans-4-Acetoxy-1-[2-(1H-indol-3-yl)ethyl]-piperidin-3-carbonsäuremethylester,
4-Amino-1-[2-(1H-indol-3-yl)ethyl]-1,2,5,6-tetrahydro-pyridin-3-carbonsäuremethylester,
4-Amino-1-[2-(1H-indol-3-yl)ethyl]-piperidin-3-carbonsäureamid,
4-Hydroxy-1-[2-(4,6-dimethyl-1H-indol-3-yl)ethyl]-1,2,5,6-tetrahydro-pyridin-3-carbonsäureethylester bzw. 1-[2-(4,6-Dimethyl-1H-indol-3-yl)ethyl]-piperidin-4-on-3-carbonsäureethylester,

4-Hydroxy-1-[2-(5-chlor-1H-indol-3-yl)ethyl]-1,2,5,6-tetrahydro-pyridin-3-carbonsäureethylester bzw. 1-[2-(5-Chlor-1H-indol-3-yl)-ethyl]-piperidin-4-on-3-carbonsäureethylester,
cis-4-Hydroxy-1-[2-(4,6-dimethyl-1H-indol-3-yl)ethyl]-piperidin-3-carbonsäureethylester,
trans-4-Hydroxy-1-[2-(4,6-dimethyl-1H-indol-3-yl)ethyl]-piperidin-3-carbonsäureethylester,
cis-4-Hydroxy-1-[2-(5-chlor-1H-indol-3-yl)ethyl]-piperidin-3-carbonsäureethylester,
trans-4-Hydroxy-1-[2-(5-chlor-1H-indol-3-yl)ethyl]-piperidin-3-carbonsäureethylester,
4-Hydroxy-1-[(1H-indol-3-yl)methyl]-1,2,5,6-tetrahydro-pyridin-3-carbonsäuremethylester bzw. 1-[(1H-indol-3-yl)methyl]-piperidin-4-on-3-carbonsäuremethylester,
cis-4-Hydroxy-1-[(1H-indol-3-yl)methyl]-piperidin-3-carbonsäuremethylester,
4-Hydroxy-1-[3-(1H-indol-3-yl)propyl]-1,2,5,6-tetrahydro-pyridin-3-carbonsäuremethylester bzw. 1-[3-(1H-indol-3-yl)propyl]-piperidin-4-on-3-carbonsäuremethylester,
cis-4-Hydroxy-1-[3-(1H-indol-3-yl)propyl]-piperidin-3-carbonsäuremethylester oder
cis-4-Amino-1-[2-(1H-indol-3-yl)ethyl]-piperidin-3-carbonsäureethylester oder jeweils ein Salz davon.

14. trans-4-Amino-1-[2-(1H-indol-3-yl)ethyl]-piperidin-3-carbonsäureethylester,
trans-4-Hydroxy-[(1H-indol-3-yl)methyl]-piperidin-3-carbonsäuremethylester,
trans-4-Hydroxy-1-[3-(1H-indol-3-yl)propyl]-piperidin-3-carbonsäuremethylester,
cis- und/oder trans-4-Benzyloxy-1-[2-(5-methoxy-1H-indol-3-yl)ethyl]-piperidin-3-carbonsäureethylester,

4-Hydroxy-1-[2-(5-methoxy-1H-indol-3-yl)ethyl]-1,2,5,6-tetrahydropyridin-3-carbonsäureethylester bzw. 1-[2-(5-Methoxy-1H-indol-3-yl)ethyl]-piperidin-4-on-3-carbonsäureethylester,

cis-4-Hydroxy-1-[2-(5-methoxy-1H-indol-3-yl)ethyl]-piperidin-3-carbonsäureethylester,

trans-4-Hydroxy-1-[2-(5-methoxy-1H-indol-3-yl)ethyl]-piperidin-3-carbonsäureethylester,

4-Hydroxy-1-[2-(5-brom-1H-indol-3-yl)ethyl]-1,2,5,6-tetrahydropyridin-3-carbonsäureethylester bzw. 1-[2-(5-Brom-1H-indol-3-yl)-ethyl]-piperidin-4-on-3-carbonsäureethylester.

cis-4-Hydroxy-1-[2-(5-brom-1H-indol-3-yl)ethyl]-piperidin-3-carbonsäureethylester,

trans-4-Hydroxy-1-[2-(5-brom-1H-indol-3-yl)ethyl]-piperidin-3-carbonsäureethylester,

cis-4-Hydroxy-1-[2-(5-fluor-1H-indol-3-yl)ethyl]-piperidin-3-carbonsäureethylester,

trans-4-Hydroxy-1-[2-(5-fluor-1H-indol-3-yl)ethyl]-piperidin-3-carbonsäureethylester,

4-Hydroxy-1-[2-(5-brom-(1H-indol-3-yl)ethyl]-1,2,5,6-tetrahydropyridin-3-carbonsäuremethylester bzw. 1-[2-(5-Brom-1H-indol-3-yl)ethyl]-piperidin-4-on-3-carbonsäuremethylester,

4-Hydroxy-1-[2-(5-fluor-1H-indol-3-yl)ethyl]-1,2,5,6-tetrahydro-pyridin-3-carbonsäureethylester bzw. 1-[2-(5-Fluor-1H-indol-3-yl)ethyl]-piperidin-4-on-3-carbonsäureethylester,

cis-4-Hydroxy-1-[2-(5-fluor-1H-indol-3-yl)ethyl]-piperidin-3-carbonsäuremethylester,

trans-4-Hydroxy-1-[2-(fluor-1H-indol-3-yl)ethyl]-piperidin-3-carbonsäuremethylester,

cis-4-Hydroxy-1-[2-(5-brom-1H-indol-3-yl)ethyl]-piperidin-3-carbonsäuremethylester,

4-Hydroxy-1-[2-(5-fluor-1H-indol-3-yl)ethyl]-1,2,5,6-tetrahydropyridin-3-carbonsäuremethylester bzw. 1-[2-(5-Fluor-1H-indol-3-yl)ethyl]-piperidin-4-on-3-carbonsäuremethylester,

trans-4-Hydroxy-1-[2-(5-brom-1H-indol-3-yl)ethyl]-piperidin-3-carbonsäuremethylester,

4-Hydroxy-1-[2-(4,6-dimethyl-1H-indol-3-yl)ethyl]-1,2,5,6-tetrahydro-pyridin-3-carbonsäuremethylester bzw. 1-[2-(4,6-Dimethyl-1H-indol-3-yl)ethyl]-piperidin-4-on-3-carbonsäuremethylester,

4-Hydroxy-1-[2-(5-chlor-1H-indol-3-yl)-ethyl]-1,2,5,6-tetrahydro-pyridin-3-carbonsäuremethylester bzw. 1-[2-(5-Chlor-1H-indol-3-yl)ethyl]-piperidin-4-on-3-carbonsäuremethylester,

cis-4-Hydroxy-1-[2-(5-chlor-1H-indol-3-yl)ethyl]-piperidin-3-carbonsäuremethylester,

trans-4-Hydroxy-1-[2-(5-chlor-1H-indol-3-yl)ethyl]-piperidin-3-carbonsäuremethylester,

cis-4-Hydroxy-1-[2-(4,6-dimethyl-1H-indol-3-yl)ethyl]-piperidin-3-carbonsäuremethylester,

trans-4-Hydroxy-1-[2-(4,6-dimethyl-1H-indol-3-yl)ethyl]-piperidin-3-carbonsäuremethylester,

cis-4-Methoxy-1-[2-(1H-indol-3-yl)ethyl]-piperidin-3-carbonsäuremethylester,

cis-4-Hydroxy-1-[2-(5-methoxy-1H-indol-3-yl)ethyl]-piperidin-3-carbonsäuremethylester,

trans-4-Hydroxy-1-[2-(5-methoxy-1H-indol-3-yl)ethyl]-piperidin-3-carbonsäuremethylester,

4-Hydroxy-1-[2-(1H-indol-3-yl)ethyl]-2,5-dihydro-1H-pyrrol-3-carbonsäureethylester bzw. 1-[2-(1H-Indol-3-yl)ethyl]-pyrrolidin-4-on-3-carbonsäureethylester,

cis-4-Amino-1-[2-(1H-indol-3-yl)ethyl]-piperidin-3-carbonsäuremethylester,

trans-4-Amino-1-[2-(1H-indol-3-yl)ethyl]-piperidin-3-carbonsäuremethylester,

4-Hydroxy-1-[2-(1H-indol-3-yl)ethyl]-2,5-dihydro-1H-pyrrol-3-carbonsäuremethylester bzw. 1-[2-(1H-Indol-yl)ethyl]-pyrrolidin-4-on-3-carbonsäuremethylester,

cis-4-Hydroxy-1-[2-(1H-indol-3-yl)ethyl]-pyrrolidin-3-carbonsäureethylester,

trans-4-Hydroxy-1-[2-(1H-indol-3-yl)ethyl]-pyrrolidin-3-carbonsäureethylester,

cis-4-Hydroxy-1-[2-(1H-indol-3-yl)ethyl]-pyrrolidin-3-carbonsäuremethylester,
trans-4-Hydroxy-1-[2-(1H-indol-3-yl)ethyl]-pyrrolidin-3-carbonsäuremethylester,
4-Hydroxy-1-[2-(1H-indol-3-yl)ethyl]-1,2,5,6-tetrahydro-7H-azepin-3-carbonsäureethylester bzw. 1-[2-(1H-Indol-3-yl)ethyl]-hexahydroazepin-4-on-3-carbonsäureethylester,
cis-4-Hydroxy-1-[2-(1H-indol-3-yl)ethyl]-hexahydroazepin-3-carbonsäureethylester,
3R,4S-cis-4-Hydroxy-1-[2-(1H-indol-3-yl)ethyl]-piperidin-3-carbonsäureethylester oder
trans-4-Hydroxy-1-[2-(1H-indol-3-yl)ethyl]-hexahydroazepin-3-carbonsäureethylester oder jeweils ein Salz davon.

15. Eine Verbindung gemäss einem der Ansprüche 1, 2, 4, 6, 8, 10 und 14 in Form eines Hydrochlorides, Hydrobromides, Fumarates, Oxalates oder Cyclamates.

16. Eine Verbindung gemäss einem der Ansprüche 3, 5, 7, 9, 11, 12 und 13 in Form eines Hydrochlorides, Hydrobromides, Fumarates, Oxalates oder Cyclamates.

17. Verfahren zur Herstellung von 1,3,4-trisubstituierten Azacycloalkanen und Azacycloalkenen der Formeln

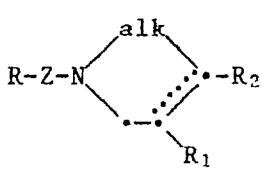

(I),

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, alk Methylen, Ethylen oder 1,3-Propylen bedeutet, $R_1$ gegebenenfalls verestertes oder amidiertes Carboxy bedeutet und $R_2$ für eine

gegebenenfalls veretherte oder acylierte Hydroxygruppe oder für eine gegebenenfalls acylierte Aminogruppe steht, und ihrer Tautomeren und/oder Salze, dadurch gekennzeichnet, dass man

a) zur Herstellung von Verbindungen der Formel I, worin alk Ethylen darstellt, in einer Verbindung der Formel

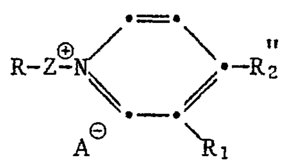

(II),

worin $A^{\ominus}$ ein Säureanion und $R_2''$ veretheres, acyliertes oder geschütztes Hydroxy oder acyliertes oder geschütztes Amino bedeutet, die überschüssigen Doppelbindungen zu Einfachbindungen reduziert und gegebenenfalls vorhandene Schutzgruppen abspaltet, oder

b) eine Verbindung der Formel

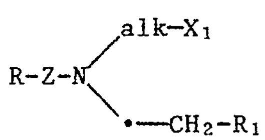

(III),

worin $X_1$ eine Gruppe der Formeln $-CH{=}R_2'$, $-C(X_2){=}R_2'$ oder Cyano darstellt, wobei $X_2$ einen abspaltbaren Rest bedeutet, oder ein Salz davon cyclisiert, oder

c) eine Verbindung der Formel

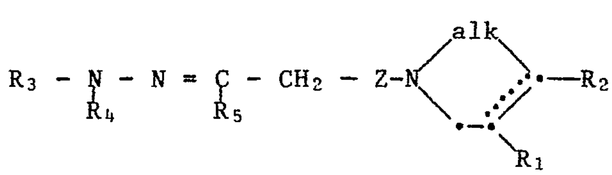

(IV),

worin $R_3$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R

vorgesehenen Substituenten bedeutet, oder ein Tautomeres und/oder Salz davon, zu der entsprechenden Verbindung der Formel I ringschliesst, oder

d) Verbindungen der Formeln

$R - Z - X_3$ (V) und 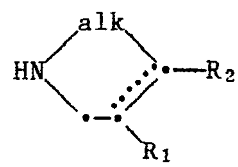 (VI),

worin $X_3$ eine nukleofuge Abgangsgruppe bedeutet, oder deren Tautomere und/oder Salze miteinander umsetzt, oder

e) zur Herstellung von Verbindungen der Formel I, worin alk Ethylen darstellt, eine Verbindung der Formel

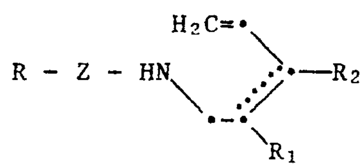 (VII)

oder ein Tautomeres und/oder Salz davon cyclisiert, oder

f) in einer Verbindung der Formel

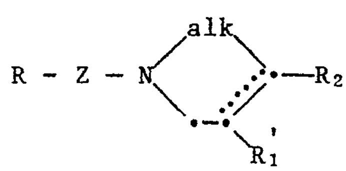 (VIII),

worin $R_1'$ einen in gegebenenfalls verestertes oder amidiertes Carboxy überführbaren Rest bedeutet, oder in einem Tautomeren und/oder Salz davon, $R_1'$ in gegebenenfalls verestertes oder amidiertes Carboxy überführt, oder

g) zur Herstellung von Verbindungen der Formel I, worin der azacycloaliphatische Rest eine Doppelbindung aufweist, eine Verbindung der Formel

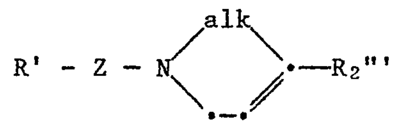 (IX),

worin R' einen in 1-Stellung substituierten oder intermediär geschützten 3-Indolylrest R und $R_2'''$ eine Gruppe $R_2$ oder intermediär geschütztes Hydroxy bedeutet, oder das entsprechende Keto- bzw. Iminotautomere davon mit einer Verbindung der Formel $R_1$-$X_6$ (VIa), worin $X_6$ einen abspaltbaren Rest bedeutet, kondensiert und gegebenenfalls die Schutzgruppe(n) abspaltet, oder

h) in einer Verbindung der Formel

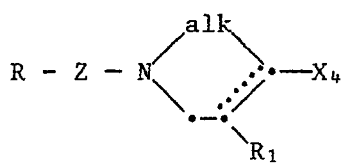 (X),

worin $X_4$ einen in eine Gruppe $R_2$ überführbaren Rest bedeutet, $X_4$ in eine Gruppe $R_2$ überführt, oder

i) zur Herstellung von Verbindungen der Formel I, worin der azacycloaliphatische Ring einfach ungesättigt ist, und $R_2$ Hydroxy bedeutet, bzw. der tautomeren Oxoverbindung, eine Verbindung der Formel

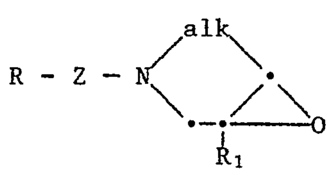 (XI),

umlagert, oder

j) zur Herstellung von Verbindungen der Formel I, worin der azacycloaliphatische Ring gesättigt ist, Verbindungen der Formeln

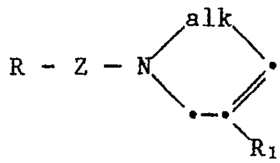 (XII) und $R_2 - H$ (XIII)

miteinander umsetzt, oder

k) zur Herstellung von Verbindungen der Formel I, worin alk den Rest R vom Stickstoffatom durch 1 C-Atom trennt, Verbindungen der Formeln

R–H (L) und 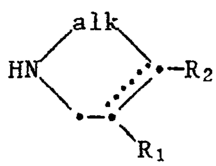 (VI)

mit einem Oxoniederalkan kondensiert und jeweils gewünschtenfalls die verfahrensgemäss erhältliche Verbindung in eine andere Verbindung der Formel I überführt, ein verfahrensgemäss erhältliches Isomerengemisch in die Komponenten auftrennt und das Isomere der Formel I isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere isoliert und/oder eine verfahrensgemäss erhältliche freie Verbindung in ein Salz oder ein verfahrensgemäss erhältliches Salz in die freie Verbindung oder in ein anderes Salz umwandelt.

18. Verbindungen der Formel

$$R - Z - \overset{R_6}{\underset{|}{N}} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3 Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1$"', wobei $R_1$"' eine der für $R_1$ angegebenen

Bedeutungen hat oder für Cyano, Hydroxymethyl oder gegebenenfalls acetalisiertes Formyl steht und alk Methylen, Ethylen oder 1,3-Propylen bedeutet, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihre Salze.

19. Eine Verbindung gemäss Anspruch 18, worin R, Z und $R_1$ die in Anspruch 78 angegebenen Bedeutungen haben, und $R_6$ für Wasserstoff oder eine Gruppe der Formel $-(CH_2)_n-R_1'''$ steht, worin n 2 darstellt und $R_1'''$ Formyl oder Iminomethyl bedeutet oder, sofern $R_1$ freies oder verestertes Carboxy darstellt, ebenfalls freies oder verestertes Carboxy bedeutet bzw., sofern $R_1$ Niederalkoxycarbonyl darstellt, Formyl, Hydroxymethyl oder Cyano bedeutet, oder ein Salz davon.

20. Eine Verbindung gemäss Anspruch 18, worin R unsubstituiertes oder in 5-Stellung durch Methoxy substituiertes 3-Indolyl bedeutet, Z für Ethylen steht, $R_1$ Methoxycarbonyl bedeutet und $R_6$ Wasserstoff oder eine Gruppe der Formel $-(CH_2)_n-R_1'''$ darstellt, in der n für 2 und $R_1'''$ für Methoxycarbonyl oder Formyl steht, oder ein Salz davon.

21. Eine Verbindung gemäss Anspruch 18, worin R unsubstituiertes oder in einer oder zwei der Stellungen 4 bis 7 durch Niederalkyl mit bis und mit 4 C-Atomen, Niederalkoxy mit bis und mit 4 C-Atomen, Halogen der Atomnummer bis und mit 35 und/oder Trifluormethyl substituiertes 3-Indolyl bedeutet, Z Niederalkylen mit bis und mit 4 C-Atomen darstellt, welches den Rest R vom Stickstoffatom durch 1 bis 3 C-Atome trennt, $R_1$ für Carboxy, Niederalkoxycarbonyl mit bis und mit 4 C-Atomen im Alkoxyteil, 3- bis 8-gliedriges Cycloalkoxycarbonyl, Carbamyl oder N-Mono- oder N,N-Diniederalkylcarbamyl mit bis und mit 4 C-Atomen in jedem Alkylteil steht bis und $R_6$ für Wasserstoff oder eine Gruppe der Formel $-(CH_2)_n-R_1'''$ bedeutet, worin n 1, 2 oder 3 ist und $R_1'''$ Carboxy, mit bis und mit 4 C-Atomen im Niederalkoxyteil, 3- bis 8-gliedriges Cycloalkoxycarbonyl, Carbamyl oder N-Mono- oder N,N-Diniederalkylcarbamyl mit bis und mit 4 C-Atomen in

jedem Alkylteil Cyano, Hydroxymethyl, Formyl, Diniederalkoxymethyl oder Niederalkylendioxymethyl mit bis und mit 4 C-Atomen in den Alkoxyteilen bzw. im Alkylendioxyteil bedeutet, oder ein Salz davon.

22. Eine Verbindung gemäss Anspruch 79, worin R unsubstituiertes oder in 4- und 7-Stellung durch Halogen der Atomnummer bis und mit 35 und/oder Niederalkyl mit bis und mit 4 C-Atomen substituiertes 3-Indolyl bedeutet, Z Ethylen darstellt, $R_1$ Niederalkoxycarbonyl mit bis und mit 4 C-Atomen im Niederalkoxyteil bedeutet und $R_6$ Wasserstoff oder eine Gruppe der Formel $-(CH_2)_n-R_1'''$ bedeutet, worin n 1 oder 2 ist und $R_1'''$ die gleiche Bedeutung wie $R_1$ hat oder Cyano, Diniederalkoxymethyl mit je bis und mit 4 Alkoxy-C-atomen, Niederalkylendioxymethyl mit 2 bis und mit 4 Alkylendioxy-C-atomen oder N,N-Diniederalkylcarbamyl mit je bis und mit 4 Alkyl-C-atomen bedeutet, oder ein Salz davon.

23. N-[2-(1H-Indol-3-yl)ethyl]-imino-di(3-propionsäure)dimethylester,
N-[2-(1H-Indol-3-yl)ethyl]-imino-di(3-propionsäure)diethylester,
N-[2-(4,6-Dimethyl-1H-indol-3-yl)ethyl]-imino-di(3-propionsäure)diethylester,
N-[2-(4,6-Dimethyl-1H-indol-3-yl)-ethyl]-imino-di(3-propionsäure)dimethylester,
N-[2-(5-Chlor-1H-indol-3-yl)ethyl]-imino-di(3-propionsäure)diethylester,
N-[2-(5-Chlor-1H-indol-3-yl)ethyl]-imino-di(3-propionsäure)dimethylester,
N-[3-(1H-Indol-3-yl)propyl]-imino-di(3-propionsäure)dimethylester,
N-[2-(5-Methoxy-1H-indol-3-yl)ethyl]-imino-di(3-propionsäure)diethylester,
3-[2-(1H-Indol-3-yl)ethyl-N-(2-cyanoethyl)-β-alaninmethylester,
N-[2-(1H-Indol-3-yl)ethyl]-N-[2-(1,3-dioxolan-2-yl)ethyl]-β-alaninmethylester,
N-[2-(1H-Indol-3-yl)ethyl]-N-[2-(N,N-dimethylcarbamyl)ethyl]-β-alaninethylester,

N-[2-(1H-Indol-3-yl)ethyl]-N-(methoxycarbonylmethyl)-β-alanin-methylester,
N-[2-(1H-Indol-3-yl)ethyl]-N-(ethoxycarbonylmethyl)-β-alanin-ethylester,
N-[2-(5-Brom-1H-indol-3-yl)ethyl]-imino-di(3-propionsäure)diethylester,
N-[2-(5-Brom-1H-indol-3-yl)ethyl]-imino-di(3-propionsäure)di-methylester,
N-[2-(5-Fluor-1H-indol-3-yl)ethyl]-imino-di(3-propionsäure)-dimethylester,
N-[2-(5-Fluor-1H-indol-3-yl)ethyl]-imino-di(3-propionsäure)-diethylester,
N-[2-(4,6-Dimethyl-1H-indol-3-yl)ethyl]-β-alaninmethylester,
N-[2-(1H-Indol-3-yl)ethyl]-N-(3-ethoxycarbonylpropyl)-β-alaninethylester,
N-[2-(1H-Indol-3-yl)ethyl]-N-(3-methoxycarbonylpropyl)-β-alaninmethylester,
N-[2-(5-Methoxy-1H-indol-3-yl)ethyl]-imino-di(3-propionsäure)-dimethylester oder
N-[2-(1H-Indol-3-yl)ethyl]-N-(4-oxobutyl)-β-alaninmethylester
oder ein Salz davon.

24. N-[2-(1H-Indol-3-yl)ethyl]-imino-di(3-propionsäure)diethylester,
N-[2-(4,6-Dimethyl-1H-indol-3-yl)ethyl]-imino-di(3-propionsäure)-diethylester,
N-[2-(4,6-Dimethyl-1H-indol-3-yl)-ethyl]-imino-di(3-propionsäure)dimethylester oder
N-[2-(5-Chlor-1H-indol-3-yl)ethyl]-imino-di(3-propionsäure)diethylester oder jeweils ein Salz davon.

25. N-[2-(1H-Indol-3-yl)ethyl]-imino-di(3-propionsäure)dimethyl-ester,
N-[2-(5-Methoxy-1H-indol-3-yl)ethyl]-imino-di(3-propionsäure)-dimethylester oder
N-[2-(1H-Indol-3-yl)ethyl]-N-(4-oxobutyl)-β-alaninmethylester.

26. Eine Verbindung gemäss einem der Ansprüche 18-25 in Form eines Hydrochlorides, Hydrobromides, Fumarates, Oxalates oder Cyclamates.

27. Verfahren zur Herstellung von Verbindungen der Formel

$$R - Z - \overset{\overset{\displaystyle R_6}{|}}{N} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1'''$, wobei $R_1'''$ eine der für $R_1$ angegebenen Bedeutungen hat oder für Cyano, Hydroxymethyl oder gegebenenfalls acetalisiertes Formyl steht und alk Methylen, Ethylen oder 1,3-Propylen bedeutet, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihrer Salze, dadurch gekennzeichnet, dass man

l) Verbindungen der Formeln

$R-Z-X_5$ (XXXII) und $X_6-CH_2CH_2-R_1$ (XXXIII),

worin einer der Reste $X_5$ und $X_6$ eine Gruppe der Formel $-N(R_6)-H$ und der andere eine nukleofuge Abgangsgruppe bedeutet, oder deren Salze miteinander umsetzt, oder

m) Verbindungen der Formeln

$R-Z-N(R_6)-H$ (XXXVII) und $CH_2=CH-R_1$ (XXXIV)

oder deren Salze miteinander umsetzt, oder

n) eine Verbindung der Formel

$$R_3 - \underset{R_4}{N} - N = \underset{R_5}{C} - CH_2 - Z - \underset{R_6}{N} - CH_2CH_2 - R_1 \qquad (XXXV)$$

worin $R_6$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel XVI ringschliesst, oder

o) in einer Verbindung der Formel

$$R - Z - \overset{R_6'}{N} - CH_2CH_2 - R_1' \qquad (XXXVI)$$

worin $R_6'$ Wasserstoff oder eine Gruppe der Formel alk-$R_1'$ und $R_1'$ einen in gegebenenfalls verestertes oder amidiertes Carboxy $R_1$ überführbaren Rest bedeutet, $R_1'$ in gegebenenfalls verestertes oder amidiertes Carboxy überführt und gewünschtenfalls die verfahrensgemäss erhältliche Verbindung in eine andere Verbindung der Formel XVI überführt, ein verfahrensgemäss erhältliches Isomerengemisch in die Komponenten auftrennt und das Isomere der Formel XVI isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere isoliert und/oder eine verfahrensgemäss erhältliche freie Verbindung in ein Salz oder ein verfahrensgemäss erhältliches Salz in die freie Verbindung oder in ein anderes Salz umwandelt.

28. Verbindungen der Formel

$$R - Z - \overset{R_6}{N} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1$"', wobei $R_1$"' eine der für $R_1$ angegebenen Bedeutungen hat oder für Cyano, Hydroxymethyl oder gegebenenfalls acetalisiertes Formyl, steht und alk Methylen, Ethylen oder 1,3-Propylen bedeutet, und ihre pharmazeutisch verwendbaren Salze zur Anwendung in einem Verfahren zur Behandlung des menschlichen oder tierischen Körpers.

29. N-[2-(1H-Indol-3-yl)ethyl]-β-alaninethylester oder N-[2-(1H-Indol-3-yl)ethyl]-β-alaninmethylester oder jeweils ein pharmazeutisch verwendbares Salz davon zur Anwendung gemäss Anspruch 28.

30. Eine Verbindung gemäss einem der Ansprüche 1, 2, 4, 6, 10, 14, 15, 18-26, 28 und 29 zur Anwendung in einem Verfahren zur therapeutischen Behandlung des menschlichen oder tierischen Körpers.

31. Eine Verbindung gemäss einem der Ansprüche 3, 5, 7-9, 11-13 und 16 zur Anwendung in einem Verfahren zur therapeutischen Behandlung des menschlichen oder tierischen Körpers.

32. Pharmazeutische Präparate enthaltend eine Verbindung gemäss einem der Ansprüche 1, 2, 4, 6, 14, 13, 15, 18-26, 29 und 30 neben üblichen pharmazeutischen Hilfs- und/oder Trägerstoffen.

33. Pharmazeutische Präparate enthaltend eine Verbindung gemäss einem der Ansprüche 3, 5, 7-9, 11-13, 16 und 31 neben üblichen pharmazeutischen Hilfs- und/oder Trägerstoffen.

34. Die gemäss dem Verfahren eines der Ansprüche 17 und 27 erhältlichen neuen Verbindungen.

35. Verwendung einer Verbindung gemäss einem der Ansprüche 1-16, 18-26 und 28-31 zur Herstellung eines Arzneimittels auf nichtchemischem Wege.

Patentansprüche (für den Vertragsstaat AT)

1. Verfahren zur Herstellung von 1,3,4-trisubstituierten Azacycloalkanen und Azacycloalkenen der Formeln

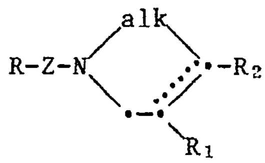

(I),

worin R einen gegebenenfalls substituierten 3-Indolylrest bedeutet, Z Niederalkylen darstellt, welches den Rest R durch 1 bis 3, Kohlenstoffatome vom Stickstoffatom trennt, alk Methylen, Ethylen oder 1,3-Propylen bedeutet, $R_1$ gegebenenfalls verestertes oder amidiertes Carboxy bedeutet und $R_2$ für eine gegebenenfalls veretherte oder acylierte Hydroxygruppe oder für eine gegebenenfalls acylierte Aminogruppe steht, und ihrer Tautomeren und/oder Salze, dadurch gekennzeichnet, dass man

a) zur Herstellung von Verbindungen der Formel I, worin alk Ethylen darstellt, in einer Verbindung der Formel

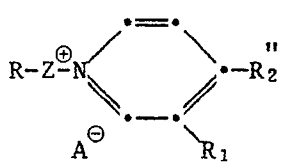

(II),

worin $A^{\ominus}$ ein Säureanion und $R_2''$ veretheres, acyliertes oder geschütztes Hydroxy oder acyliertes oder geschütztes Amino bedeutet, die überschüssigen Doppelbindungen zu Einfachbindungen reduziert und gegebenenfalls vorhandene Schutzgruppen abspaltet, oder

b) eine Verbindung der Formel

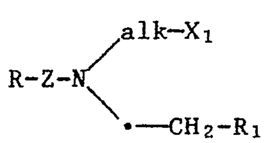

(III),

worin $X_1$ eine Gruppe der Formeln $-CH=R_2'$, $-C(X_2)=R_2'$ oder Cyano darstellt, wobei $X_2$ einen abspaltbaren Rest bedeutet, oder ein Salz davon cyclisiert, oder

c) eine Verbindung der Formel

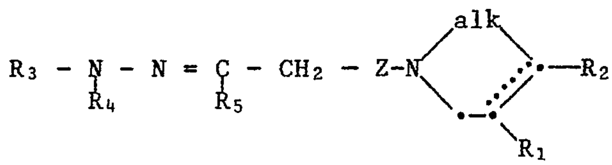 (IV),

worin $R_3$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel I ringschliesst, oder

d) Verbindungen der Formeln

$R - Z - X_3$ (V) und 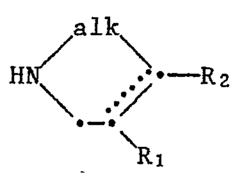 (VI),

worin $X_3$ eine nukleofuge Abgangsgruppe bedeutet, oder deren Tautomere und/oder Salze miteinander umsetzt, oder

e) zur Herstellung von Verbindungen der Formel I, worin alk Ethylen darstellt, eine Verbindung der Formel

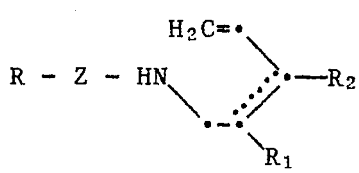 (VII)

oder ein Tautomeres und/oder Salz davon cyclisiert, oder

f) in einer Verbindung der Formel

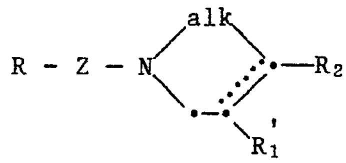 (VIII),

worin $R_1$' einen in gegebenenfalls verestertes oder amidiertes Carboxy überführbaren Rest bedeutet, oder in einem Tautomeren und/oder Salz davon, $R_1$' in gegebenenfalls verestertes oder amidiertes Carboxy überführt, oder

g) zur Herstellung von Verbindungen der Formel I, worin der azacycloaliphatische Rest eine Doppelbindung aufweist, eine Verbindung der Formel

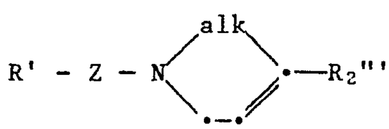 (IX),

worin R' einen in 1-Stellung substituierten oder intermediär geschützten 3-Indolylrest R und $R_2$"' eine Gruppe $R_2$ oder intermediär geschütztes Hydroxy bedeutet, oder das entsprechende Keto- bzw. Iminotautomere davon mit einer Verbindung der Formel $R_1$-$X_6$ (VIa), worin $X_6$ einen abspaltbaren Rest bedeutet, kondensiert und gegebenenfalls die Schutzgruppe(n) abspaltet, oder

h) in einer Verbindung der Formel

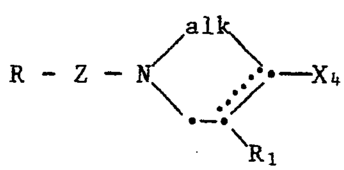 (X),

worin $X_4$ einen in eine Gruppe $R_2$ überführbaren Rest bedeutet, $X_4$ in eine Gruppe $R_2$ überführt, oder

i) zur Herstellung von Verbindungen der Formel I, worin der azacycloaliphatische Ring einfach ungesättigt ist, und $R_2$ Hydroxy bedeutet, bzw. der tautomeren Oxoverbindung eine Verbindung der Formel

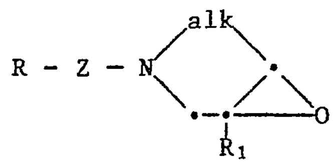 (XI),

umlagert, oder

j) zur Herstellung von Verbindungen der Formel I, worin der azacycloaliphatische Ring gesättigt ist, Verbindungen der Formeln

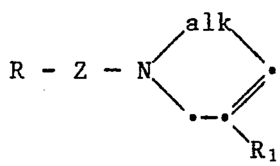 (XII) und $R_2 - H$ (XIII)

miteinander umsetzt, oder

k) zur Herstellung von Verbindungen der Formel I, worin alk den Rest R vom Stickstoffatom durch 1 C-Atom trennt, Verbindungen der Formeln

R–H (L) und 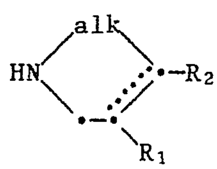 (VI)

mit einem Oxoniederalkan kondensiert und jeweils gewünschtenfalls die verfahrensgemäss erhältliche Verbindung in eine andere Verbindung der Formel I überführt, ein verfahrensgemäss erhältliches Isomerengemisch in die Komponenten auftrennt und das Isomere der Formel I isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere

isoliert und/oder eine verfahrensgemäss erhältliche freie Verbindung in ein Salz oder ein verfahrensgemäss erhältliches Salz in die freie Verbindung oder in ein anderes Salz umwandelt.

2. Verfahren gemäss Anspruch 1 zur Herstellung von Verbindungen der Formel I, worin alk Ethylen ist, dadurch gekennzeichnet, dass man

a) in einer Verbindung der Formel

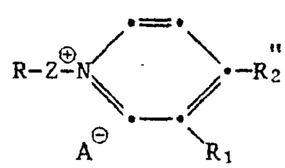

(II),

worin $A^{\ominus}$ ein Säureanion und $R_2$" veretheres, acyliertes oder geschütztes Hydroxy oder acyliertes oder geschütztes Amino bedeutet, die überschüssigen Doppelbindungen zu Einfachbindungen reduziert und gegebenenfalls vorhandene Schutzgruppen abspaltet, oder

b) eine Verbindung der Formel

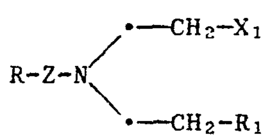

(III'),

worin $X_1$ eine Gruppe der Formeln $-CH=R_2'$, $-C(X_2)=R_2'$ oder Cyano darstellt, wobei $X_2$ einen abspaltbaren Rest bedeutet, oder ein Salz davon cyclisiert, oder

c) eine Verbindung der Formel

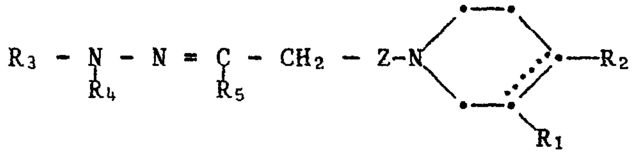

(IV'),

worin $R_3$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel I ringschliesst, oder

d) Verbindungen der Formeln

$R - Z - X_3$ (V) und 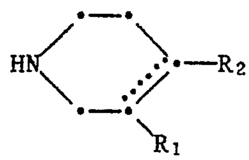 (VI'),

worin $X_3$ eine nukleofuge Abgangsgruppe bedeutet, oder deren Tautomere und/oder Salze miteinander umsetzt, oder

e) eine Verbindung der Formel

$R - Z -$ 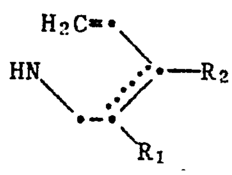 (VII)

oder ein Tautomeres und/oder Salz davon cyclisiert, oder

f) in einer Verbindung der Formel

R – alk – 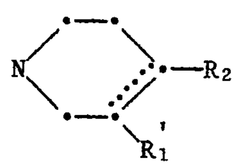 (VIII'),

worin $R_1$' einen in gegebenenfalls verestertes oder amidiertes Carboxy überführbaren Rest bedeutet, oder in einem Tautomeren und/oder Salz davon, $R_1$' in gegebenenfalls verestertes oder amidiertes Carboxy überführt, oder

g) zur Herstellung von Verbindungen der Formel I, worin der azacycloaliphatische Rest eine Doppelbindung aufweist, eine Verbindung der Formel

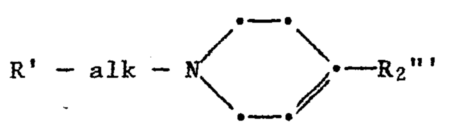

(IX'),

worin R' einen in 1-Stellung substituierten oder intermediär geschützten 3-Indolylrest R und $R_2$"' eine Gruppe $R_2$ oder intermediär geschütztes Hydroxy bedeutet, oder das entsprechende Keto- bzw. Iminotautomere davon mit einer Verbindung der Formel $R_1$-$X_6$ (VIa), worin $X_6$ einen abspaltbaren Rest bedeutet, kondensiert und gegebenenfalls die Schutzgruppe(n) abspaltet und jeweils gewünschtenfalls die verfahrensgemäss erhältliche Verbindung in eine andere Verbindung der Formel I überführt, ein verfahrensgemäss erhältliches Isomerengemisch in die Komponenten auftrennt und das Isomere der Formel I isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere isoliert und/oder eine verfahrensgemäss erhältliche freie Verbindung in ein Salz oder ein verfahrensgemäss erhältliches Salz in die freie Verbindung oder in ein anderes Salz umwandelt.

3. Verfahren gemäss Anspruch 1 zur Herstellung von Verbindungen der Formel I, worin R unsubstituiertes oder in 5-Stellung durch Methoxy substituiertes 3-Indolyl bedeutet, Z und alk Ethylen darstellen, $R_1$ Methoxycarbonyl bedeutet und $R_2$ Hydroxy oder Amino bedeutet oder, sofern der azacyclische Ring keine Doppelbindung

aufweist, Niederalkanoyloxy mit bis und mit 4 C-Atomen, z.B. Acetoxy, darstellt, und ihrer Tautomeren und/oder Salze, dadurch gekennzeichnet, dass man

b) eine Verbindung der Formel

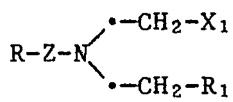

(III')

worin R gegebenenfalls in 5-Stellung durch Methoxy substituiertes 3-Indolyl, Z Ethylen, $R_1$ Methoxycarbonyl und $X_1$ Methoxycarbonyl, Cyano oder eine Gruppe $-CH=R_2'$ bedeutet, in der $R_2'$ Oxo oder Imino ist, cyclisiert und gewünschtenfalls in einer verfahrensgemäss erhältlichen Verbindung, worin $R_2$ Hydroxy oder Amino bedeutet und der azacycloaliphatische Ring eine Doppelbindung aufweist, bzw. in dessen Keto- oder Iminotautomeren die Doppelbindung zur Einfachbindung bzw. die Oxo- oder Iminogruppe zur Hydroxy- oder Aminogruppe reduziert, in einer verfahrensgemäss erhältlichen Verbindung, worin $R_2$ Hydroxy ist und der azacycloaliphatische Ring keine Doppelbindung aufweist, die Hydroxygruppe zu Niederalkanoyloxy verestert und gewünschtenfalls eine verfahrensgemäss erhältliche freie Verbindung in ein Salz oder ein verfahrensgemäss erhältliches Salz in die freie Verbindung oder in ein anderes Salz umwandelt.

4. Verfahren gemäss Anspruch 1, dadurch gekennzeichnet, dass man eine Verbindung der Formel I, worin R unsubstituiertes oder in mindestens einer der Stellungen 1, 2 sowie 4 bis 7 substituiertes 3-Indolyl bedeutet, wobei als Substituent in 1-Stellung Niederalkyl oder Niederalkenyl, in 2-Stellung Niederalkyl und als Substituent(en) in 4- bis 7-Stellung Niederalkyl, Niederalkenyl, Niederalkoxy, Niederalkenyloxy, Hydroxy, Halogen, Trifluormethyl und/oder an benachbarte C-Atome gebundenes Niederalkyl(id)endioxy in Betracht kommen, Z Niederalkylen darstellt, welches den Rest R vom Stickstoffatom durch 1 bis 3 C-Atome trennt, alk Methylen, Ethylen oder 1,3-Propylen bedeutet, $R_1$ für Carboxy, Niederalkoxycarbonyl, gegebenenfalls durch Niederalkyl, Niederalkoxy, Halogen, Nitro

und/oder Trifluormethyl substituiertes Phenylniederalkoxycarbonyl, 3- bis 8-gliedriges Cycloalkoxycarbonyl, Carbamyl, Mono- oder Diniederalkylcarbamyl, Niederalkylen- bzw. Aza-, Oxa- oder Thia-niederalkylencarbamyl, N-Niederalkanoylcarbamyl, N-Niederalkoxy-carbonylcarbamyl, Ureidocarbonyl, N'-Mono- oder N',N'-Diniederalkylureidocarbonyl, N',N'-Niederalkylen- bzw. N',N'-(Aza-, Oxa-oder Thia)niederalkylenureidocarbonyl steht, und $R_2$ für Hydroxy, Niederalkoxy, gegebenenfalls durch Niederalkyl, Niederalkoxy, Halogen, Trifluormethyl und/oder Nitro substituiertes Phenylniederalkoxy, Amino, Niederalkanoylamino, Niederalkoxycarbonylamino oder gegebenenfalls durch Niederalkyl, Niederalkoxy, Halogen, Trifluormethyl und/oder Nitro substituiertes Phenylniederalkoxycarbonylamino steht, wobei Niederalkylen alk bis und mit 4, die übrigen niederen Reste insgesamt bis und mit 7 und in jeder niederen Teilstruktur bis und mit 4 C-Atome aufweisen, oder ein Tautomeres und/oder Salz davon herstellt.

5. Verfahren gemäss Anspruch 1 dadurch gekennzeichnet, dass man eine Verbindung der Formel I, worin R unsubstituiertes oder in einer oder zwei der Stellungen 4 bis 7 durch Niederalkyl mit bis und mit 4 C-Atomen, Niederalkoxy mit bis und mit 4 C-Atomen, Halogen der Atomnummer bis und mit 35 und/oder Trifluormethyl substituiertes 3-Indolyl bedeutet, Z Niederalkylen mit bis und mit 4 C-Atomen darstellt, welches den Rest R vom Stickstoffatom durch 1 bis 3 C-Atome trennt, alk Methylen, Ethylen oder 1,3-Propylen bedeutet, $R_1$ für Carboxy, Niederalkoxycarbonyl mit bis und mit 4 C-Atomen im Alkoxyteil, Phenylniederalkoxy mit 7 bis und mit 10 C-Atomen 3-bis 8-gliedriges Cycloalkoxycarbonyl, Carbamyl oder N-Mono- oder N,N-Diniederalkylcarbamyl mit bis und mit 4 C-Atomen in jedem Alkylteil steht und $R_2$ Hydroxy, Niederalkoxy mit bis und mit 4 C-Atomen, Niederalkanoyloxy mit bis und mit 4 C-Atomen, Amino oder Niederalkanoylamino mit bis und mit 4 C-Atomen bedeutet, oder ein Tautomeres und/oder Salz davon herstellt.

6. Verfahren gemäss Anspruch 1 oder 2, dadurch gekennzeichnet, dass man eine Verbindung der Formel I, worin R unsubstituiertes oder in einer oder zwei der Stellungen 1 und 4 bis 7 substituiertes 3-Indolyl bedeutet, wobei als Substituent in 1-Stellung Niederalkyl, und als Substituent(en) in 4- bis 7-Stellung Niederalkoxy mit bis und mit 4 C-Atomen, Halogen der Atomnummer bis und mit 35 und/oder Niederalkyl mit bis und mit 4 C-Atomen in Betracht kommen, alk Ethylen darstellt, Z 1,1-, 1,2- oder 1,3-Niederalkylen mit bis und mit 4 C-Atomen darstellt und $R_1$ Niederalkoxycarbonyl mit bis und mit 4 C-Atomen im Niederalkoxyteil darstellt und $R_2$ Hydroxy oder Amino bedeutet, oder ein Tautomeres und/oder Salz davon herstellt.

7. Verfahren gemäss Anspruch 1, dadurch gekennzeichnet, dass man eine Verbindung der Formel I, worin R unsubstituiertes oder in einer oder zwei der Stellungen 4 bis 7 durch Niederalkyl mit bis und mit 4 C-Atomen und/oder Halogen der Atomnummer bis und mit 35 substituiertes 3-Indolyl bedeutet, Z geradkettiges 1,1-, 1,2- oder 1,3-Niederalkylen mit bis und mit 4 C-Atomen bedeutet, alk Methylen oder Ethylen bedeutet, $R_1$ Niederalkoxycarbonyl mit bis und mit 4 C-Atomen Alkoxyteil darstellt und $R_2$ Hydroxy bedeutet, oder ein Tautomeres und/oder Salz davon herstellt.

8. Verfahren gemäss Anspruch 1 oder 2, dadurch gekennzeichnet, dass man eine Verbindung der Formel I, worin R unsubstituiertes oder in 4- und 7-Stellung durch Niederalkyl mit bis und mit 4 C-Atomen substituiertes 3-Indolyl bedeutet, Z und alk Ethylen darstellen, $R_1$ Niederalkoxycarbonyl mit bis und mit 4 C-Atomen im Niederalkoxyteil bedeutet und $R_2$ für Hydroxy steht, oder ein Tautomeres und/oder Salz davon herstellt.

9. Verfahren gemäss einem der Ansprüche 2, 6 und 8, dadurch gekennzeichnet, dass man eine Verbindung der Formel I, worin der azacycloaliphatische Ring keine Doppelbindung aufweist und $R_1$ und $R_2$ zueinander cis-ständig sind, oder ein Tautomeres und/oder Salz davon herstellt.

10. Verfahren gemäss einem der Ansprüche 1, 3, 4, 5 und 7, dadurch gekennzeichnet, dass man eine Verbindung der Formel I, worin der azacycloaliphatische Ring keine Doppelbindung aufweist und $R_1$ und $R_2$ zueinander cis-ständig sind, oder ein Tautomeres und/oder Salz davon herstellt.

11. Verfahren gemäss einem der Ansprüche 1-3, dadurch gekennzeichnet, dass man 4-Hydroxy-1-[2-(1H-indol-3-yl)ethyl]-1,2,5,6-pyridin-3-carbonsäuremethylester bzw. 1-[2-(1H-Indol-3-yl)ethyl]-piperidin-4-on-3-carbonsäuremethylester, 4-Hydroxy-1-[2-(5-Methoxy-1H-indol-3-yl)ethyl]-1,2,5,6-tetrahydro-pyridin-3-carbonsäuremethylester bzw. 1-[2-(5-Methoxy-1H-indol-3-yl)ethyl]-piperidin-4-on-3-carbonsäuremethylester oder ein Isomerengemisch von cis- und trans-4-Hydroxy-1-[2-(1H-indol-3-yl)ethyl]-piperidin-3-carbonsäuremethylester oder ihrer Salze, bzw. cis- und trans-Hydroxy-1-[2-(5-methoxy-1H-indol-3-yl)ethyl]-piperidin-3-carbonsäuremethylester oder ihrer Salze herstellt.

12. Verfahren gemäss Anspruch 1 oder 2, dadurch gekennzeichnet, dass man cis-4-Hydroxy-1-[2-(1H-indol-3-yl)ethyl]-piperidin-3-carbonsäureethylester oder ein Salz davon herstellt.

13. Verfahren gemäss Anspruch 1 oder 2, dadurch gekennzeichnet, dass man trans-4-Hydroxy-1-[2-(1H-indol-3-yl)ethyl]-piperidin-3-carbonsäureethylester,
cis-4-Hydroxy-1-[2-(1H-indol-3-yl)ethyl]-piperidin-3-carbonsäuremethylester,
4-Hydroxy-1-[2-(1H-indol-3-yl)ethyl]-1,2,5,6-pyridin-3-carbonsäureethylester bzw. 1-[2-(1H-Indol-3-yl)ethyl]-piperidin-4-on-3-carbonsäureethylester,
trans-4-Hydroxy-1-[2-(1H-indol-3-yl)ethyl]-piperidin-3-carbonsäuremethylester,
cis-4-Acetoxy-1-[2-(1H-indol-3-yl)ethyl]-piperidin-3-carbonsäuremethylester,
trans-4-Acetoxy-1-[2-(1H-indol-3-yl)ethyl]-piperidin-3-carbonsäuremethylester,

4-Amino-1-[2-(1H-indol-3-yl)ethyl]-1,2,5,6-tetrahydro-pyridin-3-carbonsäuremethylester,

4-Amino-1-[2-(1H-indol-3-yl)ethyl]-piperidin-3-carbonsäureamid,

4-Hydroxy-1-[2-(4,6-dimethyl-1H-indol-3-yl)ethyl]-1,2,5,6-tetrahydro-pyridin-3-carbonsäureethylester bzw. 1-[2-(4,6-Dimethyl-1H-indol-3-yl)ethyl]-piperidin-4-on-3-carbonsäureethylester,

4-Hydroxy-1-[2-(5-chlor-1H-indol-3-yl)ethyl]-1,2,5,6-tetrahydro-pyridin-3-carbonsäureethylester bzw. 1-[2-(5-Chlor-1H-indol-3-yl)-ethyl]-piperidin-4-on-3-carbonsäureethylester,

cis-4-Hydroxy-1-[2-(4,6-dimethyl-1H-indol-3-yl)ethyl]-piperidin-3-carbonsäureethylester,

trans-4-Hydroxy-1-[2-(4,6-dimethyl-1H-indol-3-yl)ethyl]-piperidin-3-carbonsäureethylester,

cis-4-Hydroxy-1-[2-(5-chlor-1H-indol-3-yl)ethyl]-piperidin-3-carbonsäureethylester,

trans-4-Hydroxy-1-[2-(5-chlor-1H-indol-3-yl)ethyl]-piperidin-3-carbonsäureethylester,

4-Hydroxy-1-[(1H-indol-3-yl)methyl]-1,2,5,6-tetrahydro-pyridin-3-carbonsäuremethylester bzw. 1-[(1H-indol-3-yl)methyl]-piperidin-4-on-3-carbonsäuremethylester,

trans-4-Hydroxy-1-[(1H-indol-3-yl)methyl]-piperidin-3-carbonsäuremethylester,

cis-4-Hydroxy-1-[(1H-indol-3-yl)methyl]-piperidin-3-carbonsäuremethylester,

4-Hydroxy-1-[3-(1H-indol-3-yl)propyl]-1,2,5,6-tetrahydro-pyridin-3-carbonsäuremethylester bzw. 1-[3-(1H-indol-3-yl)propyl]-piperidin-4-on-3-carbonsäuremethylester,

cis-4-Hydroxy-1-[3-(1H-indol-3-yl)propyl]-piperidin-3-carbonsäuremethylester oder

cis-4-Amino-1-[2-(1H-indol-3-yl)ethyl]-piperidin-3-carbonsäureethylester oder jeweils ein Salz davon herstellt.

14. Verfahren gemäss Anspruch 1, dadurch gekennzeichnet, dass man trans-4-Amino-1-[2-(1H-indol-3-yl)ethyl]-piperidin-3-carbonsäureethylester,

trans-4-Hydroxy-1-[(1H-indol-3-yl)methyl]-piperidin-3-carbonsäuremethylester,
trans-4-Hydroxy-1-[3-(1H-indol-3-yl)propyl]-piperidin-3-carbonsäuremethylester,
cis- und/oder trans-4-Benzyloxy-1-[2-(5-methoxy-1H-indol-3-yl)ethyl]-piperidin-3-carbonsäureethylester,
4-Hydroxy-1-[2-(5-methoxy-1H-indol-3-yl)ethyl]-1,2,5,6-tetrahydropyridin-3-carbonsäureethylester bzw. 1-[2-(5-Methoxy-1H-indol-3-yl)ethyl]-piperidin-4-on-3-carbonsäureethylester,
cis-4-Hydroxy-1-[2-(5-methoxy-1H-indol-3-yl)ethyl]-piperidin-3-carbonsäureethylester,
trans-4-Hydroxy-1-[2-(5-methoxy-1H-indol-3-yl)ethyl]-piperidin-3-carbonsäureethylester,
4-Hydroxy-1-[2-(5-brom-1H-indol-3-yl)ethyl]-1,2,5,6-tetrahydropyridin-3-carbonsäureethylester bzw. 1-[2-(5-Brom-1H-indol-3-yl)-ethyl]-piperidin-4-on-3-carbonsäureethylester,
cis-4-Hydroxy-1-[2-(5-brom-1H-indol-3-yl)ethyl]-piperidin-3-carbonsäureethylester,
trans-4-Hydroxy-1-[2-(5-brom-1H-indol-3-yl)ethyl]-piperidin-3-carbonsäureethylester,
cis-4-Hydroxy-1-[2-(5-fluor-1H-indol-3-yl)ethyl]-piperidin-3-carbonsäureethylester,
trans-4-Hydroxy-1-[2-(5-fluor-1H-indol-3-yl)ethyl]-piperidin-3-carbonsäureethylester,
4-Hydroxy-1-[2-(5-brom-(1H-indol-3-yl)ethyl]-1,2,5,6-tetrahydropyridin-3-carbonsäuremethylester bzw. 1-[2-(5-Brom-1H-indol-3-yl)ethyl]-piperidin-4-on-3-carbonsäuremethylester,
4-Hydroxy-1-[2-(5-fluor-1H-indol-3-yl)ethyl]-1,2,5,6-tetrahydro-pyridin-3-carbonsäureethylester bzw. 1-[2-(5-Fluor-1H-indol-3-yl)ethyl]-piperidin-4-on-3-carbonsäureethylester,
cis-4-Hydroxy-1-[2-(5-fluor-1H-indol-3-yl)ethyl]-piperidin-3-carbonsäuremethylester,
trans-4-Hydroxy-1-[2-(fluor-1H-indol-3-yl)ethyl]-piperidin-3-carbonsäuremethylester,
cis-4-Hydroxy-1-[2-(5-brom-1H-indol-3-yl)ethyl]-piperidin-3-carbonsäuremethylester,

4-Hydroxy-1-[2-(5-fluor-1H-indol-3-yl)ethyl]-1,2,5,6-tetrahydro-pyridin-3-carbonsäuremethylester bzw. 1-[2-(5-Fluor-1H-indol-3-yl)ethyl]-piperidin-4-on-3-carbonsäuremethylester,
trans-4-Hydroxy-1-[2-(5-brom-1H-indol-3-yl)ethyl]-piperidin-3-carbonsäuremethylester,
4-Hydroxy-1-[2-(4,6-dimethyl-1H-indol-3-yl)ethyl]-1,2,5,6-tetrahydro-pyridin-3-carbonsäuremethylester bzw. 1-[2-(4,6-Dimethyl-1H-indol-3-yl)ethyl]-piperidin-4-on-3-carbonsäuremethylester,
4-Hydroxy-1-[2-(5-chlor-1H-indol-3-yl)-ethyl]-1,2,5,6-tetrahydro-pyridin-3-carbonsäuremethylester bzw. 1-[2-(5-Chlor-1H-indol-3-yl)ethyl]-piperidin-4-on-3-carbonsäuremethylester,
cis-4-Hydroxy-1-[2-(5-chlor-1H-indol-3-yl)ethyl]-piperidin-3-carbonsäuremethylester,
trans-4-Hydroxy-1-[2-(5-chlor-1H-indol-3-yl)ethyl]-piperidin-3-carbonsäuremethylester,
cis-4-Hydroxy-1-[2-(4,6-dimethyl-1H-indol-3-yl)ethyl]-piperidin-3-carbonsäuremethylester,
trans-4-Hydroxy-1-[2-(4,6-dimethyl-1H-indol-3-yl)ethyl]-piperidin-3-carbonsäuremethylester,
cis-4-Methoxy-1-[2-(1H-indol-3-yl)ethyl]-piperidin-3-carbonsäuremethylester,
cis-4-Hydroxy-1-[2-(5-methoxy-1H-indol-3-yl)ethyl]-piperidin-3-carbonsäuremethylester,
trans-4-Hydroxy-1-[2-(5-methoxy-1H-indol-3-yl)ethyl]-piperidin-3-carbonsäuremethylester,
4-Hydroxy-1-[2-(1H-indol-3-yl)ethyl]-2,5-dihydro-1H-pyrrol-3-carbonsäureethylester bzw. 1-[2-(1H-Indol-3-yl)ethyl]-pyrrolidin-4-on-3-carbonsäureethylester,
cis-4-Amino-1-[2-(1H-indol-3-yl)ethyl]-piperidin-3-carbonsäuremethylester,
trans-4-Amino-1-[2-(1H-indol-3-yl)ethyl]-piperidin-3-carbonsäuremethylester,
4-Hydroxy-1-[2-(1H-indol-3-yl)ethyl]-2,5-dihydro-1H-pyrrol-3-carbonsäuremethylester bzw. 1-[2-(1H-Indol-yl)ethyl]-pyrrolidin-4-on-3-carbonsäuremethylester,

cis-4-Hydroxy-1-[2-(1H-indol-3-yl)ethyl]-pyrrolidin-3-carbonsäureethylester,
trans-4-Hydroxy-1-[2-(1H-indol-3-yl)ethyl]-pyrrolidin-3-carbonsäureethylester,
cis-4-Hydroxy-1-[2-(1H-indol-3-yl)ethyl]-pyrrolidin-3-carbonsäuremethylester,
trans-4-Hydroxy-1-[2-(1H-indol-3-yl)ethyl]-pyrrolidin-3-carbonsäuremethylester,
4-Hydroxy-1-[2-(1H-indol-3-yl)ethyl]-1,2,5,6-tetrahydro-7H-azepin-3-carbonsäureethylester bzw. 1-[2-(1H-Indol-3-yl)ethyl]-tetrahydroazepin-4-on-3-carbonsäureethylester,
cis-4-Hydroxy-1-[2-(1H-indol-3-yl)ethyl]-hexahydroazepin-3-carbonsäureethylester oder
trans-4-Hydroxy-1-[2-(1H-indol-3-yl)ethyl]-hexahydroazepin-3-carbonsäureethylester oder jeweils ein Salz davon herstellt.

15. Verfahren gemäss einem der Ansprüche 1, 3-5, 7, 10, 11, und 14, dadurch gekennzeichnet, dass man eine verfahrensgemäss erhältliche Verbindung in Form eines Hydrochlorides, Hydrobromides, Fumarates, Oxalates oder Cyclamates herstellt.

16. Verfahren gemäss einem der Ansprüche 2, 6, 8, 9, 12 und 13, dadurch gekennzeichnet, dass man eine verfahrensgemäss erhältliche Verbindung in Form eines Hydrochlorides, Hydrobromides, Fumarates, Oxalates oder Cyclamates herstellt.

17. Verfahren zur Herstellung von Verbindungen der Formel

$$R - Z - \overset{R_6}{\overset{|}{N}} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3 Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1'''$, wobei $R_1'''$ eine der für $R_1$ angegebenen Bedeu-

tungen hat oder für Cyano, Hydroxymethyl oder gegebenenfalls acetalisiertes Formyl steht und alk Methylen, Ethylen oder 1,3-Propylen bedeutet, mit der Massgabe, dass in Verbindungen der Formel XVI, worin R unsubstituiert und $R_6$ Wasserstoff ist, Z von Ethylen verschieden ist, wenn $R_1$ Methoxycarbonyl oder Ethoxycarbonyl bedeutet, und ihrer Salze, dadurch gekennzeichnet, dass man

l) Verbindungen der Formeln

$$R\text{-}Z\text{-}X_5 \quad (XXXII) \quad \text{und} \quad X_6\text{-}CH_2CH_2\text{-}R_1 \quad (XXXIII),$$

worin einer der Reste $X_5$ und $X_6$ eine Gruppe der Formel $-N(R_6)-H$ und der andere eine nukleofuge Abgangsgruppe bedeutet, oder deren Salze miteinander umsetzt, oder

m) Verbindungen der Formeln

$$R\text{-}Z\text{-}N(R_6)\text{-}H \quad (XXXVII) \quad \text{und} \quad CH_2{=}CH\text{-}R_1 \quad (XXXIV)$$

oder deren Salze miteinander umsetzt, oder

n) eine Verbindung der Formel

$$R_3 - \underset{\substack{|\\R_4}}{N} - N = \underset{\substack{|\\R_5}}{C} - CH_2 - Z - \underset{\substack{|\\R_6}}{N} - CH_2CH_2 - R_1 \quad (XXXV)$$

worin $R_6$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel XVI ringschliesst, oder

o) in einer Verbindung der Formel

$$R - Z - \overset{R_6'}{\overset{|}{N}} - CH_2CH_2 - R_1' \quad (XXXVI)$$

worin $R_6'$ Wasserstoff oder eine Gruppe der Formel alk-$R_1'$ und $R_1'$ einen in gegebenenfalls verestertes oder amidiertes Carboxy $R_1$ überführbaren Rest bedeutet, $R_1'$ in gegebenenfalls verestertes oder amidiertes Carboxy überführt und gewünschtenfalls die verfahrensgemäss erhältliche Verbindung in eine andere Verbindung der Formel XVI überführt, ein verfahrensgemäss erhältliches Isomerengemisch in die Komponenten auftrennt und das Isomere der Formel XVI isoliert, ein verfahrensgemäss erhältliches Diastereomerengemisch bzw. Enantiomerengemisch in die Diastereomeren bzw. Enantiomeren aufspaltet und das gewünschte Diastereomere bzw. Enantiomere isoliert und/oder eine verfahrensgemäss erhältliche freie Verbindung in ein Salz oder ein verfahrensgemäss erhältliches Salz in die freie Verbindung oder in ein anderes Salz umwandelt.

18. Verfahren gemäss Anspruch 17, dadurch gekennzeichnet, dass man Verbindungen der Formel XVI, worin R, Z und $R_1$ die in Anspruch 17 angegebenen Bedeutungen haben, und $R_6$ für Wasserstoff oder eine Gruppe der Formel $-(CH_2)_n-R_1'''$ steht, worin n 2 darstellt und $R_1'''$ Formyl oder Iminomethyl bedeutet oder, sofern $R_1$ freies oder verestertes Carboxy darstellt, ebenfalls freies oder verestertes Carboxy bedeutet bzw., sofern $R_1$ Niederalkoxycarbonyl darstellt, Formyl, Hydroxymethyl oder Cyano bedeutet und ihrer Salze, dadurch gekennzeichnet, dass man

1) eine Verbindung der Formel

$$R-Z-X_3 \quad (V),$$

worin $X_3$ reaktionsfähig verestertes Hydroxy bedeutet, mit einer Verbindung der Formel

$H_2N-CH_2CH_2-R_1$ (XVa)

umsetzt und die erhaltene Verbindung der Formel XVI, worin $R_6$ Wasserstoff ist, gewünschtenfalls mit Acrolein oder einem Aldehyd der Formel $Y_1-CH_2CH_2-CH=R_2'$ (XVIIa), worin $Y_1$ reaktionsfähiges verestertes Hydroxy und $R_2'$ Oxo bedeutet, weiter umsetzt, oder

m) eine Verbindung der Formel

$R-Z-NH_2$ (XVIII)

mit der annähernd doppeltmolaren Menge einer Verbindung der Formel $Y_1-CH_2CH_2-R_1''$ (XIXa) bzw. $CH_2=CH-R_1''$ (XIXb), worin $Y_1$ reaktionsfähiges verestertes Hydroxy und $R_1''$ freies oder verestertes Carboxy bedeutet, umsetzt und gewünschtenfalls eine verfahrensgemäss erhältliche freie Verbindung in ein Salz oder ein verfahrensgemäss erhältliches Salz in die freie Verbindung oder in ein anderes Salz umwandelt.

19. Verfahren gemäss Anspruch 17, dadurch gekennzeichnet, dass man Verbindungen der Formel XVI, worin R unsubstituiertes oder in 5-Stellung durch Methoxy substituiertes 3-Indolyl bedeutet, Z für Ethylen steht, $R_1$ Methoxycarbonyl bedeutet und $R_6$ Wasserstoff oder eine Gruppe der Formel $-(CH_2)_n-R_1'''$ darstellt, in der n für 2 und $R_1'''$ für Methoxycarbonyl oder Formyl steht, und ihrer Salze, dadurch gekennzeichnet, dass man

l) eine Verbindung der Formel

$R-Z-X_3$ (V),

worin $X_3$ reaktionsfähig verestertes Hydroxy bedeutet, mit einer Verbindung der Formel

$H_2N-CH_2CH_2-R_1$ (XVa)

umsetzt und die erhaltene Verbindung der Formel XVI, worin $R_6$ Wasserstoff ist, gewünschtenfalls mit Acrolein oder einem Aldehyd der Formel $Y_1-CH_2CH_2-CH=R_2'$ (XVIIa), worin $Y_1$ reaktionsfähiges verestertes Hydroxy und $R_2'$ Oxo bedeutet, weiter umsetzt, oder

m) eine Verbindung der Formel

$R-Z-NH_2$ (XVIII)

mit der annähernd doppeltmolaren Menge einer Verbindung der Formel $Y_1-CH_2CH_2-R_1''$ (XIXa) bzw. $CH_2=CH-R_1''$ (XIXb), worin $Y_1$ reaktionsfähiges verestertes Hydroxy und $R_1''$ Methoxycarbonyl bedeutet, umsetzt und gewünschtenfalls eine verfahrensgemäss erhältliche freie Verbindung in ein Salz oder ein verfahrensgemäss erhältliches Salz in die freie Verbindung oder in ein anderes Salz umwandelt.

20. Verfahren gemäss Anspruch 17, dadurch gekennzeichnet, dass man den Verbindungen der Formel XVI, worin R unsubstituiertes oder in einer oder zwei der Stellungen 4 bis 7 durch Niederalkyl mit bis und mit 4 C-Atomen, Niederalkoxy mit bis und mit 4 C-Atomen, Halogen der Atomnummer bis und mit 35 und/oder Trifluormethyl substituiertes 3-Indolyl bedeutet, Z Niederalkylen mit bis und mit 4 C-Atomen darstellt, welches den Rest R vom Stickstoffatom durch 1 bis 3 C-Atome trennt, $R_1$ für Carboxy, Niederalkoxycarbonyl mit bis und mit 4 C-Atomen im Alkoxyteil, 3- bis 8-gliedriges Cycloalkoxycarbonyl, Carbamyl oder N-Mono- oder N,N-Diniederalkylcarbamyl mit bis und mit 4 C-Atomen in jedem Alkylteil steht bis und $R_6$ für Wasserstoff oder eine Gruppe der Formel $-(CH_2)_n-R_1'''$ bedeutet, worin n 1, 2 oder 3 ist und $R_1'''$ Carboxy, Niederalkoxycarbonyl mit bis und mit 4 C-Atomen im Niederalkoxyteil, 3- bis 8-gliedriges Cycloalkoxycarbonyl, Carbamyl oder N-Mono-oder N,N-Diniederalkylcarbamyl mit bis und mit 4 C-Atomen in jedem Alkylteil Cyano, Hydroxymethyl, Formyl, Diniederalkoxymethyl oder Niederalkylendioxymethyl mit bis und mit 4 C-Atomen in den Alkoxyteilen bzw. im Alkylendioxyteil bedeutet, oder ein Salz davon herstellt.

21. Verfahren gemäss Anspruch 17, dadurch gekennzeichnet, dass man den Verbindungen der Formel XVI, worin R unsubstituiertes oder in 4- und 7-Stellung durch Halogen der Atomnummer bis und mit 35 und/oder Niederalkyl mit bis und mit 4 C-Atomen substituiertes 3-Indolyl bedeutet, Z Ethylen darstellt, $R_1$ Niederalkoxycarbonyl mit bis und mit 4 C-Atomen im Niederalkoxyteil bedeutet und $R_6$ Wasserstoff oder eine Gruppe der Formel $-(CH_2)_n-R_1'''$ bedeutet, worin n 1 oder 2 ist und $R_1'''$ die gleiche Bedeutung wie $R_1$ hat oder Cyano, Dinieder-alkoxymethyl mit je bis und mit 4 Alkoxy-C-atomen, Niederalkylendioxymethyl mit 2 bis und mit 4 Alkylendioxy-C-atomen oder N,N-Diniederalkylcarbamyl mit je bis und mit 4 Alkyl-C-atomen bedeutet, oder ein Salz davon.

22. Verfahren gemäss Anspruch 17, dadurch gekennzeichnet, dass man
N-[2-(1H-Indol-3-yl)ethyl]-imino-di(3-propionsäure)dimethylester,
N-[2-(1H-Indol-3-yl)ethyl]-imino-di(3-propionsäure)diethylester,
N-[2-(4,6-Dimethyl-1H-indol-3-yl)ethyl]-imino-di(3-propionsäure)diethylester,
N-[2-(4,6-Dimethyl-1H-indol-3-yl)-ethyl]-imino-di(3-propionsäure)dimethylester,
N-[2-(5-Chlor-1H-indol-3-yl)ethyl]-imino-di(3-propionsäure)diethylester,
N-[2-(5-Chlor-1H-indol-3-yl)ethyl]-imino-di(3-propionsäure)di-methylester,
N-[3-(1H-Indol-3-yl)propyl]-imino-di(3-propionsäure)dimethyl-ester,
N-[2-(5-Methoxy-1H-indol-3-yl)ethyl]-imino-di(3-propionsäure)diethylester,
3-[2-(1H-Indol-3-yl)ethyl-N-(2-cyanoethyl)-β-alaninmethylester,
N-[2-(1H-Indol-3-yl)ethyl]-N-[2-(1,3-dioxolan-2-yl)ethyl]-β-alaninmethylester,
N-[2-(1H-Indol-3-yl)ethyl]-N-[2-(N,N-dimethylcarbamyl)ethyl]-β-alaninethylester,
N-[2-(1H-Indol-3-yl)ethyl]-N-(methoxycarbonylmethyl)-β-alanin-methylester,

N-[2-(1H-Indol-3-yl)ethyl]-N-(ethoxycarbonylmethyl)-ß-alanin-ethylester,
N-[2-(5-Brom-1H-indol-3-yl)ethyl]-imino-di(3-propionsäure)diethylester,
N-[2-(5-Brom-1H-indol-3-yl)ethyl]-imino-di(3-propionsäure)di-methylester,
N-[2-(5-Fluor-1H-indol-3-yl)ethyl]-imino-di(3-propionsäure)-dimethylester,
N-[2-(5-Fluor-1H-indol-3-yl)ethyl]-imino-di(3-propionsäure)-diethylester,
N-[2-(4,6-Dimethyl-1H-indol-3-yl)ethyl]-ß-alaninmethylester,
N-[2-(1H-Indol-3-yl)ethyl]-N-(3-ethoxycarbonylpropyl)-ß-alaninethylester,
N-[2-(1H-Indol-3-yl)ethyl]-N-(3-methoxycarbonylpropyl)-ß-alaninmethylester,
N-[2-(5-Methoxy-1H-indol-3-yl)ethyl]-imino-di(3-propionsäure)-dimethylester oder
N-[2-(1H-Indol-3-yl)ethyl]-N-(4-oxobutyl)-ß-alaninmethylester
oder jeweils ein Salz davon herstellt.

23. Verfahren gemäss Anspruch 18, dadurch gekennzeichnet, dass man
N-[2-(1H-Indol-3-yl)ethyl]-imino-di(3-propionsäure)diethylester,
N-[2-(4,6-Dimethyl-1H-indol-3-yl)ethyl]-imino-di(3-propionsäure)-diethylester,
N-[2-(4,6-Dimethyl-1H-indol-3-yl)-ethyl]-imino-di(3-propionsäure)-dimethylester, oder
N-[N-[2-(5-Chlor-1H-indol-3-yl)ethyl]-imino-di(3-propionsäure)diethylester oder jeweils ein Salz davon herstellt.

24. Verfahren gemäss 19, dadurch gekennzeichnet, dass man
N-[2-(1H-Indol-3-yl)ethyl]imino-di(3-propionsäure)dimethylester,
N-[2-(5-Methoxy-1H-indol-3-yl)ethyl]-imino-di(3-propionsäure)-dimethylester oder
N-[2-(1H-Indol-3-yl)ethyl]-N-(4-oxobutyl)-ß-alaninmethylester oder jeweils ein Salz davon herstellt.

25. Verfahren gemäss einem der Ansprüche 17-24, dadurch gekennzeichnet, dass man eine verfahrensgemäss erhältliche Verbindung in Form eines Hydrochlorides, Hydrobromides, Fumarates, Oxalates oder Cyclamates herstellt.

26. Verfahren zur Herstellung pharmazeutischer Präparate dadurch gekennzeichnet, dass man

l) Verbindungen der Formeln

$R-Z-X_5$ (XXXII) und $X_6-CH_2CH_2-R_1$ (XXXIII),

worin einer der Reste $X_5$ und $X_6$ eine Gruppe der Formel $-N(R_6)-H$ und der andere eine nukleofuge Abgangsgruppe bedeutet, oder deren Salze miteinander umsetzt, oder

m) Verbindungen der Formeln

$R-Z-N(R_6)-H$ (XXXVII) und $CH_2{=}CH-R_1$ (XXXIV)

oder deren Salze miteinander umsetzt, oder

n) eine Verbindung der Formel

$$R_3 - \underset{R_4}{N} - N = \underset{R_5}{C} - CH_2 - Z - \underset{R_6}{N} - CH_2CH_2 - R_1 \quad (XXXV)$$

worin $R_6$ einen in 3- bis 6-Stellung gegebenenfalls wie für die Stellungen 4 bis 7 des Restes R angegeben substituierten Phenylrest, $R_4$ Wasserstoff oder einen für die 1-Stellung von R vorgesehenen Substituenten und $R_5$ Wasserstoff oder einen für die 2-Stellung von R vorgesehenen Substituenten bedeutet, oder ein Tautomeres und/oder Salz davon, zu der entsprechenden Verbindung der Formel XVI ringschliesst, oder

o) in einer Verbindung der Formel

$$R - Z - \overset{\overset{R_6'}{|}}{N} - CH_2CH_2 - R_1' \quad (XXXVI)$$

worin $R_6$' Wasserstoff oder eine Gruppe der Formel alk-$R_1$' und $R_1$' einen in gegebenenfalls verestertes oder amidiertes Carboxy $R_1$ überführbaren Rest bedeutet, $R_1$' in gegebenenfalls verestertes oder amidiertes Carboxy überführt, worin R, Z, $R_1$ und $R_6$ die in Anspruch 18 angegebenen Bedeutungen haben, und die jeweils erhaltene Verbindung der Formel

$$R - Z - \overset{\overset{R_6}{|}}{N} - CH_2CH_2 - R_1 \qquad (XVI),$$

worin R einen gegebenenfalls substituierten 3-Indolylrest bedeutet, Z Niederalkylen darstellt, welches den Rest R durch 1 bis 3, vorzugsweise 2, Kohlenstoffatome vom Stickstoffatom trennt, $R_1$ gegebenenfalls verestertes oder amidiertes Carboxy bedeutet und $R_6$ Wasserstoff oder eine Gruppe der Formel alk-$R_1$"', wobei $R_1$"' eine der für $R_1$ angegebenen Bedeutungen hat oder für Cyano, Hydroxymethyl oder gegebenenfalls acetalisiertes Formyl, steht und alk Methylen, Ethylen oder 1,3-Propylen bedeutet, oder eines ihrer pharmazeutisch verwendbaren Salze mit üblichen pharmazeutischen Hilfsstoffen vermischt.

27. Verfahren gemäss Anspruch 26, dadurch gekennzeichnet, dass man pharmazeutische Präparate herstellt, die als Wirkstoff N-[2-(1H-Indol-3-yl)ethyl]-β-alaninethylester oder N-[2-(1H-Indol-3-yl)-ethyl]-β-alaninmethylester oder jeweils ein pharmazeutisch verwendbares Salz davon enthalten.

28. Verfahren zur Herstellung pharmazeutischer Präparate, dadurch gekennzeichnet, dass man eine Verbindung, erhältlich gemäss einem der Ansprüche 1, 3-5, 7, 12, 14, 15, 17, 19-21, 23 und 24, mit üblichen pharmazeutischen Hilfs- und/oder Trägerstoffen vermischt.

29. Verfahren zur Herstellung pharmazeutischer Präparate, dadurch gekennzeichnet, dass man eine Verbindung, erhältlich gemäss einem der Ansprüche 2, 5, 6, 8, 9, 12, 16, 18 und 25, mit üblichen pharmazeutischen Hilfs- und/oder Trägerstoffen vermischt.

30. Die gemäss dem Verfahren eines der Ansprüche 1-3 und 17-19 erhältlichen neuen Verbindungen.

FO 7.4 KVB/cs*/bg*/sm*/co*